# EUROPEAN PATENT SPECIFICATION

(11) **EP 3 428 989 B1**
(45) Date of publication and mention of the grant of the patent: **21.04.2021**
(21) Application number: 17763222.1
(22) Date of filing: 07.03.2017
(51) Int. Cl.: H01L 51/50, H01L 51/00, C09K 11/06, H05B 33/12, C07D 403/04, C07F 15/00

(54) **LIGHT-EMITTING ELEMENT**
LICHTEMITTIERENDES ELEMENT
ÉLÉMENT LUMINESCENT

(30) Priority: 10.03.2016 JP 2016046686
(43) Date of publication of application: 16.01.2019
(73) Proprietor: Sumitomo Chemical Company Limited, Tokyo 104-8260 (JP)
(72) Inventor: SASADA, Toshiaki, Tsukuba-shi Ibaraki 300-3294 (JP); FUKUSHIMA, Daisuke, Tsukuba-shi Ibaraki 300-3294 (JP); TANAKA, Shin-ya, Tsukuba-shi Ibaraki 300-3294 (JP)
(74) Representative: J A Kemp LLP
(86) International application number: PCT/JP2017/008949
(87) International publication number: WO 2017/154882

(56) References cited:
- EP-A1- 2 832 761
- EP-A1- 3 522 248
- WO-A1-2012/089294
- WO-A1-2013/017189
- WO-A1-2013/146806
- WO-A1-2014/090368
- WO-A1-2014/090368
- WO-A1-2015/186539
- WO-A1-2015/194448
- WO-A1-2016/002646
- JP-A- 2014 045 101
- JP-A- 2014 075 344
- JP-A- 2014 503 661
- JP-A- 2014 527 522

## Description

### Technical Field

The present invention relates to a light emitting device.

### Background Art

An organic electroluminescent device (hereinafter, referred to also as "light emitting device") can be suitably used in applications of display and illumination, and is under active research and development. For example, Patent Document 1 discloses a light emitting device comprising an organic layer comprising a crosslinked body of a crosslinkable material and a light emitting layer comprising a compound (H0-1) represented by the following formula and a phosphorescent compound (G0-1) represented by the following formula. The compound (H0-1) is a compound which does not have a group represented by the below-mentioned formula (H1-1) .

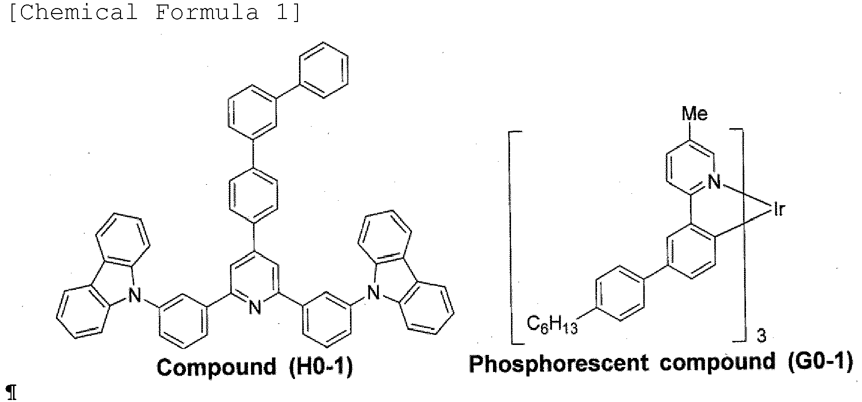

WO 2014/090368 A1 discloses a device similar to the present invention.

### Prior Art Document

### Patent Document

Patent Document 1: JP 2010-155985 A

### Summary of the Invention

### Problems to be Solved by the Invention

However, the above-mentioned light emitting devices were not always sufficient in luminance life.

Accordingly, it is an object of the present invention to provide a light emitting device excellent in luminance life.

### Means for Solving the Problems

The present invention provides the following [1] to [11] .
[1] A light emitting device comprising an anode, a cathode, a first organic layer disposed between the anode and the cathode, and a second organic layer disposed between the anode and the cathode, wherein
   the first organic layer is a layer comprising a phosphorescent compound represented by formula (1) and a compound represented by formula (H), and
   the second organic layer is a layer comprising a crosslinked body of a crosslinkable material: 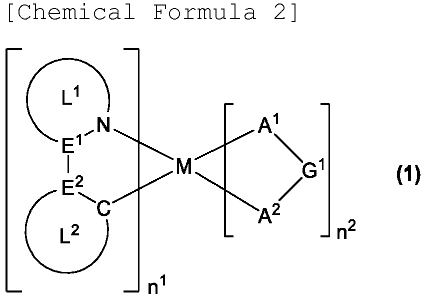 wherein
   M represents a ruthenium atom, a rhodium atom, a palladium atom, an iridium atom or a platinum atom,
   n¹ represents an integer of 1 or more, n² represents an integer of 0 or more, n¹+n² is 2 or 3, n¹+n² is 3 when M is a ruthenium atom, a rhodium atom or an iridium atom, and n¹+n² is 2 when M is a palladium atom or a platinum atom,
   E¹ and E² each independently represent a carbon atom or a nitrogen atom, and at least one of E¹ and E² is a carbon atom, and when a plurality of E¹ and E² are present, they may be the same or different at each occurrence,
   ring L¹ represents an aromatic heterocyclic ring, and the ring optionally has a substituent, and when a plurality of the substituents are present, they may be the same or different and may be combined together to form a ring together with the atoms to which they are attached, and when a plurality of the rings L¹ are present, they may be the same or different,
   ring L² represents an aromatic hydrocarbon ring or an aromatic heterocyclic ring, these rings each optionally have a substituent, and when a plurality of the substituents are present, they may be the same or different and may be combined together to form a ring together with the atoms to which they are attached, and when a plurality of the rings L² are present, they may be the same or different,
   the substituent which the ring L¹ optionally has and the substituent which the ring L² optionally has may be combined together to form a ring together with the atoms to which they are attached, and
   A¹-G¹-A² represents an anionic bidentate ligand, A¹ and A² each independently represent a carbon atom, an oxygen atom or a nitrogen atom, and these atoms each may be an atom constituting a ring, G¹ represents a single bond or an atomic group constituting a bidentate ligand together with A¹ and A², and when a plurality of A¹-G¹-A² are present, they may be the same or different: 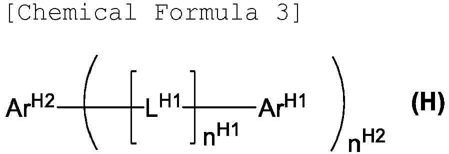
   wherein
   n^{H1} represents an integer of 0 or more and 5 or less, and when a plurality of n^{H1} are present, they may be the same or different,
   n^{H2} represents an integer of 1 or more and 10 or less,
   Ar^{H1} represents a group represented by formula (H1-1), and when a plurality of Ar^{H1} are present, they may be the same or different,
   L^{H1} represents an alkylene group, a cycloalkylene group, an arylene group, a divalent heterocyclic group, a group represented by -NR^{H1'}-, an oxygen atom or a sulfur atom, and these groups each optionally have a substituent, R^{H1'} represents a hydrogen atom, an alkyl group, a cycloalkyl group, an aryl group or a monovalent heterocyclic group, and these groups each optionally have a substituent, and when a plurality of L^{H1} are present, they may be the same or different, and
   Ar^{H2} represents an aromatic hydrocarbon group or an aromatic heterocyclic group, and these groups each optionally have a substituent: 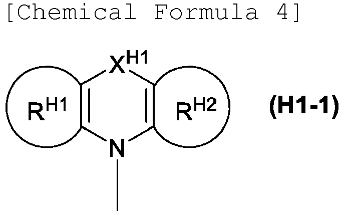
   wherein
   ring R^{H1} and ring R^{H2} each independently represent a monocyclic or fused-ring aromatic hydrocarbon ring, or a monocyclic or fused-ring aromatic heterocyclic ring, and these rings each optionally have a substituent, and when a plurality of the substituents are present, they may be the same or different and may be combined together to form a ring together with the atoms to which they are attached,
   at least one of the ring R^{H1} and the ring R^{H2} represents a fused-ring aromatic hydrocarbon ring or a fused-ring aromatic heterocyclic ring, and these rings each optionally have a substituent,
   X^{H1} represents a single bond, an oxygen atom, a sulfur atom, a group represented by -N(R^{XH1})- or a group represented by -C(R^{XH1'})₂-, R^{XH1} and R^{XH1'} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and these groups each optionally have a substituent, and the plurality of R^{XH1'} may be the same or different and may be combined together to form a ring together with the carbon atoms to which they are attached, and
   R^{XH1} and the substituent which the ring R^{H1} optionally has, R^{XH1} and the substituent which the ring R^{H2} optionally has, R^{XH1'} and the substituent which the ring R^{H1} optionally has, and R^{XH1'} and the substituent which the ring R^{H2} optionally has each may be combined together to form a ring together with the atoms to which they are attached.
[2] The light emitting device according to [1], wherein the crosslinkable material is a low molecular weight compound having at least one crosslinkable group selected from Group A of crosslinkable group, or a polymer compound comprising a crosslinkable constitutional unit having at least one crosslinkable group selected from Group A of crosslinkable group:
   (Group A of Crosslinkable Group) 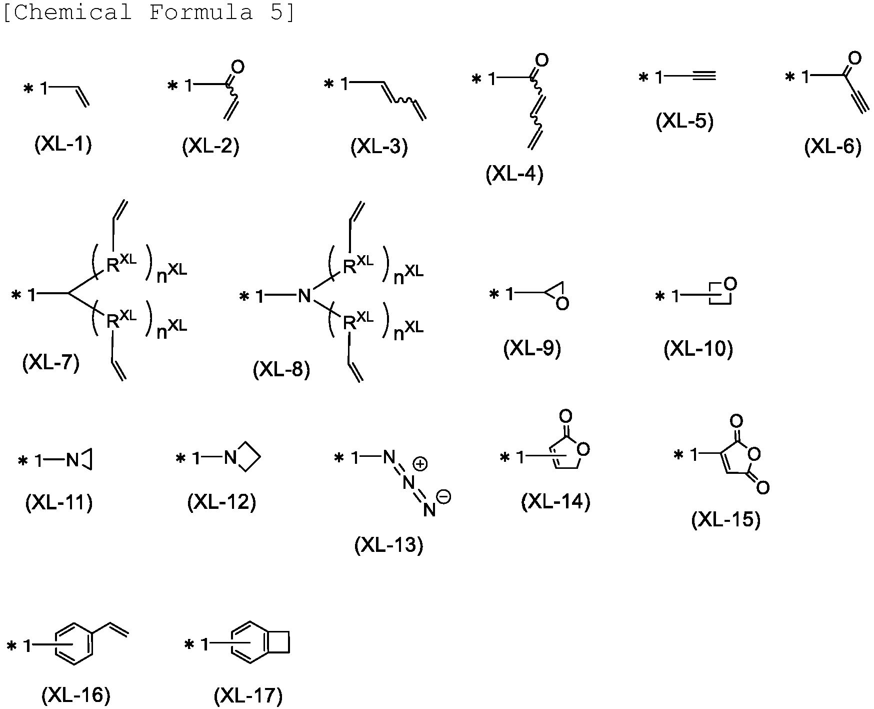 wherein R^{XL} represents a methylene group, an oxygen atom or a sulfur atom, n^{XL} represents an integer of 0 to 5, and when a plurality of R^{XL} are present, they may be the same or different, and when a plurality of n^{XL} are present, they may be the same or different, *1 represents a binding site, and these crosslinkable groups each optionally have a substituent.
[3] The light emitting device according to [2], wherein the crosslinkable material is a polymer compound comprising a crosslinkable constitutional unit having at least one crosslinkable group selected from Group A of crosslinkable group.
[4] The light emitting device according to [3], wherein the crosslinkable constitutional unit is a constitutional unit represented by formula (2) or a constitutional unit represented by formula (2'): 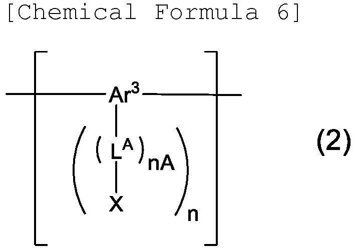 wherein
   nA represents an integer of 0 to 5, n represents 1 or 2, and when a plurality of nA are present, they may be the same or different,
   Ar³ represents an aromatic hydrocarbon group or a heterocyclic group, and these groups each optionally have a substituent,
   L^{A} represents an alkylene group, a cycloalkylene group, an arylene group, a divalent heterocyclic group, a group represented by -NR'-, an oxygen atom or a sulfur atom, and these groups each optionally have a substituent, R' represents a hydrogen atom, an alkyl group, a cycloalkyl group, an aryl group or a monovalent heterocyclic group, and these groups each optionally have a substituent, and when a plurality of L^{A} are present, they may be the same or different, and
   X represents a crosslinkable group selected from Group A of crosslinkable group, and when a plurality of X are present, they may be the same or different: 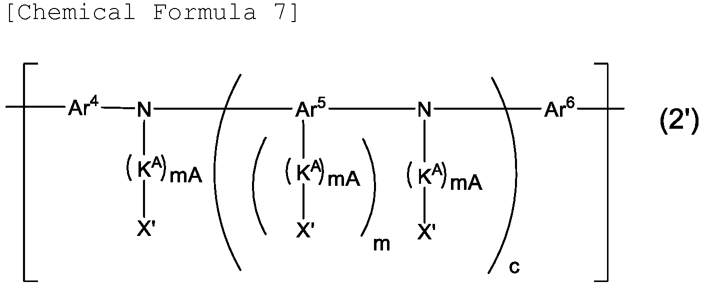
   wherein
   mA represents an integer of 0 to 5, m represents an integer of 1 to 4, c represents an integer of 0 or 1, and when a plurality of mA are present, they may be the same or different,
   Ar⁵ represents an aromatic hydrocarbon group, a heterocyclic group, or a group in which at least one aromatic hydrocarbon ring and at least one heterocyclic ring are bonded directly to each other, and these groups each optionally have a substituent,
   Ar⁴ and Ar⁶ each independently represent an arylene group or a divalent heterocyclic group, and these groups each optionally have a substituent,
   Ar⁴, Ar⁵ and Ar⁶ each may be bonded directly or via an oxygen atom or a sulfur atom to a group other than these groups bonding to the nitrogen atom to which these groups are attached, thereby forming a ring,
   K^{A} represents an alkylene group, a cycloalkylene group, an arylene group, a divalent heterocyclic group, a group represented by -NR'-, an oxygen atom or a sulfur atom, and these groups each optionally have a substituent, R' represents a hydrogen atom, an alkyl group, a cycloalkyl group, an aryl group or a monovalent heterocyclic group, and these groups each optionally have a substituent, and when a plurality of K^{A} are present, they may be the same or different, and
   X' represents a crosslinkable group selected from Group A of crosslinkable group, a hydrogen atom, an alkyl group, a cycloalkyl group, an aryl group or a monovalent heterocyclic group, and these groups each optionally have a substituent, and when a plurality of X' are present, they may be the same or different, and at least one X' is a crosslinkable group selected from Group A of crosslinkable group.
[5] The light emitting device according to any one of [2] to [4], wherein the crosslinkable group which the crosslinkable material has is a group represented by formula (XL-2), formula (XL-3), formula (XL-4), formula (XL-5), formula (XL-6), formula (XL-7), formula (XL-8), formula (XL-9), formula (XL-10), formula (XL-11), formula (XL-12), formula (XL-13), formula (XL-14), formula (XL-15) or formula (XL-17).
[6] The light emitting device according to any one of [1] to [5], wherein the group represented by formula (H1-1) is a group represented by formula (H1-1B), a group represented by formula (H1-1C) or a group represented by formula (H1-1D): 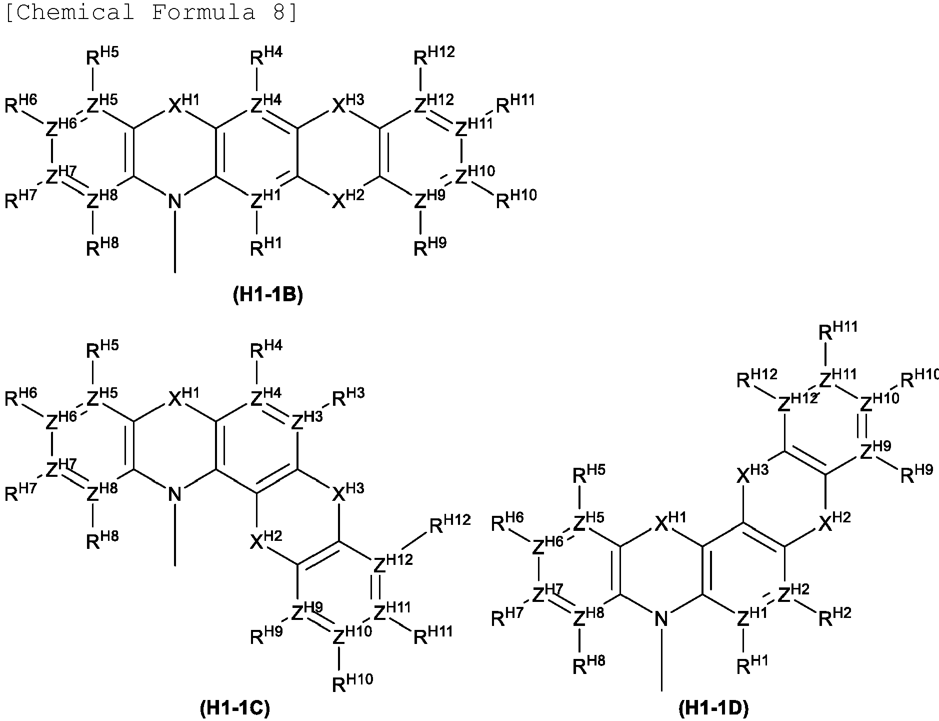 wherein
   X^{H1} represents the same meaning as defined above,
   X^{H2} and X^{H3} each independently represent a single bond, an oxygen atom, a sulfur atom, a group represented by-N(R^{XH2})- or a group represented by -C(R^{XH2'} )₂-, R^{XH2} and R^{XH2'} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and these groups each optionally have a substituent, and the plurality of R^{XH2'} may be the same or different and may be combined together to form a ring together with the carbon atoms to which they are attached,
   Z^{H1}, Z^{H2}, ZH³, Z^{H4}, Z^{H5}, Z^{H6}, Z^{H7} , Z^{H8}, Z^{H9}, Z^{H10}, Z^{H11} and Z^{H12} each independently represent a carbon atom or a nitrogen atom,
   R^{H1}, R^{H2}, R^{H3}, R^{H4}, R^{H5}, R^{H6}, R^{H7}, R^{H8}, R^{H9}, R^{H10}, R^{H11} and R^{H12} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and these groups each optionally have a substituent,
   R^{H1} is not present when Z^{H1} is a nitrogen atom, R^{H2} is not present when Z^{H2} is a nitrogen atom, R^{H3} is not present when Z^{H3} is a nitrogen atom, R^{H4} is not present when Z^{H4} is a nitrogen atom, R^{H5} is not present when Z^{H5} is a nitrogen atom, R^{H6} is not present when Z^{H6} is a nitrogen atom, R^{H7} is not present when Z^{H7} is a nitrogen atom, R^{H8} is not present when Z^{H8} is a nitrogen atom, R^{H9} is not present when Z^{H9} is a nitrogen atom, R^{H10} is not present when Z^{H10} is a nitrogen atom, R^{H11} is not present when Z^{H11} is a nitrogen atom, and R^{H12} is not present when Z^{H12} is a nitrogen atom, and
   R^{H1} and R^{H2}, R^{H3} and R^{H4}, R^{H5} and R^{H6}, R^{H6} and R^{H7}, R^{H7} and R^{H8}, R^{H9} and R^{H10}, R^{H10} and R^{H11}, and R^{H11} and R^{H12} each may be combined together to form a ring together with the carbon atoms to which they are attached.
[7] The light emitting device according to any one of [1] to [6], wherein the phosphorescent compound represented by formula (1) is a phosphorescent compound represented by formula (1-B): 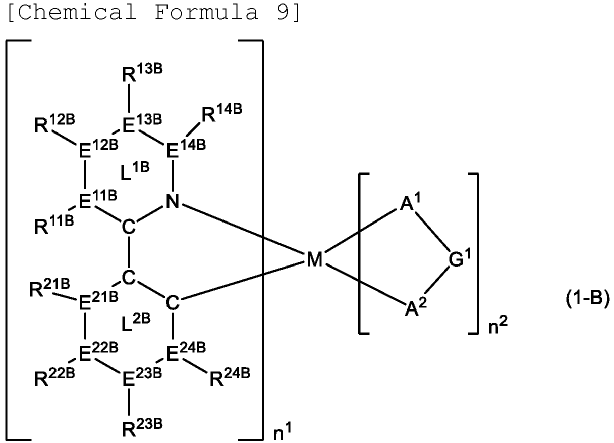 wherein
   M, n¹, n² and A¹-G¹-A² represent the same meaning as defined above,
   E^{11B}, E^{12B}, E^{13B}, E^{14B}, E^{21B}, E^{22B}, E^{23B} and E^{24B} each independently represent a nitrogen atom or a carbon atom, and when a plurality of E^{11B}, E^{12B}, E^{13B}, E^{14B}, E^{21B}, E^{22B}, E^{23B} and E^{24B} are present, they may be the same or different at each occurrence, R^{11B} is not present when E^{11B} is a nitrogen atom, R^{12B} is not present when E^{12B} is a nitrogen atom, R^{13B} is not present when E^{13B} is a nitrogen atom, R^{14B} is not present when E^{14B} is a nitrogen atom, R^{21B} is not present when E^{21B} is a nitrogen atom, R^{22B} is not present when E^{22B} is a nitrogen atom, R^{23B} is not present when E^{23B} is a nitrogen atom, and R^{24B} is not present when E^{24B} is a nitrogen atom,
   R^{11B}, R^{12B}, R^{13B}, R^{14B}, R^{21B}, R^{22B}, R^{23B} and R^{24B} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and these groups each optionally have a substituent, and when a plurality of R^{11B}, R^{12B}, R^{13B}, R^{14B}, R^{21B}, R^{22B}, R^{23B} and R^{24B} are present, they may be the same or different at each occurrence, and R^{11B} and R^{12B}, R^{12B} and R^{13B}, R^{13B} and R^{14B}, R^{11B} and R^{21B}, R^{21B} and R^{22B}, R^{22B} and R^{23B}, and R^{23B} and R^{24B} each may be combined together to form a ring together with the atoms to which they are attached,
   ring L^{1B} represents a pyridine ring or a pyrimidine ring constituted of a nitrogen atom, a carbon atom, E^{11B}, E12B, E^{13B} and E^{14B}, and
   ring L^{2B} represents a benzene ring, a pyridine ring or a pyrimidine ring constituted of two carbon atoms, E^{21B}, E^{22B} , E^{23B} and E^{24B}.
[8] The light emitting device according to [7], wherein the phosphorescent compound represented by formula (1-B) is a phosphorescent compound represented by formula (1-B1), a phosphorescent compound represented by formula (1-B2), a phosphorescent compound represented by formula (1-B3), a phosphorescent compound represented by formula (1-B4) or a phosphorescent compound represented by formula (1-B5): 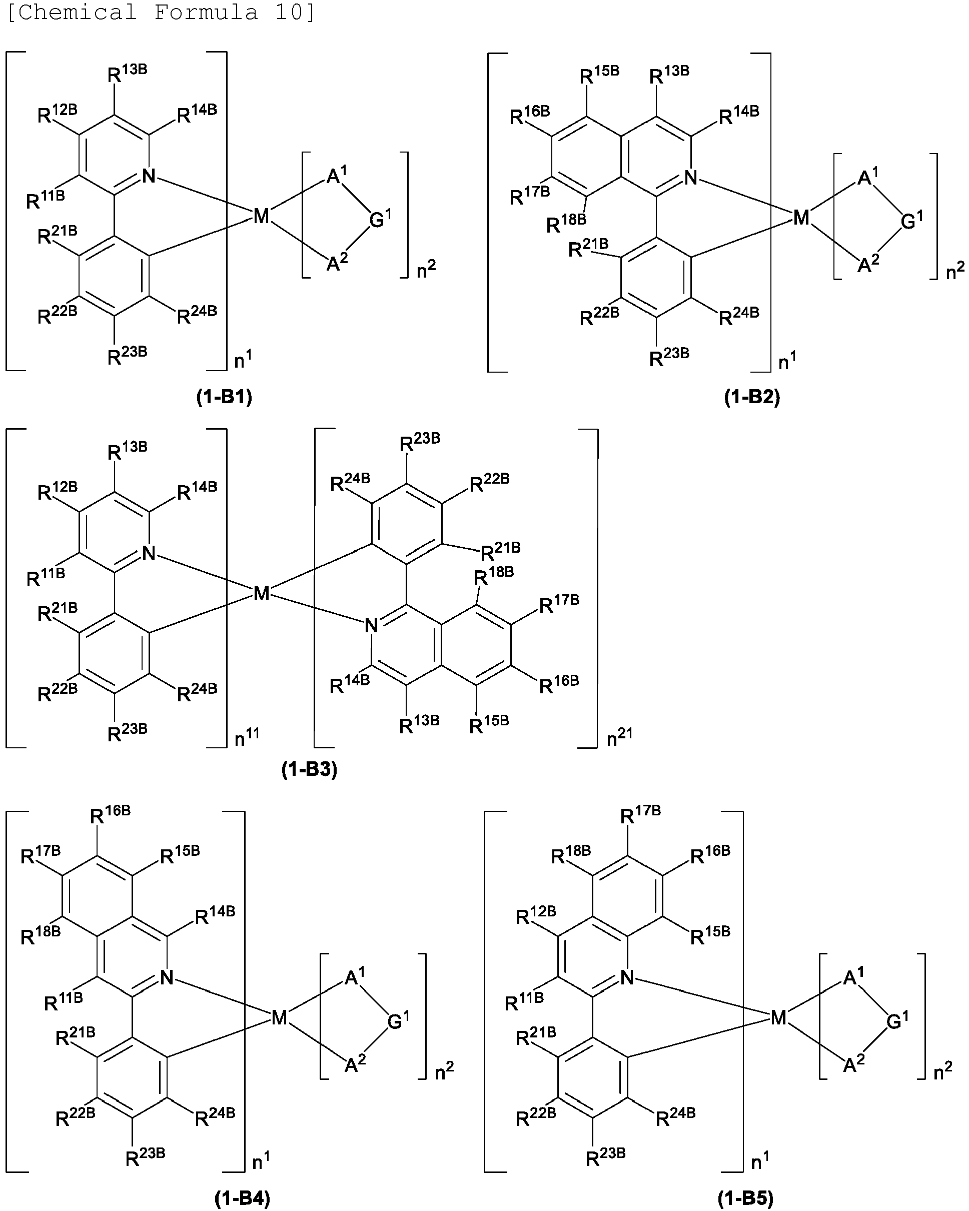 wherein
   M, n¹, n², A¹-G¹-A², R^{11B}, R^{12B}, R^{13B}, R^{14B}, R^{21B}, R^{22B}, R^{23B} and R^{24B} represent the same meaning as defined above,
   n¹¹ and n¹² each independently represent an integer of 1 or more, n¹¹+n¹² is 2 or 3, n¹¹+n¹² is 3 when M is a ruthenium atom, a rhodium atom or an iridium atom, and n¹¹+n¹² is 2 when M is a palladium atom or a platinum atom, and
   R^{15B}, R^{16B}, R^{17B} and R^{18B} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and these groups each optionally have a substituent, and when a plurality of R^{15B}, R^{16B}, R^{17B} and R^{18B} are present, they may be the same or different at each occurrence, and R^{13B} and R^{15B}, R^{15B} and R^{16B}, R^{16B} and R^{17B}, R^{17B} and R^{18B}, and R^{18B} and R^{21B} each may be combined together to form a ring together with the atoms to which they are attached.
[9] The light emitting device according to any one of [1] to [6], wherein the phosphorescent compound represented by formula (1) is a phosphorescent compound represented by formula (1-A): 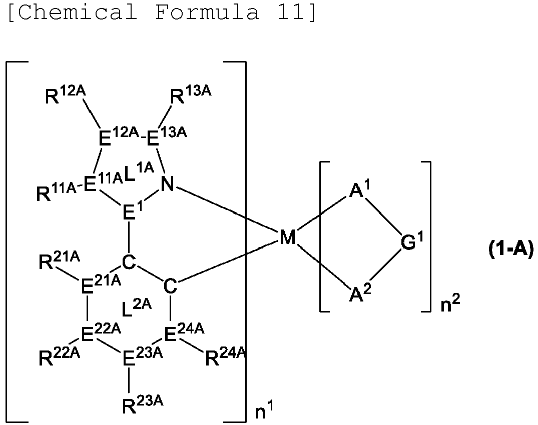 wherein
   M, n¹, n², E¹ and A¹-G¹-A² represent the same meaning as defined above,
   E^{11A}, E^{12A}, E^{13A}, E^{21A}, E^{22A}, E^{23A} and E^{24A} each independently represent a nitrogen atom or a carbon atom, and when a plurality of E^{11A}, E^{12A}, E^{13A}, E^{21A}, E^{22A}, E^{23A} and E^{24A} are present, they may be the same or different at each occurrence, R^{11A} may be either present or not present when E^{11A} is a nitrogen atom, R^{12A} may be either present or not present when E^{12A} is a nitrogen atom, R^{13A} may be either present or not present when E^{13A} is a nitrogen atom, R^{21A} is not present when E^{21A} is a nitrogen atom, R^{22A} is not present when E^{22A} is a nitrogen atom, R^{23A} is not present when E^{23A} is a nitrogen atom, and R^{24A} is not present when E^{24A} is a nitrogen atom,
   R^{11A}, R^{12A}, R^{13A}, R^{21A}, R^{22A}, R^{23A} and R^{24A} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and these groups each optionally have a substituent, and when a plurality of R^{11A}, R^{12A}, R^{13A}, R^{21A}, R^{22A}, R^{23A} and R^{24A} are present, they may be the same or different at each occurrence, and R^{11A} and R^{12A}, R^{12A} and R^{13A}, R^{11A} and R^{21A}, R^{21A} and R^{22A}, R^{22A} and R^{23A}, and R^{23A} and R^{24A} each may be combined together to form a ring together with the atoms to which they are attached,
   ring L^{1A} represents a triazole ring or a diazole ring constituted of a nitrogen atom, E¹, E^{11A}, E^{12A} and E^{13A}, and
   ring L^{2A} represents a benzene ring, a pyridine ring or a pyrimidine ring constituted of two carbon atoms, E^{21A}, E^{22A}, E^{23A} and E^{24A}.
[10] The light emitting device according to any one of [1] to [9], wherein the first organic layer and the second organic layer are adjacent to each other.
[11] The light emitting device according to any one of [1] to [10], wherein the second organic layer is a layer disposed between the anode and the first organic layer.

### Effects of the Invention

According to the embodiment of the present invention, a light emitting device excellent in luminance life can be provided.

### Modes for Carrying Out the Invention

Suitable embodiments of the present invention will be described in detail below.

### <Description of Common Term>

Terms commonly used herein have the following meanings unless otherwise stated.

Me represents a methyl group, Et represents an ethyl group, Bu represents a butyl group, i-Pr represents an isopropyl group, and t-Bu represents a tert-butyl group.

A hydrogen atom may be a heavy hydrogen atom or a light hydrogen atom.

A solid line representing a bond to a central metal in formula representing a metal complex means a covalent bond or a coordinate bond.

"Polymer compound" means a polymer having molecular weight distribution and having a polystyrene-equivalent number average molecular weight of 1 × 10³ to 1 × 10⁸.

A polymer compound may be any of a block copolymer, a random copolymer, an alternating copolymer and a graft copolymer, and may also be another embodiment.

An end group of a polymer compound is preferably a stable group because if a polymerization active group remains intact at the end, when the polymer compound is used for fabrication of a light emitting device, the light emitting property or luminance life possibly becomes lower. This end group is preferably a group having a conjugated bond to the main chain and includes, for example, groups bonding to an aryl group or a monovalent heterocyclic group via a carbon-carbon bond.

"Low molecular weight compound" means a compound having no molecular weight distribution and having a molecular weight of 1 × 10⁴ or less.

"Constitutional unit" means a unit structure found once or more in a polymer compound.

"Alkyl group" may be any of linear or branched. The number of carbon atoms of the linear alkyl group, excluding the number of carbon atoms of a substituent, is usually 1 to 50, preferably 3 to 30, and more preferably 4 to 20. The number of carbon atoms of the branched alkyl group, excluding the number of carbon atoms of a substituent, is usually 3 to 50, preferably 3 to 30, and more preferably 4 to 20.

The alkyl group optionally has a substituent, and examples thereof include a methyl group, an ethyl group, a propyl group, an isopropyl group, a butyl group, a 2-butyl group, an isobutyl group, a tert-butyl group, a pentyl group, an isoamyl group, a 2-ethylbutyl group, a hexyl group, a heptyl group, an octyl group, a 2-ethylhexyl group, a 3-propylheptyl group, a decyl group, a 3,7-dimethyloctyl group, a 2-ethyloctyl group, a 2-hexyldecyl group and a dodecyl group, and groups obtained by substituting a hydrogen atom in these groups with a cycloalkyl group, an alkoxy group, a cycloalkoxy group, an aryl group, a fluorine atom or the like, and the alkyl group having a substituent includes a trifluoromethyl group, a pentafluoroethyl group, a perfluorobutyl group, a perfluorohexyl group, a perfluorooctyl group, a 3-phenylpropyl group, a 3-(4-methylphenyl)propyl group, a 3-(3,5-di-hexylphenyl) propyl group and a 6-ethyloxyhexyl group.

The number of carbon atoms of "cycloalkyl group", excluding the number of carbon atoms of a substituent, is usually 3 to 50, preferably 3 to 30, and more preferably 4 to 20.

The cycloalkyl group optionally has a substituent, and examples thereof include a cyclohexyl group, a cyclohexylmethyl group and a cyclohexylethyl group.

"Aryl group" denotes an atomic group remaining after removing from an aromatic hydrocarbon one hydrogen atom linked directly to a carbon atom constituting the ring. The number of carbon atoms of the aryl group, excluding the number of carbon atoms of a substituent, is usually 6 to 60, preferably 6 to 20, and more preferably 6 to 10.

The aryl group optionally has a substituent, and examples thereof include a phenyl group, a 1-naphthyl group, a 2-naphthyl group, a 1-anthracenyl group, a 2-anthracenyl group, a 9-anthracenyl group, a 1-pyrenyl group, a 2-pyrenyl group, a 4-pyrenyl group, a 2-fluorenyl group, a 3-fluorenyl group, a 4-fluorenyl group, a 2-phenylphenyl group, a 3-phenylphenyl group, a 4-phenylphenyl group, and groups obtained by substituting a hydrogen atom in these groups with an alkyl group, a cycloalkyl group, an alkoxy group, a cycloalkoxy group, an aryl group, a fluorine atom or the like.

"Alkoxy group" may be any of linear or branched. The number of carbon atoms of the linear alkoxy group, excluding the number of carbon atoms of a substituent, is usually 1 to 40, and preferably 4 to 10. The number of carbon atoms of the branched alkoxy group, excluding the number of carbon atoms of a substituent, is usually 3 to 40, and preferably 4 to 10.

The alkoxy group optionally has a substituent, and examples thereof include a methoxy group, an ethoxy group, a propyloxy group, an isopropyloxy group, a butyloxy group, an isobutyloxy group, a tert-butyloxy group, a pentyloxy group, a hexyloxy group, a heptyloxy group, an octyloxy group, a 2-ethylhexyloxy group, a nonyloxy group, a decyloxy group, a 3,7-dimethyloctyloxy group and a lauryloxy group, and groups obtained by substituting a hydrogen atom in these groups with a cycloalkyl group, an alkoxy group, a cycloalkoxy group, an aryl group, a fluorine atom or the like.

The number of carbon atoms of "cycloalkoxy group", excluding the number of carbon atoms of a substituent, is usually 3 to 40, and preferably 4 to 10.

The cycloalkoxy group optionally has a substituent, and examples thereof include a cyclohexyloxy group.

The number of carbon atoms of "aryloxy group", excluding the number of carbon atoms of a substituent, is usually 6 to 60, and preferably 6 to 48.

The aryloxy group optionally has a substituent, and examples thereof include a phenoxy group, a 1-naphthyloxy group, a 2-naphthyloxy group, a 1-anthracenyloxy group, a 9-anthracenyloxy group, a 1-pyrenyloxy group, and groups obtained by substituting a hydrogen atom in these groups with an alkyl group, a cycloalkyl group, an alkoxy group, a cycloalkoxy group, a fluorine atom or the like.

"p-Valent heterocyclic group" (p represents an integer of 1 or more) means an atomic group remaining after removing from a heterocyclic compound p hydrogen atoms among hydrogen atoms directly linked to a carbon atom or a hetero atom constituting the ring. Of p-valent heterocyclic groups, preferred are "p-valent aromatic heterocyclic groups" as an atomic group remaining after removing from an aromatic heterocyclic compound p hydrogen atoms among hydrogen atoms directly linked to a carbon atom or a hetero atom constituting the ring.

"Aromatic heterocyclic compound" means a compound in which the heterocyclic ring itself shows aromaticity, such as oxadiazole, thiadiazole, thiazole, oxazole, thiophene, pyrrole, phosphole, furan, pyridine, pyrazine, pyrimidine, triazine, pyridazine, quinoline, isoquinoline, carbazole and dibenzophosphole, and a compound in which an aromatic ring is fused to the heterocyclic ring even if the heterocyclic ring itself shows no aromaticity, such as phenoxazine, phenothiazine, dibenzoborole, dibenzosilole and benzopyran.

The number of carbon atoms of the monovalent heterocyclic group, excluding the number of carbon atoms of a substituent, is usually 2 to 60, and preferably 4 to 20.

The monovalent heterocyclic group optionally has a substituent, and examples thereof include a thienyl group, a pyrrolyl group, a furyl group, a pyridinyl group, a piperidinyl group, a quinolinyl group, an isoquinolinyl group, a pyrimidinyl group, a triazinyl group, and groups obtained by substituting a hydrogen atom in these groups with an alkyl group, a cycloalkyl group, an alkoxy group, a cycloalkoxy group or the like.

"Halogen atom" means a fluorine atom, a chlorine atom, a bromine atom or an iodine atom.

"Amino group" optionally has a substituent, and a substituted amino group is preferable. The substituent which an amino group has is preferably an alkyl group, a cycloalkyl group, an aryl group or a monovalent heterocyclic group.

The substituted amino group includes, for example, a dialkylamino group, a dicycloalkylamino group and a diarylamino group.

The amino group includes, for example, a dimethylamino group, a diethylamino group, a diphenylamino group, a bis(4-methylphenyl)amino group, a bis(4-tert-butylphenyl)amino group and a bis(3,5-di-tert-butylphenyl)amino group.

"Alkenyl group" may be any of linear or branched. The number of carbon atoms of the linear alkenyl group, excluding the number of carbon atoms of the substituent, is usually 2 to 30, and preferably 3 to 20. The number of carbon atoms of the branched alkenyl group, excluding the number of carbon atoms of the substituent, is usually 3 to 30, and preferably 4 to 20.

The number of carbon atoms of "cycloalkenyl group", excluding the number of carbon atoms of the substituent, is usually 3 to 30, and preferably 4 to 20.

The alkenyl group and cycloalkenyl group each optionally have a substituent, and examples thereof include a vinyl group, a 1-propenyl group, a 2-propenyl group, a 2-butenyl group, a 3-butenyl group, a 3-pentenyl group, a 4-pentenyl group, a 1-hexenyl group, a 5-hexenyl group, a 7-octenyl group, and these groups having a substituent.

"Alkynyl group" may be any of linear or branched. The number of carbon atoms of the alkynyl group, excluding the number of carbon atoms of the substituent, is usually 2 to 20, preferably 3 to 20. The number of carbon atoms of the branched alkynyl group, excluding the number of carbon atoms of the substituent, is usually 4 to 30, and preferably 4 to 20.

The number of carbon atoms of "cycloalkynyl group", excluding the number of carbon atoms of the substituent, is usually 4 to 30, and preferably 4 to 20.

The alkynyl group and cycloalkynyl group each optionally have a substituent, and examples thereof include an ethynyl group, a 1-propynyl group, a 2-propynyl group, a 2-butynyl group, a 3-butynyl group, a 3-pentynyl group, a 4-pentynyl group, a 1-hexynyl group, a 5-hexynyl group, and these groups having a substituent.

"Arylene group" means an atomic group remaining after removing from an aromatic hydrocarbon two hydrogen atoms linked directly to carbon atoms constituting the ring. The number of carbon atoms of the arylene group, excluding the number of carbon atoms of a substituent, is usually 6 to 60, preferably 6 to 30, and more preferably 6 to 18.

The arylene group optionally has a substituent, and examples thereof include a phenylene group, a naphthalenediyl group, an anthracenediyl group, a phenanthrenediyl group, a dihydrophenanthrenediyl group, a naphthacenediyl group, a fluorenediyl group, a pyrenediyl group, a perylenediyl group, a chrysenediyl group, and these groups having a substituent, and preferably groups represented by formulas (A-1) to (A-20). The arylene group includes groups obtained by linking a plurality of these groups. 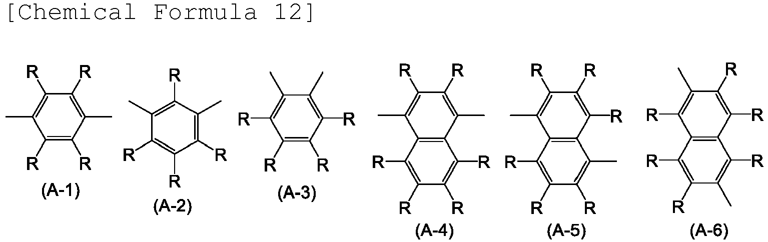 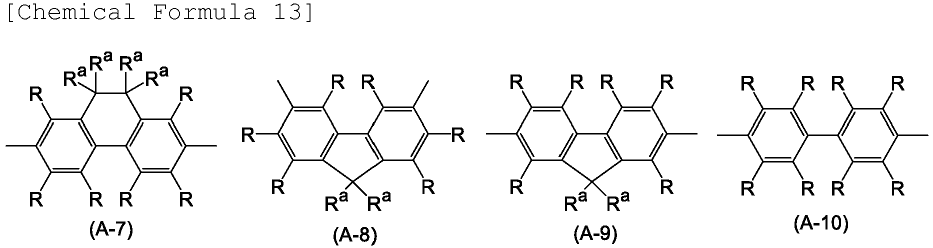 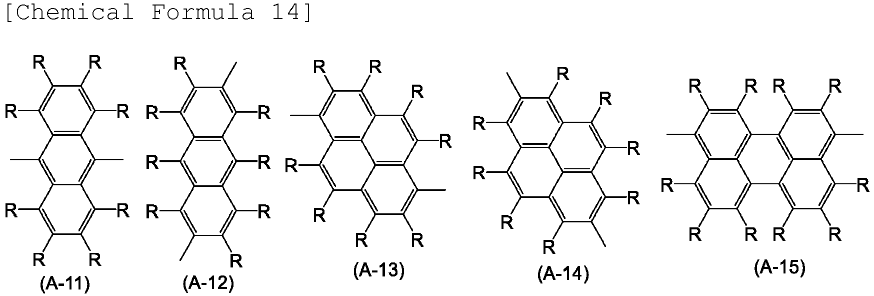 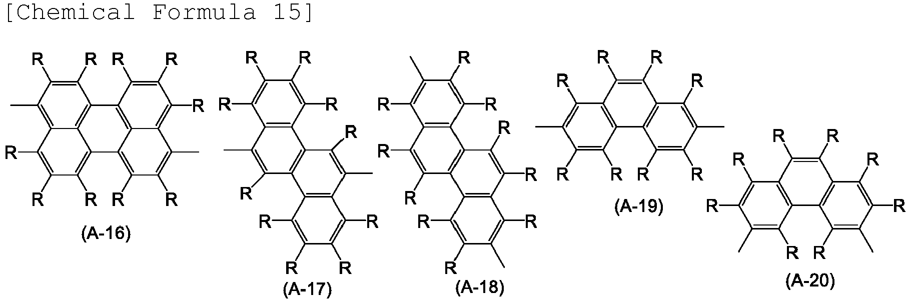 wherein R and R^{a} each independently represent a hydrogen atom, an alkyl group, a cycloalkyl group, an aryl group or a monovalent heterocyclic group, and the plurality of R and R^{a} each may be the same or different and groups R^{a} may be combined together to form a ring together with the atoms to which they are attached.

The number of carbon atoms of the divalent heterocyclic group, excluding the number of carbon atoms of a substituent, is usually 2 to 60, preferably 3 to 20, more preferably 4 to 15.

The divalent heterocyclic group optionally has a substituent, and examples thereof include divalent groups obtained by removing from pyridine, diazabenzene, triazine, azanaphthalene, diazanaphthalene, carbazole, dibenzofuran, dibenzothiophene, dibenzosilole, phenoxazine, phenothiazine, acridine, dihydroacridine, furan, thiophene, azole, diazole and triazole two hydrogen atoms among hydrogen atoms linking directly to a carbon atom or a hetero atom constituting the ring, and preferably groups represented by formulas (AA-1) to (AA-34). The divalent heterocyclic group includes groups obtained by linking a plurality of these groups. 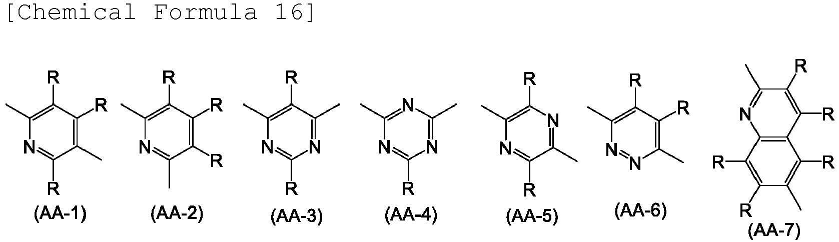 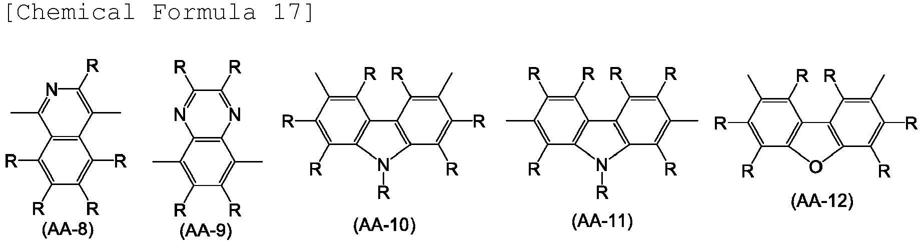 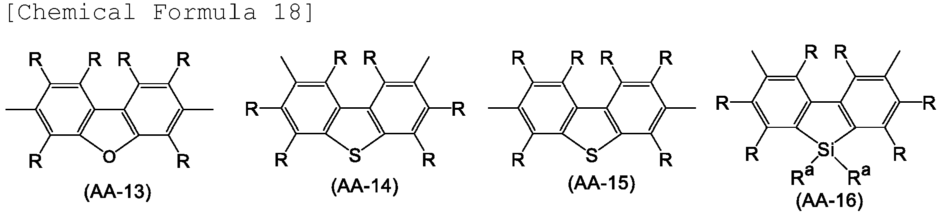 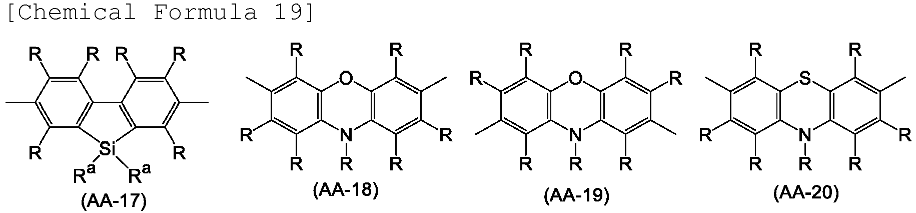 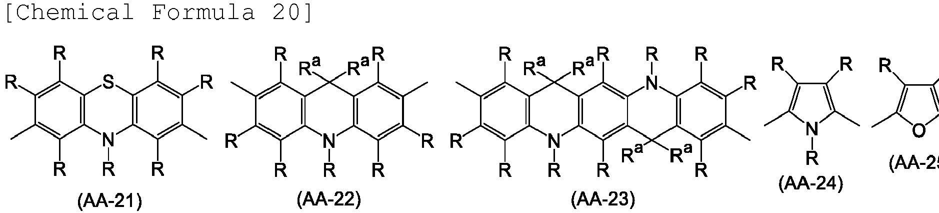 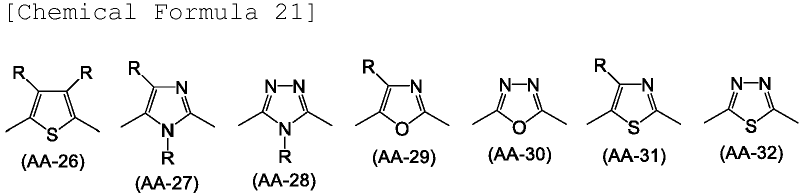 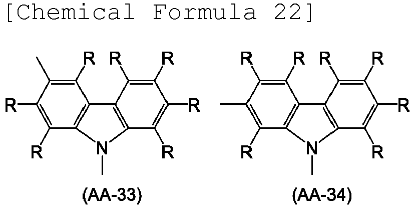 wherein R and R^{a} represent the same meaning as defined above.

"Crosslinkable group" is a group capable of forming a new bond by being subjected to heating, ultraviolet irradiation, near ultraviolet irradiation, visible light irradiation, infrared irradiation, a radical reaction and the like, and the crosslinkable groups are preferably groups represented by formulas (XL-1) to (XL-17) in Group A of crosslinkable group.

"Substituent" represents a halogen atom, a cyano group, an alkyl group, a cycloalkyl group, an aryl group, a monovalent heterocyclic group, an alkoxy group, a cycloalkoxy group, an aryloxy group, an amino group, a substituted amino group, an alkenyl group, a cycloalkenyl group, an alkynyl group or a cycloalkynyl group. The substituent may be a crosslinkable group.

### <Light Emitting Device>

The light emitting device of the present invention is a light emitting device comprising an anode, a cathode, a first organic layer disposed between the anode and the cathode, a second organic layer disposed between the anode and the cathode, wherein the first organic layer is a layer comprising a phosphorescent compound represented by formula (1) and a compound represented by formula (H), and the second organic layer is a layer comprising a crosslinked body of a crosslinkable material.

The method for forming a first organic layer and a second organic layer includes, for example, a dry process such as a vacuum deposition method, and a wet process such as a spin coating method and an ink-jet printing method, and the wet process is preferable.

When the first organic layer is formed by the wet process, it is preferable to use a first ink mentioned below.

When the second organic layer is formed by the wet process, it is preferable to use a second ink mentioned below. After forming the second organic layer, a crosslinkable material contained in the second organic layer can be crosslinked by heating or light irradiation, and it is preferable to crosslink the crosslinkable material contained in the second organic layer by heating. When a crosslinkable material in a crosslinked state (a crosslinked body of a crosslinkable material) is contained in the second organic layer, the second organic layer is substantially insolubilized in a solvent. For this reason, the second organic layer can be suitably used for lamination of a light emitting device.

The temperature of heating for crosslinking is usually 25°C to 300°C, preferably 50°C to 250°C, more preferably 150°C to 200°C, and still more preferably 170°C to 190°C.

The heating time is usually 0.1 minute to 1,000 minutes, preferably 0.5 minute to 500 minutes, more preferably 1 minute to 120 minutes, and still more preferably 30 minutes to 90 minutes.

Light used for light irradiation is, for example, ultraviolet light, near-ultraviolet light and visible light.

The method of analyzing components contained in the first organic layer or the second organic layer includes, for example, chemical separation analysis methods such as extraction, instrumental analysis methods such as IR spectroscopy (IR), nuclear magnetic resonance spectroscopy (NMR) and mass spectroscopy (MS), and analysis methods using chemical separation analysis methods and instrumental analysis methods in combination.

It is possible to separate into a component substantially insoluble in an organic solvent (insoluble component) and a component soluble in an organic solvent (soluble component) by subjecting the first organic layer or the second organic layer to solid-liquid extraction with an organic solvent such as toluene, xylene, chloroform or tetrahydrofuran. The resultant insoluble component can be analyzed by IR spectroscopy or nuclear magnetic resonance spectroscopy, and the resultant soluble component can be analyzed by nuclear magnetic resonance spectroscopy or mass spectroscopy.

### <First Organic Layer>

A first organic layer is a layer comprising a phosphorescent compound represented by formula (1) and a compound represented by formula (H).

### [Phosphorescent Compound Represented by Formula (1)]

A phosphorescent compound represented by formula (1) is a compound which usually shows phosphorescence at room temperature (25°C), and preferably a compound which shows light emission from a triplet excited state at room temperature.

The phosphorescent compound represented by formula (1) is constituted of central metal M, a ligand whose number is defined by a subscript n¹, and a ligand whose number is defined by a subscript n².

M is preferably an iridium atom or a platinum atom, and more preferably an iridium atom, because the light emitting device according to the embodiment of the present invention is excellent in luminance life.

When M is a ruthenium atom, a rhodium atom or an iridium atom, n¹ is preferably 2 or 3, and more preferably 3.

When M is a palladium atom or a platinum atom, n¹ is preferably 2.

E¹ and E² each are preferably a carbon atom.

The ring L¹ is preferably a 5-membered aromatic heterocyclic ring or a 6-membered aromatic heterocyclic ring, more preferably, a 5-membered aromatic heterocyclic ring having 2 or more and 4 or less nitrogen atoms as a constituent atom or a 6-membered aromatic heterocyclic ring having 1 or more and 4 or less nitrogen atoms as a constituent atom, and still more preferably a 5-membered aromatic heterocyclic ring having 2 or more and 3 or less nitrogen atoms as a constituent atom or a 6-membered aromatic heterocyclic ring having 1 or more and 2 or less nitrogen atoms as a constituent atom, and these rings each optionally have a substituent. When the ring L¹ is a 6-membered aromatic heterocyclic ring, E¹ is preferably a carbon atom.

The ring L¹ includes, for example, a diazole ring, a triazole ring, a pyridine ring, diazabenzene ring, a triazine ring, a quinoline ring and an isoquinoline ring, and is preferably a diazole ring, a triazole ring, a pyridine ring, a pyrimidine ring, a quinoline ring or an isoquinoline ring, more preferably a diazole ring, a triazole ring, a pyridine ring, a quinoline ring or an isoquinoline ring, still more preferably a pyridine ring, a quinoline ring or an isoquinoline ring, and particularly preferably a pyridine ring or an isoquinoline ring, and these rings each optionally have a substituent.

The ring L² is preferably a 5-membered or 6-membered aromatic hydrocarbon ring or a 5-membered or 6-membered aromatic heterocyclic ring, more preferably a 6-membered aromatic hydrocarbon ring or a 6-membered aromatic heterocyclic ring, and still more preferably a 6-membered aromatic hydrocarbon ring, and these rings each optionally have a substituent. When the ring R² is a 6-membered aromatic heterocyclic ring, E² is preferably a carbon atom.

The ring L² includes, for example, a benzene ring, a naphthalene ring, a fluorene ring, a phenanthrene ring, an indene ring, a pyridine ring, a diazabenzene ring and a triazine ring, and is preferably a benzene ring, a naphthalene ring, a fluorene ring, a pyridine ring or a pyrimidine ring, more preferably a benzene ring, a pyridine ring or a pyrimidine ring, and still more preferably a benzene ring, and these rings each optionally have a substituent.

The substituent which the ring L¹ and the ring L² optionally have is preferably an alkyl group, a cycloalkyl group, an alkoxy group, a cycloalkoxy group, an aryl group, an aryloxy group, a monovalent heterocyclic group, a substituted amino group or a halogen atom, more preferably an alkyl group, a cycloalkyl group, an alkoxy group, a cycloalkoxy group, an aryl group, a monovalent heterocyclic group or a fluorine atom, still more preferably an alkyl group, a cycloalkyl group, an aryl group or a monovalent heterocyclic group, and particularly preferably an aryl group or a monovalent heterocyclic group, and these groups each optionally further have a substituent.

The aryl group in the substituent which the ring L¹ and the ring L² optionally have is preferably a phenyl group, a naphthyl group, an anthracenyl group, a phenanthrenyl group, a dihydrophenanthrenyl group, a fluorenyl group or a pyrenyl group, more preferably a phenyl group, a naphthyl group or a fluorenyl group, and still more preferably a phenyl group, and these groups each optionally further have a substituent.

The monovalent heterocyclic group in the substituent which the ring L¹ and the ring L² optionally have is preferably a pyridyl group, a pyrimidinyl group, a triazinyl group, a quinolinyl group, an isoquinolinyl group, a dibenzofuranyl group, a dibenzothienyl group, a carbazolyl group, an azacarbazolyl group, a diazacarbazolyl group, a phenoxadinyl group or a phenothiadinyl group, more preferably a pyridyl group, a pyrimidinyl group, a triazinyl group, a carbazolyl group, an azacarbazolyl group or a diazacarbazolyl group, still more preferably a pyridyl group, a pyrimidinyl group or a triazinyl group, and particularly preferably a triazinyl group, and these groups each optionally further have a substituent.

In the substituted amino group in the substituent which the ring L¹ and the ring L² optionally have, the substituent which the amino group has is preferably an aryl group or a monovalent heterocyclic group, and more preferably an aryl group, and these groups each optionally further have a substituent. The examples and the preferable range of the aryl group in the substituent which the amino group has are the same as the examples and the preferable range of the aryl group in the substituent which the ring L¹ and the ring L² optionally have. The examples and the preferable range of the monovalent heterocyclic group in the substituent which the amino group has are the same as the examples and the preferable range of the monovalent heterocyclic group in the substituent which the ring L¹ and the ring L² optionally have.

The substituent which the substituent which the ring L¹ and the ring L² optionally have optionally further has is preferably an alkyl group, a cycloalkyl group, an alkoxy group, a cycloalkoxy group, an aryl group, an aryloxy group, a monovalent heterocyclic group, a substituted amino group or a halogen atom, more preferably an alkyl group, a cycloalkyl group, an alkoxy group, a cycloalkoxy group, an aryl group, a monovalent heterocyclic group or a fluorine atom, still more preferably an alkyl group, a cycloalkyl group, an aryl group or a monovalent heterocyclic group, and particularly preferably an alkyl group, a cycloalkyl group or an aryl group, and these groups each optionally further have a substituent.

The aryl group, the monovalent heterocyclic group or the substituted amino group in the substituent which the ring L¹ and the ring L² optionally have is preferably a group represented by formula (D-A), formula (D-B) or formula (D-C), more preferably a group represented by formula (D-A) or formula (D-B), and still more preferably a group represented by formula (D-A), because the light emitting device according to the embodiment of the present invention is more excellent in luminance life: 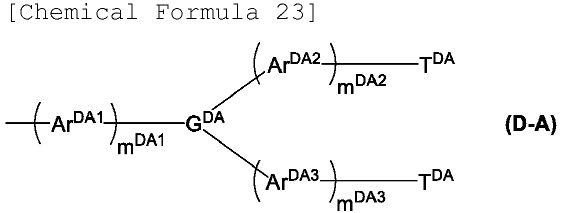 wherein
m^{DA1}, m^{DA2} and m^{DA3} each independently represent an integer of 0 or more,
G^{DA} represents a nitrogen atom, an aromatic hydrocarbon group or a heterocyclic group, and these groups each optionally have a substituent,
Ar^{DA1}, Ar^{DA2} and Ar^{DA3} each independently represent an arylene group or a divalent heterocyclic group, and these groups each optionally have a substituent, and when a plurality of Ar^{DA1}, Ar^{DA2} and Ar^{DA3} are present, they may be the same or different at each occurrence, and
T^{DA} represents an aryl group or a monovalent heterocyclic group, and these groups each optionally have a substituent, and the plurality of T^{DA} may be the same or different: 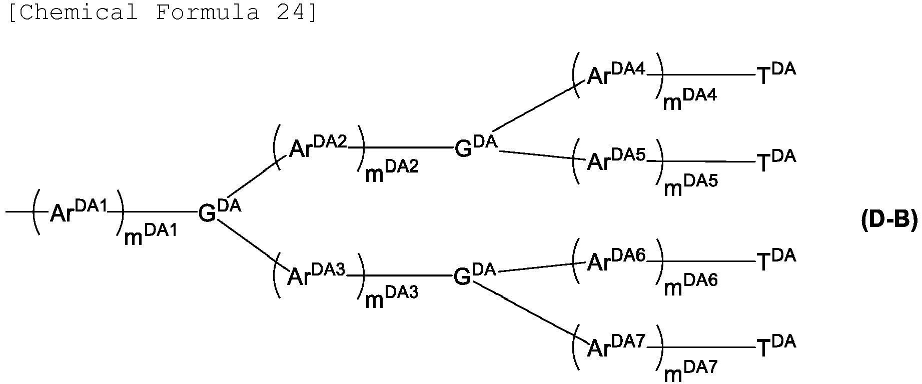
wherein
m^{DA1}, m^{DA2}, m^{DA3}, m^{DA4}, m^{DA5}, m^{DA6} and m^{DA7} each independently represent an integer of 0 or more,
G^{DA} represents a nitrogen atom, an aromatic hydrocarbon group or a heterocyclic group, and these groups each optionally have a substituent, and the plurality of G^{DA} may be the same or different,
Ar^{DA1}, Ar^{DA2}, Ar^{DA3}, Ar^{DA4}, Ar^{DA5}, Ar^{DA6} and Ar^{DA7} each independently represent an arylene group or a divalent heterocyclic group, and these groups each optionally have a substituent, and when a plurality of Ar^{DA1}, Ar^{DA2}, Ar^{DA3}, Ar^{DA4}, Ar^{DA5}, Ar^{DA6} and Ar^{DA7} are present, they may be the same or different at each occurrence, and
T^{DA} represents an aryl group or a monovalent heterocyclic group, and these groups each optionally have a substituent, and the plurality of T^{DA} may be the same or different: 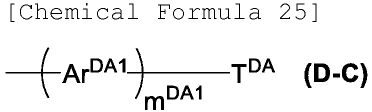
wherein
m^{DA1} represents an integer of 0 or more,
Ar^{DA1} represents an arylene group or a divalent heterocyclic group, and these groups each optionally have a substituent, and when a plurality of Ar^{DA1} are present, they may be the same or different, and
T^{DA} represents an aryl group or a monovalent heterocyclic group, and these groups each optionally have a substituent.

m^{DA1}, m^{DA2}, m^{DA3}, m^{DA4}, m^{DA5}, m^{DA6} and m^{DA7} represent usually an integer of 10 or less, preferably an integer of 5 or less, more preferably an integer of 2 or less, and still more preferably 0 or 1. It is preferable that m^{DA2}, m^{DA3}, m^{DA4}, m^{DA5}, m^{DA6} and m^{DA7} are preferably the same integer.

G^{DA} is preferably an aromatic hydrocarbon group or a heterocyclic group, and more preferably a group obtained by removing from a benzene ring, a pyridine ring, a pyrimidine ring, a triazine ring or a carbazole ring three hydrogen atoms linked directly to a carbon atom or a nitrogen atom constituting the ring, and these groups each optionally have a substituent.

The substituent which G^{DA} optionally has is preferably an alkyl group, a cycloalkyl group, an alkoxy group, a cycloalkoxy group, an aryl group or a monovalent heterocyclic group, more preferably an alkyl group, a cycloalkyl group, an alkoxy group or a cycloalkoxy group, and still more preferably an alkyl group or a cycloalkyl group, and these groups each optionally have a substituent.

G^{DA} is preferably a group represented by formula (GDA-11) to formula (GDA-15), more preferably a group represented by formula (GDA-11) to formula (GDA-14), and still more preferably a group represented by formula (GDA-11) or formula (GDA-14) : 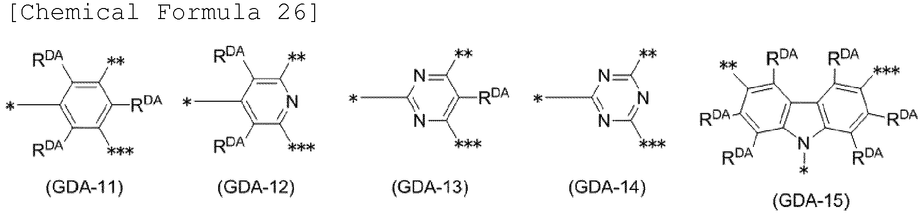 wherein
* represents a bond to Ar^{DA1} in formula (D-A), Ar^{DA1} in formula (D-B), Ar^{DA2} in formula (D-B) or Ar^{DA3} in formula (D-B),
** represents a bond to Ar^{DA2} in formula (D-A), Ar^{DA2} in formula (D-B), Ar^{DA4} in formula (D-B) or Ar^{DA6} in formula (D-B),
*** represents a bond to Ar^{DA3} in formula (D-A), Ar^{DA3} in formula (D-B), Ar^{DA5} in formula (D-B) or Ar^{DA7} in formula (D-B), and
R^{DA} represents a hydrogen atom, an alkyl group, a cycloalkyl group, an alkoxy group, a cycloalkoxy group, an aryl group or a monovalent heterocyclic group, and these groups each optionally have a substituent, and when a plurality of R^{DA} are present, they may be the same or different.

R^{DA} is preferably a hydrogen atom, an alkyl group, a cycloalkyl group, an alkoxy group or a cycloalkoxy group, and more preferably a hydrogen atom, an alkyl group or a cycloalkyl group, and these groups each optionally have a substituent.

Ar^{DA1}, Ar^{DA2}, Ar^{DA3}, Ar^{DA4}, Ar^{DA5}, Ar^{DA6} and Ar^{DA7} represent preferably a phenylene group, a fluorenediyl group or a carbazolediyl group, more preferably a group represented by formula (ArDA-1) to formula (ArDA-5), still more preferably a group represented by formula (ArDA-1) to formula (ArDA-3), particularly preferably a group represented by formula (ArDA-1) or formula (ArDA-2), and especially preferably a group represented by formula (ArDA-2), and these groups each optionally have a substituent: 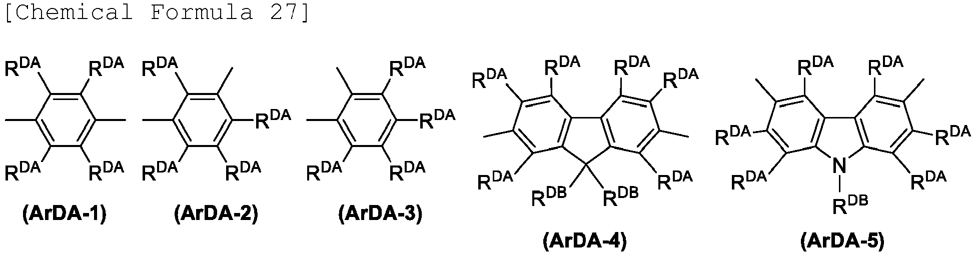 wherein
R^{DA} represents the same meaning as defined above, and
R^{DB} represents a hydrogen atom, an alkyl group, a cycloalkyl group, an aryl group or a monovalent heterocyclic group, and these groups each optionally have a substituent, and when a plurality of R^{DB} are present, they may be the same or different.

R^{DB} is preferably an alkyl group, a cycloalkyl group, an aryl group or a monovalent heterocyclic group, more preferably an aryl group or a monovalent heterocyclic group, and still more preferably an aryl group, and these groups each optionally have a substituent.

The examples and the preferable range of the substituent which Ar^{DA1}, Ar^{DA2}, Ar^{DA3}, Ar^{DA4}, Ar^{DA5}, Ar^{DA6} and Ar^{DA7} optionally have are the same as the examples and the preferable range of the substituent which G^{DA} optionally has.

T^{DA} represents preferably a group represented by formula (TDA-1) to formula (TDA-3), and more preferably a group represented formula (TDA-1): 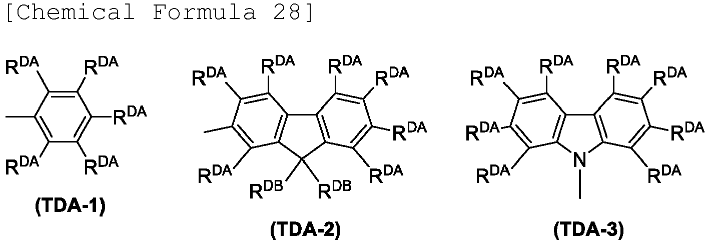 wherein R^{DA} and R^{DB} represent the same meaning as defined above.

The group represented formula (D-A) is preferably a group represented by formula (D-A1) to formula (D-A5), more preferably a group represented by formula (D-A1) or formula (D-A3) to formula (D-A5), and still more preferably a group represented by formula (D-A1), formula (D-A3) or formula (D-A5) : 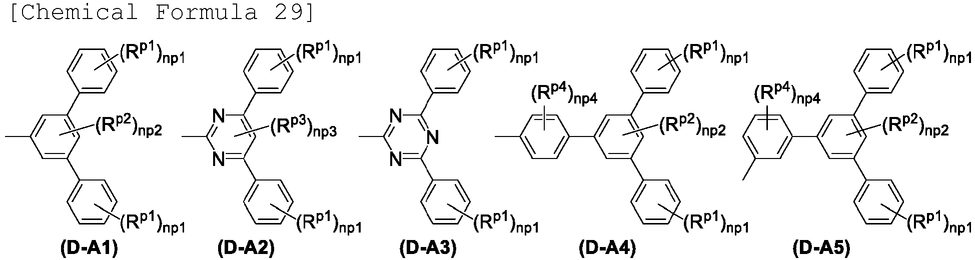 wherein
R^{p1}, R^{p2}, R^{p3} and R^{p4} each independently represent an alkyl group, a cycloalkyl group, an alkoxy group, a cycloalkoxy group or a halogen atom, and when a plurality of R^{p1}, R^{p2} and R^{p4} are present, they may be the same or different at each occurrence, and
np1 represents an integer of 0 to 5, np2 represents an integer of 0 to 3, np3 represents 0 or 1, and np4 represents an integer of 0 to 4, and the plurality of np1 may be the same or different.

The group represented by formula (D-B) is preferably a group represented by formula (D-B1) to formula (D-B6), more preferably a group represented by formula (D-B1) to formula (D-B3), formula (D-B5) or formula (D-B6), still more preferably a group represented by formula (D-B1), formula (D-B3) or formula (D-B5), and particularly preferably a group represented by formula (D-B1): 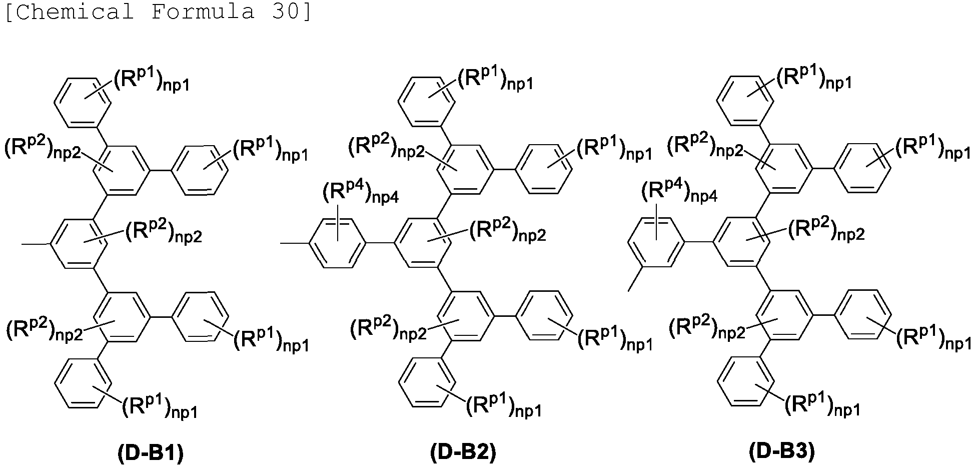 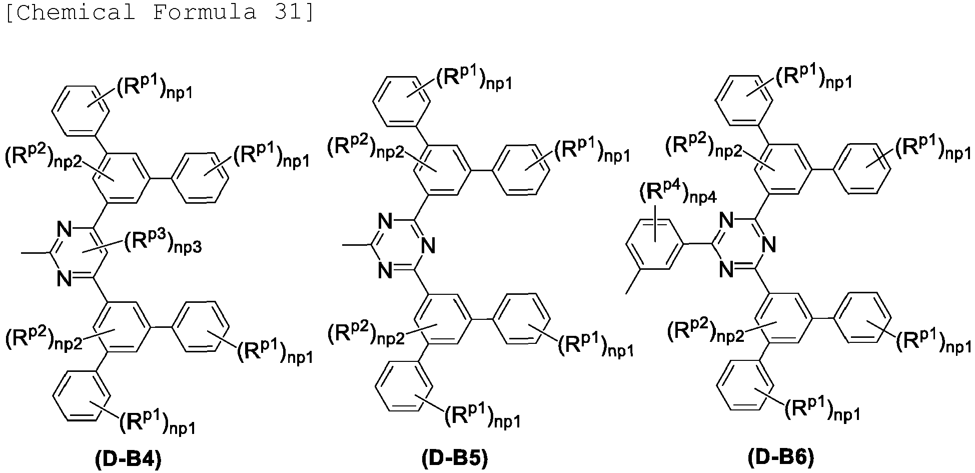 wherein
R^{p1}, R^{p2}, R^{p3} and R^{p4} each independently represent an alkyl group, a cycloalkyl group, an alkoxy group, a cycloalkoxy group or a halogen atom, and when a plurality of R^{p1}, R^{p2} and R^{p4} are present, they may be the same or different at each occurrence, and
np1 represents an integer of 0 to 5, np2 represents an integer of 0 to 3, np3 represents 0 or 1, np4 represents an integer of 0 to 4, the plurality of np1 may be the same or different, and the plurality of np2 may be the same or different.

The group represented by formula (D-C) is preferably a group represented by formula (D-C1) to formula (D-C4), more preferably a group represented by formula (D-C1) to formula (D-C3), still more preferably a group represented by formula (D-C1) or formula (D-C2), and particularly preferably a group represented by formula (D-C1): 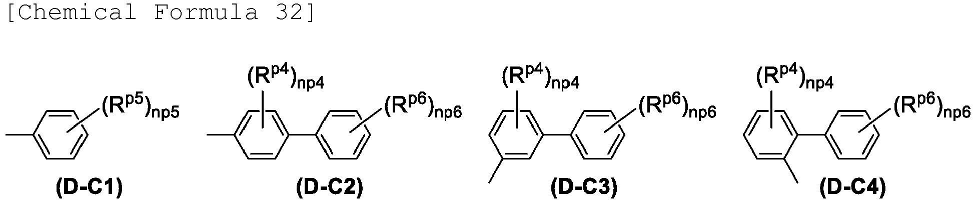 wherein
R^{p4}, R^{p5} and R^{p6} each independently represent an alkyl group, a cycloalkyl group, an alkoxy group, a cycloalkoxy group or a halogen atom, and when a plurality of R^{p4}, R^{p5} and R^{p6} are present, they may be the same or different at each occurrence, and
np4 represents an integer of 0 to 4, np5 represents an integer of 0 to 5, and np6 represents an integer of 0 to 5.

np1 is preferably an integer of 0 to 2, and more preferably 0 or 1. np2 is preferably 0 or 1, and more preferably 0. np3 is preferably 0. np4 is preferably an integer of 0 to 2, and more preferably 0. np5 is preferably an integer of 0 to 3, and more preferably 0 or 1. np6 is preferably an integer of 0 to 2, and more preferably 0 or 1.

The alkyl group or cycloalkyl group in R^{p1}, R^{p2}, _{R}^{p3}, R^{p4}, R^{p5} and R^{p6} is preferably a methyl group, an ethyl group, an isopropyl group, a tert-butyl group, a hexyl group, a 2-ethylhexyl group, a cyclohexyl group or a tert-octyl group.

The alkoxy group or cycloalkoxy group in R^{p1}, R^{p2}, R^{p3}, R^{p4}, R^{p5} and R^{p6} is preferably a methoxy group, a 2-ethylhexyloxy group or a cyclohexyloxy group.

R^{p1}, R^{p2}, R^{p3}, R^{p4}, R^{p5} and R^{p6} preferably represent an alkyl group optionally having a substituent or a cycloalkyl group optionally having a substituent, more preferably an alkyl group optionally having a substituent, still more preferably a methyl group, an ethyl group, an isopropyl group, a tert-butyl group, a hexyl group, a 2-ethylhexyl group or a tert-octyl group.

The group represented by formula (D-A) includes, for example, groups represented by formula (D-A-1) to formula (D-A-12) : 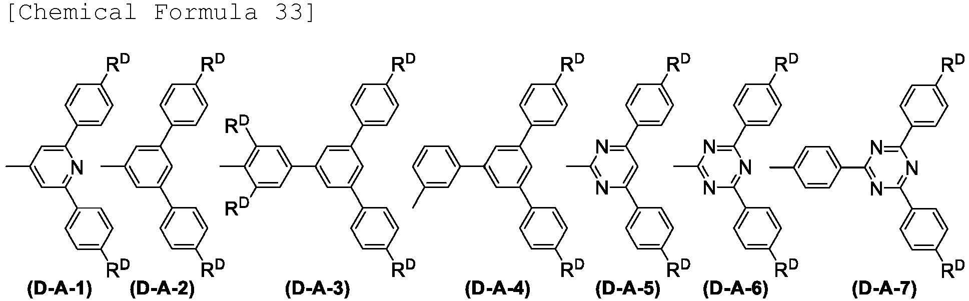 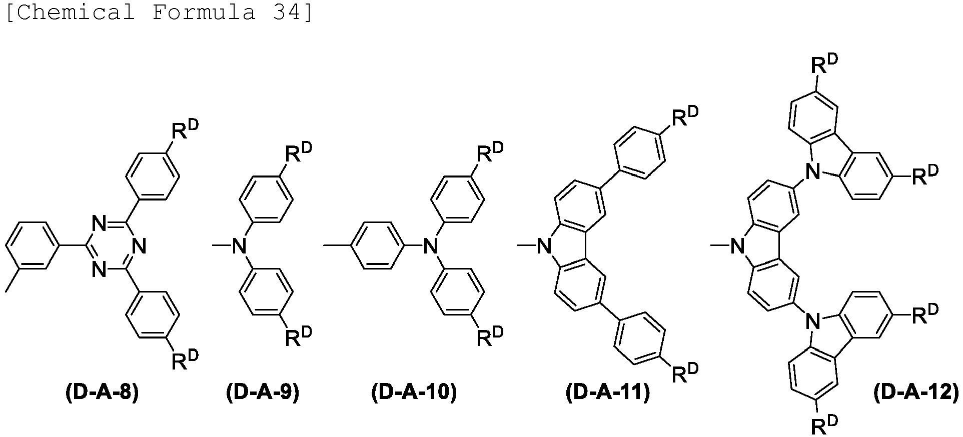 wherein R^{D} represents a hydrogen atom, a methyl group, an ethyl group, an isopropyl group, a tert-butyl group, a hexyl group, a 2-ethylhexyl group, a tert-octyl group, a cyclohexyl group, a methoxy group, a 2-ethylhexyloxy group or a cyclohexyloxy group, and when a plurality of R^{D} are present, they may be the same or different.

The group represented by formula (D-B) includes, for example, groups represented by formula (D-B-1) to formula (D-B-7) : 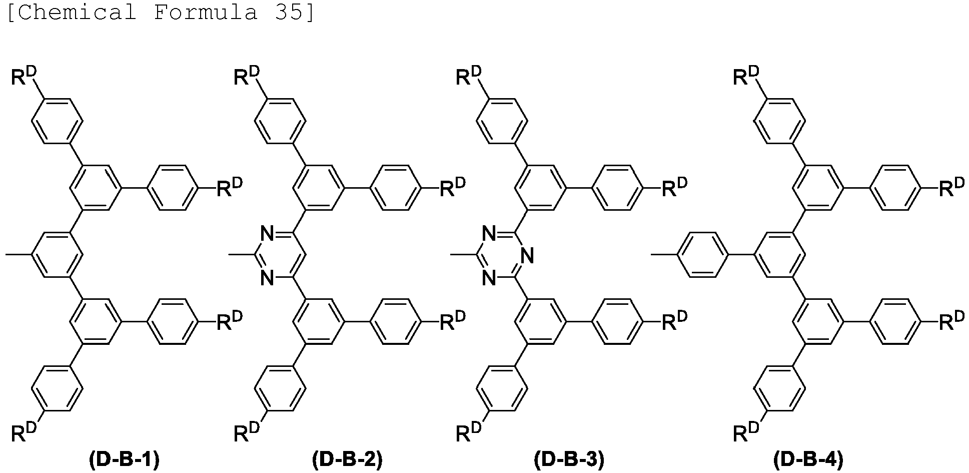 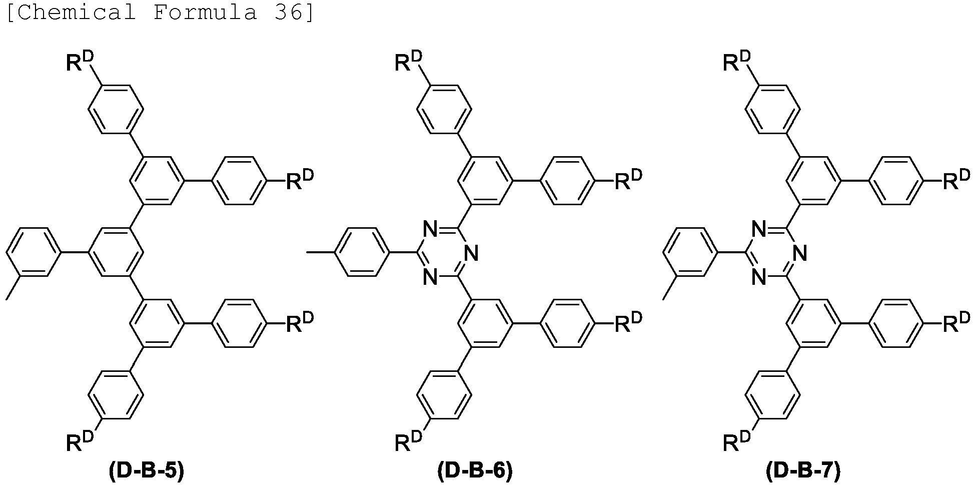 wherein R^{D} represents the same meaning as defined above.

The group represented by formula (D-C) includes, for example, groups represented by formula (D-C-1) to formula (D-C-13) : 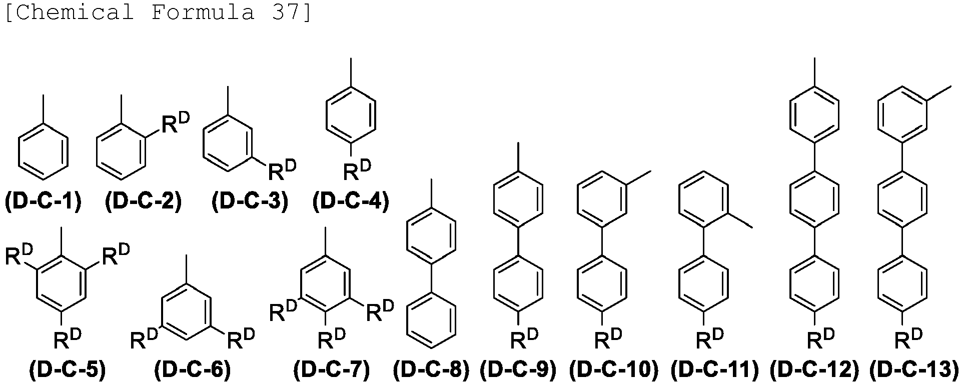 wherein R^{D} represents the same meaning as defined above.

R^{D} is preferably a hydrogen atom, a methyl group, an ethyl group, an isopropyl group, a tert-butyl group, a hexyl group, a 2-ethylhexyl group or a tert-octyl group, and more preferably a hydrogen atom, a tert-butyl group or a tert-octyl group.

When a plurality of the substituents which the ring L¹ optionally has are present, they may be the same or different and may be combined together to form a ring together with the atoms to which they are attached.

When a plurality of the substituents which the ring L² optionally has are present, they may be the same or different and may be combined together to form a ring together with the atoms to which they are attached.

The substituent which the ring L¹ optionally has and the substituent which the ring L² optionally has each may be combined together to form a ring together with the atoms to which they are attached.

### [Anionic Bidentate Ligand]

The anionic bidentate ligand represented by A¹-G¹-A² includes, for example, ligands represented by the following formulas, and the anionic bidentate ligand represented by A¹-G¹-A² is different from the ligand whose number is defined by a subscript n¹: 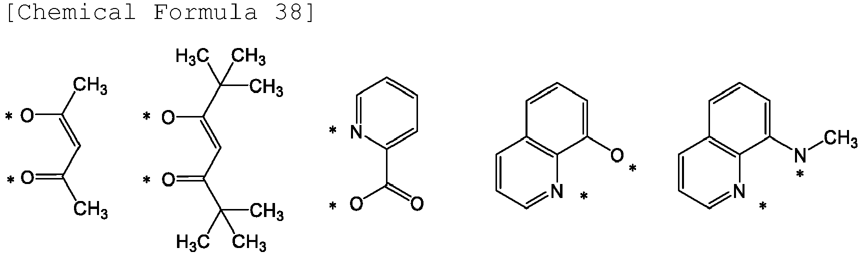 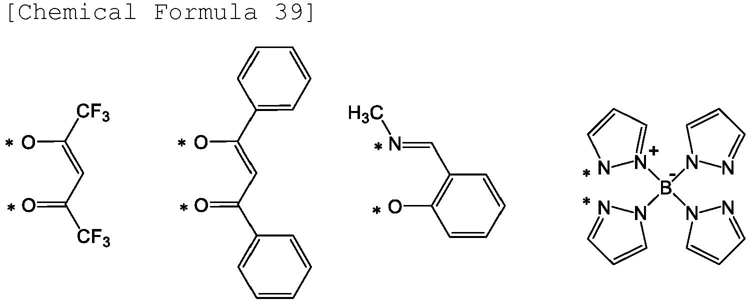 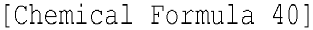 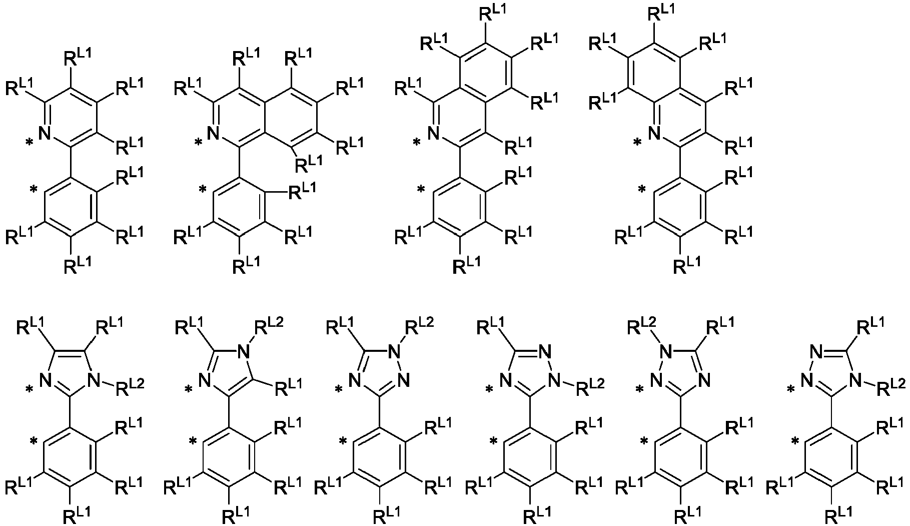 wherein
* represents a site binding to M,
R^{L1} represents a hydrogen atom, an alkyl group, a cycloalkyl group, an aryl group, a monovalent heterocyclic group or a halogen atom, and these groups each optionally have a substituent, and the plurality of R^{L1} may be the same or different, and
R^{L2} represents an alkyl group, a cycloalkyl group, an aryl group, a monovalent heterocyclic group or a halogen atom, and these groups each optionally have a substituent.

R^{L1} is preferably a hydrogen atom, an alkyl group, a cycloalkyl group, an aryl group or a fluorine atom, and more preferably a hydrogen atom or an alkyl group, and these groups each optionally have a substituent.

R^{L2} is preferably an alkyl group or an aryl group, and these groups each optionally have a substituent.

The phosphorescent compound represented by formula (1) is preferably a phosphorescent compound represented by formula (1-A) or formula (1-B), and more preferably a phosphorescent compound represented by formula (1-B), because the light emitting device according to the embodiment of the present invention is excellent in luminance life.

### [Phosphorescent Compound Represented by Formula (1-A)]

When the ring L^{1A} is a diazole ring, preferred is an imidazole ring in which E^{11A} is a nitrogen atom or an imidazole ring in which E^{12A} is a nitrogen atom, and more preferred is an imidazole ring in which E^{11A} is a nitrogen atom.

When the ring L^{1A} is a triazole ring, preferred is a triazole ring in which E^{11A} and E^{12A} represent a nitrogen atom or a triazole ring in which E^{11A} and E^{13A} represent a nitrogen atom, and more preferred is a triazole ring in which E^{11A} and E^{12A} represent a nitrogen atom.

The examples and the preferable range of the aryl group, the monovalent heterocyclic group and the substituted amino group in R^{11A}, R^{12A}, R^{13A}, R^{21A}, R^{22A}, R^{23A} and R^{24A} are the same as the examples and the preferable range of the aryl group, the monovalent heterocyclic group and the substituted amino group as the substituent which the ring L¹ and the ring L² optionally have, respectively.

The examples and the preferable range of the substituent which R^{11A}, R^{12A}, R^{13A}, R^{21A}, R^{22A}, R^{23A} and R^{24A} optionally have are the same as the examples and the preferable range of the substituent which the substituent which the ring L¹ and the ring L² optionally have further optionally has.

When E^{11A} is a nitrogen atom and R^{11A} is present, R^{11A} is preferably an alkyl group, a cycloalkyl group, an aryl group or a monovalent heterocyclic group, more preferably an aryl group or a monovalent heterocyclic group, and still more preferably an aryl group, and these groups each optionally have a substituent.

When E^{11A} is a carbon atom, R^{11A} is preferably a hydrogen atom, an alkyl group, a cycloalkyl group, an aryl group, a monovalent heterocyclic group or a substituted amino group, more preferably a hydrogen atom, an alkyl group, a cycloalkyl group or an aryl group, still more preferably a hydrogen atom, an alkyl group or a cycloalkyl group, and particularly preferably a hydrogen atom, and these groups each optionally have a substituent.

When E^{12A} is a nitrogen atom and R^{12A} is present, R^{12A} is preferably an alkyl group, a cycloalkyl group, an aryl group or a monovalent heterocyclic group, more preferably an aryl group or a monovalent heterocyclic group, and still more preferably an aryl group, and these groups each optionally have a substituent.

When E^{12A} is a carbon atom, R^{12A} is preferably a hydrogen atom, an alkyl group, a cycloalkyl group, an aryl group, a monovalent heterocyclic group or a substituted amino group, more preferably a hydrogen atom, an alkyl group, a cycloalkyl group or an aryl group, still more preferably a hydrogen atom, an alkyl group or a cycloalkyl group, and particularly preferably a hydrogen atom, and these groups each optionally have a substituent.

When E^{13a} is a nitrogen atom and R^{13A} is present, R^{13A} is preferably an alkyl group, a cycloalkyl group, an aryl group or a monovalent heterocyclic group, more preferably an aryl group or a monovalent heterocyclic group, and still more preferably an aryl group, and these groups each optionally have a substituent.

When E^{13a} is a carbon atom, R^{13A} is preferably a hydrogen atom, an alkyl group, a cycloalkyl group, an aryl group, a monovalent heterocyclic group or a substituted amino group, more preferably a hydrogen atom, an alkyl group, a cycloalkyl group or an aryl group, still more preferably a hydrogen atom, an alkyl group or a cycloalkyl group, and particularly preferably a hydrogen atom, and these groups each optionally have a substituent.

When the ring L^{2A} is a pyridine ring, preferred is a pyridine ring in which E^{21A} is a nitrogen atom, a pyridine ring in which E^{22A} is a nitrogen atom, or a pyridine ring in which E^{23A} is a nitrogen atom, and more preferred is a pyridine ring in which E^{22A} is a nitrogen atom.

When the ring L^{2A} is a pyrimidine ring, preferred is a pyrimidine ring in which E^{22A} and E^{24A} represent a nitrogen atom.

The ring L^{2A} is preferably a benzene ring.

R^{21A}, R^{22A}, R^{23A} and R^{24A} represent preferably a hydrogen atom, an alkyl group, a cycloalkyl group, an alkoxy group, a cycloalkoxy group, an aryl group, a monovalent heterocyclic group, a fluorine atom or a substituted amino group, more preferably a hydrogen atom, an alkyl group, a cycloalkyl group, an aryl group, a monovalent heterocyclic group or a substituted amino group, still more preferably a hydrogen atom, an alkyl group, a cycloalkyl group or a group represented by formula (D-A), formula (D-B) or formula (D-C), particularly preferably a hydrogen atom or a group represented by formula (D-A), formula (D-B) or formula (D-C), and especially preferably a hydrogen atom or a group represented by formula (D-A), and these groups each optionally have a substituent.

When the ring L^{2A} has an aryl group, a monovalent heterocyclic group or a substituted amino group, R^{22A} or R^{23A} is preferably an aryl group, a monovalent heterocyclic group or a substituted amino group, and R^{22A} is more preferably an aryl group, a monovalent heterocyclic group or a substituted amino group.

The phosphorescent compound represented by formula (1-A) is preferably a phosphorescent compound represented by formula (1-A1), a phosphorescent compound represented by formula (1-A2), a phosphorescent compound represented by formula (1-A3) or a phosphorescent compound represented by formula (1-A4), more preferably a phosphorescent compound represented by formula (1-A1) or a phosphorescent compound represented by formula (1-A3), and still more preferably a phosphorescent compound represented by formula (1-A1), because the light emitting device according to the embodiment of the present invention is more excellent in luminance life: 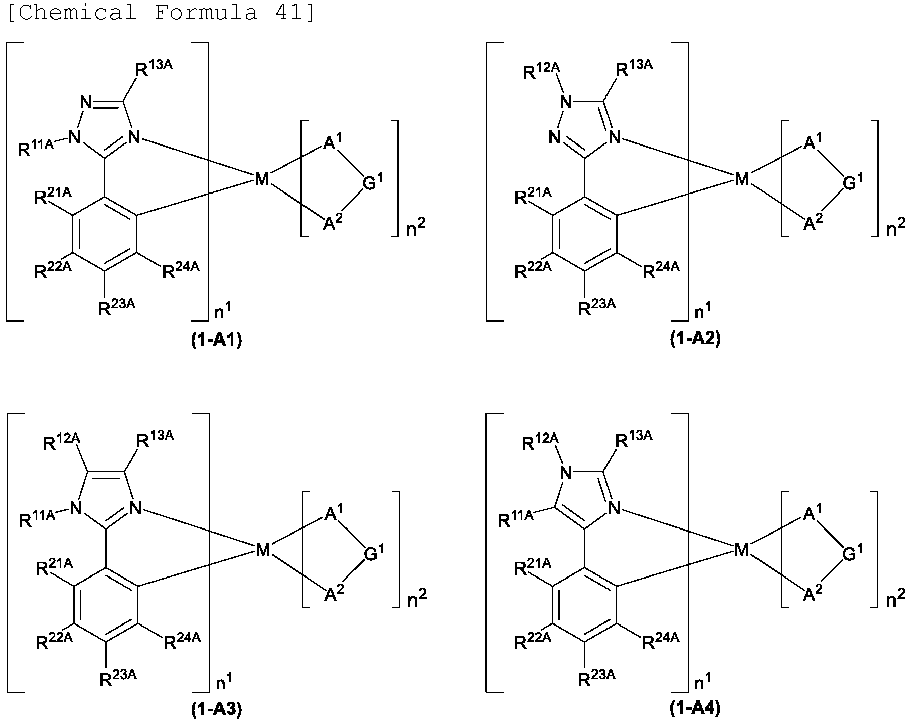 wherein
M, n¹, n², R^{11A}, R^{12A}, R^{13A}, R^{21A}, R^{22A}, R^{23A}, R^{24A} and A¹-G¹-A² represent the same meaning as defined above.

### [Phosphorescent Compound Represented by Formula (1-B)]

When the ring L^{1B} is a pyrimidine ring, preferred is a pyrimidine ring in which E^{11B} is a nitrogen atom.

When the ring L^{2B} is a pyridine ring, preferred is a pyridine ring in which E^{21B} is a nitrogen atom, a pyridine ring in which E^{22B} is a nitrogen atom or a pyridine ring in which E^{23B} is a nitrogen atom, and more preferred is a pyridine ring in which E^{22B} is a nitrogen atom.

When the ring L^{2B} is a pyrimidine ring, preferred is a pyrimidine ring in which E^{22B} and E^{24B} represent a nitrogen atom.

The ring L^{2B} is preferably a benzene ring.

The examples and the preferable range of the aryl group, the monovalent heterocyclic group and the substituted amino group in R^{11B}, R^{12B}, R^{13B}, R^{14B}, R^{21B}, R^{22B}, R^{23B} and R^{24B} are the same as the examples and the preferable range of the aryl group, the monovalent heterocyclic group and the substituted amino group as the substituent which the ring L¹ and the ring L² optionally have, respectively.

The examples and the preferable range of the substituent which R^{11B}, R^{12B}, R^{13B}, R^{14B}, R^{21B}, R^{22B}, R^{23B} and R^{24B} optionally have are the same as the examples and the preferable range of the substituent which the substituent which the ring L¹ and the ring L² optionally have further optionally has.

R^{11B}, R^{12B}, R^{13B}, R^{14B}, R^{21B}, R^{22B}, R^{23B} and R^{24B} represent preferably a hydrogen atom, an alkyl group, a cycloalkyl group, an alkoxy group, a cycloalkoxy group, a fluorine atom, an aryl group, a monovalent heterocyclic group or a substituted amino group, more preferably a hydrogen atom, an alkyl group, a cycloalkyl group, an aryl group, a monovalent heterocyclic group or a substituted amino group, still more preferably a hydrogen atom, an alkyl group, a cycloalkyl group or a group represented by formula (D-A), formula (D-B) or formula (D-C), and particularly preferably a hydrogen atom or a group represented by formula (D-A), formula (D-B) or formula (D-C), and these groups each optionally have a substituent.

When the ring L^{1B} has an aryl group, a monovalent heterocyclic group or a substituted amino group, it is preferable that R^{11B}, R^{12B} or R^{13B} is an aryl group, a monovalent heterocyclic group or a substituted amino group, it is more preferable that R^{12B} or R^{13B} is an aryl group, a monovalent heterocyclic group or a substituted amino group, and it is still more preferable that R^{13B} is an aryl group, a monovalent heterocyclic group or a substituted amino group.

When the ring L^{2B} has an aryl group, a monovalent heterocyclic group or a substituted amino group, it is preferable that R^{22B} or R^{23B} is an aryl group, a monovalent heterocyclic group or a substituted amino group, and it is more preferable that R^{22B} is an aryl group, a monovalent heterocyclic group or a substituted amino group.

The phosphorescent compound represented by formula (1-B) is preferably a phosphorescent compound represented by formula (1-B1), a phosphorescent compound represented by formula (1-B2), a phosphorescent compound represented by formula (1-B3), a phosphorescent compound represented by formula (1-B4) or a phosphorescent compound represented by formula (1-B5), more preferably a phosphorescent compound represented by formula (1-B1), a phosphorescent compound represented by formula (1-B2) or a phosphorescent compound represented by formula (1-B3), still more preferably a phosphorescent compound represented by formula (1-B1) or a phosphorescent compound represented by formula (1-B3), and particularly preferably a phosphorescent compound represented by formula (1-B1), because the light emitting device according to the embodiment of the present invention is more excellent in luminance life.

The examples and the preferable range of the aryl group, the monovalent heterocyclic group and the substituted amino group in R^{15B}, R^{16B}, R^{17B} and R^{18B} are the same as the examples and the preferable range of the aryl group, the monovalent heterocyclic group and the substituted amino group as the substituent which the ring L¹ and the ring L² optionally have, respectively.

The examples and the preferable range of the substituent which R^{15B}, R^{16B}, R^{17B} and R^{18B} optionally have are the same as the examples and the preferable range of the substituent which the substituent which the ring L¹ and the ring L² optionally have further optionally has.

R^{15B}, R^{16B}, R^{17B} and R^{18B} represent preferably a hydrogen atom, an alkyl group, a cycloalkyl group, an alkoxy group, a cycloalkoxy group, a fluorine atom, an aryl group, a monovalent heterocyclic group or a substituted amino group, more preferably a hydrogen atom, an alkyl group, a cycloalkyl group, an alkoxy group, a cycloalkoxy group, an aryl group, a monovalent heterocyclic group or a substituted amino group, still more preferably a hydrogen atom, an alkyl group, a cycloalkyl group, an aryl group, a monovalent heterocyclic group or a substituted amino group, particularly preferably a hydrogen atom, an alkyl group or a cycloalkyl group, and especially preferably a hydrogen atom, and these groups each optionally have a substituent.

The phosphorescent compound represented by formula (1) includes, for example, phosphorescent compounds represented by the following formulas.

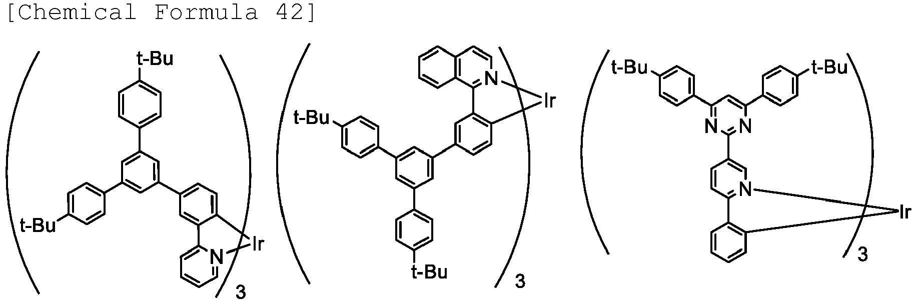

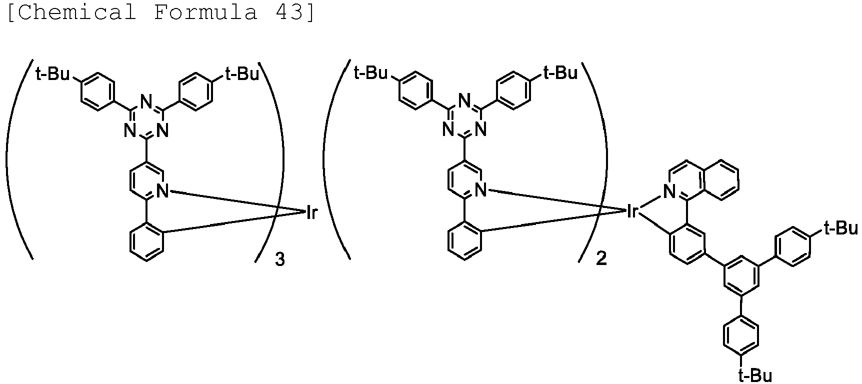

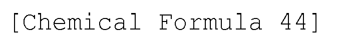

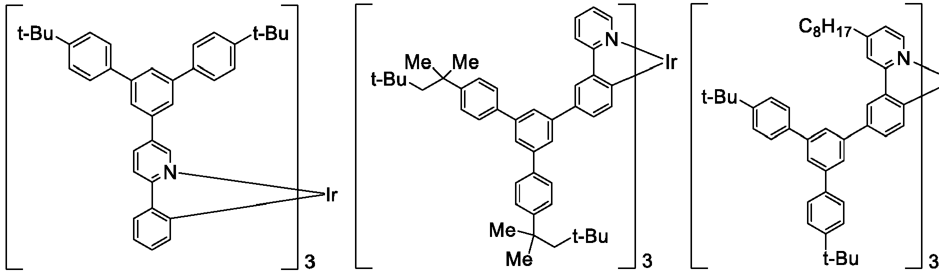

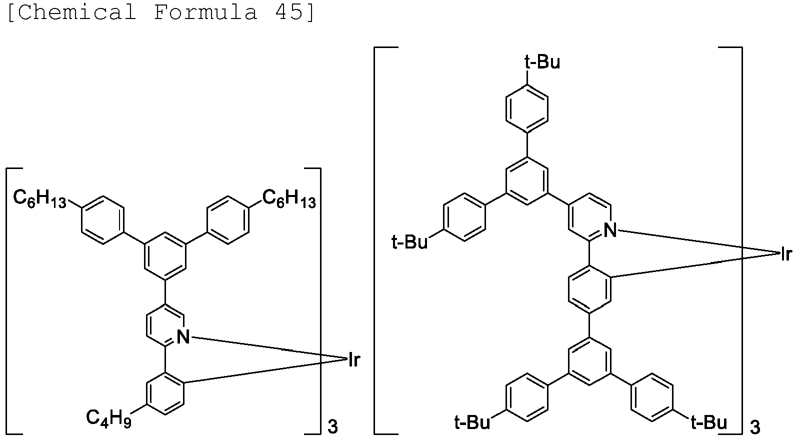

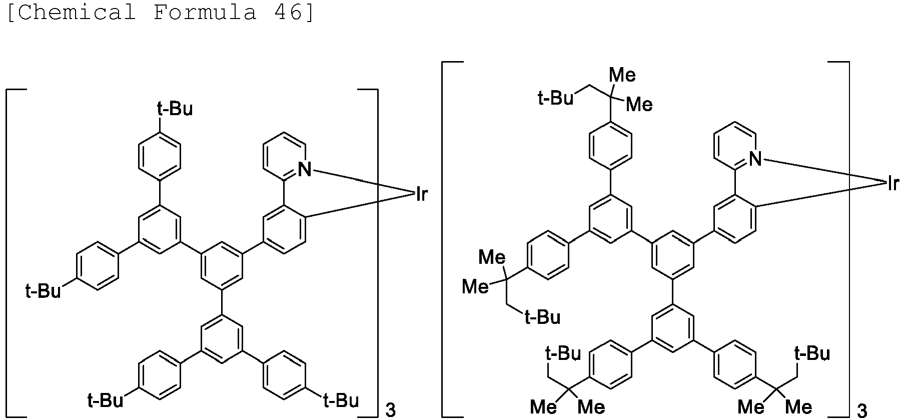

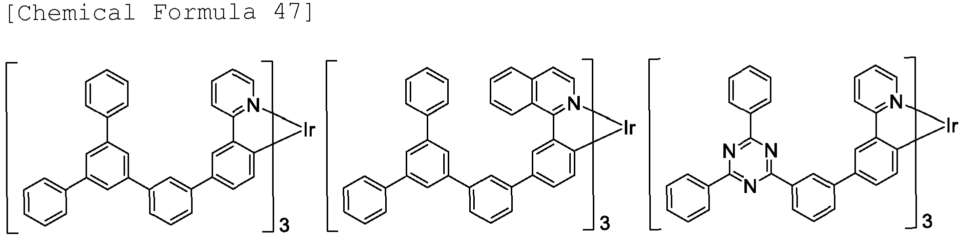

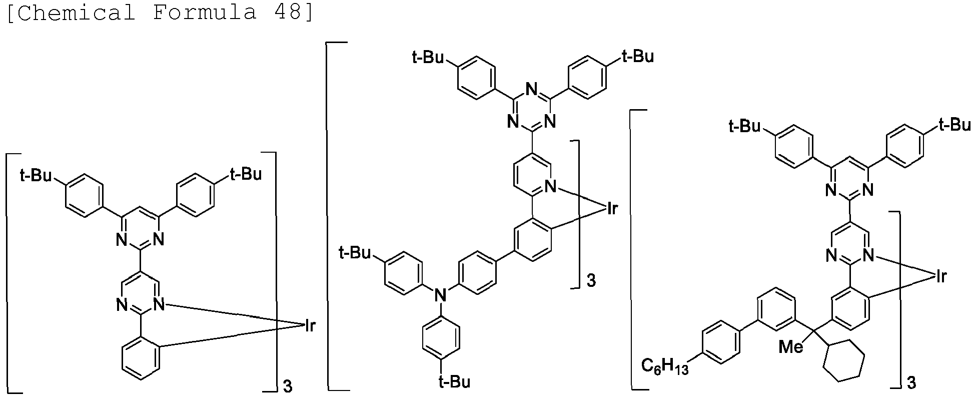

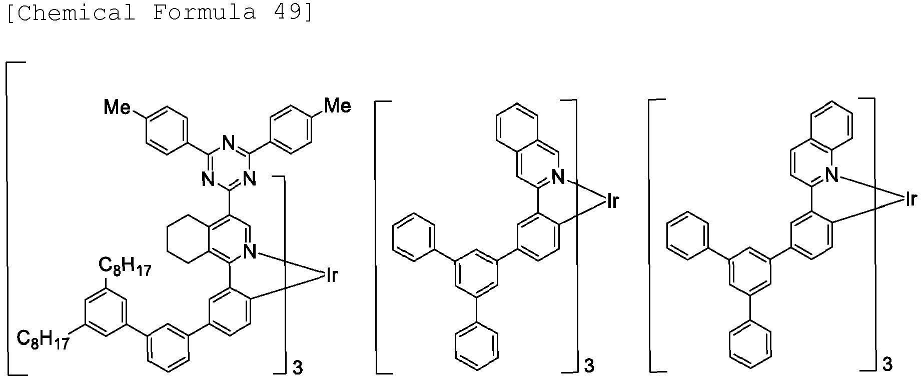

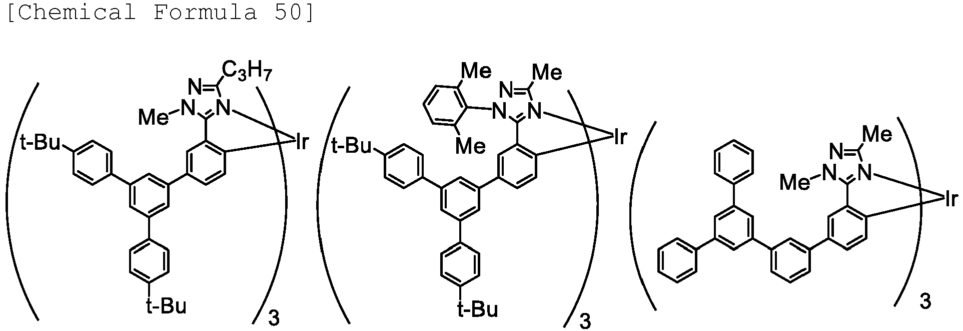

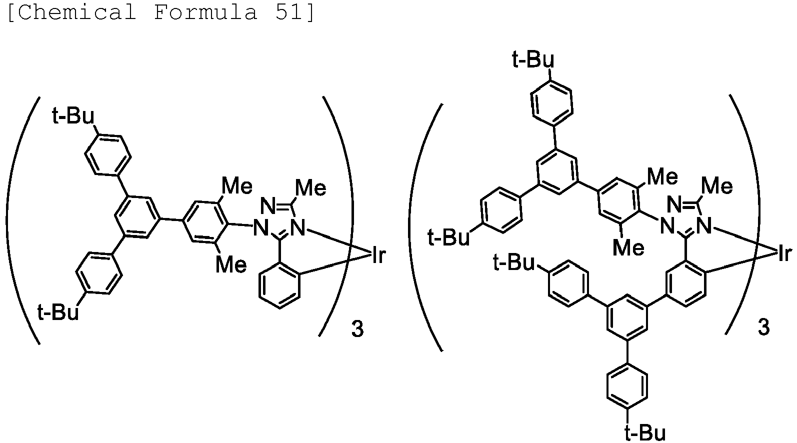

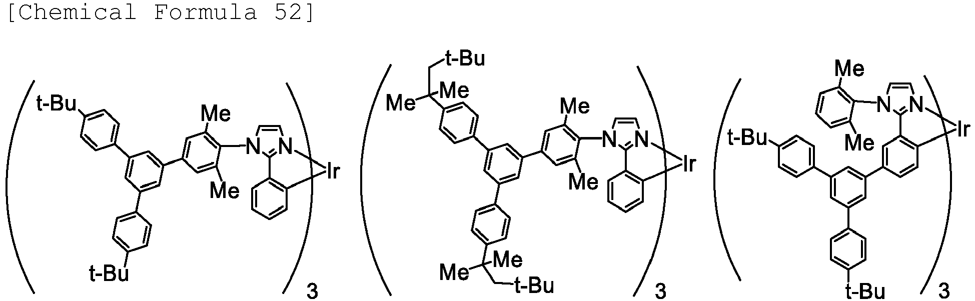

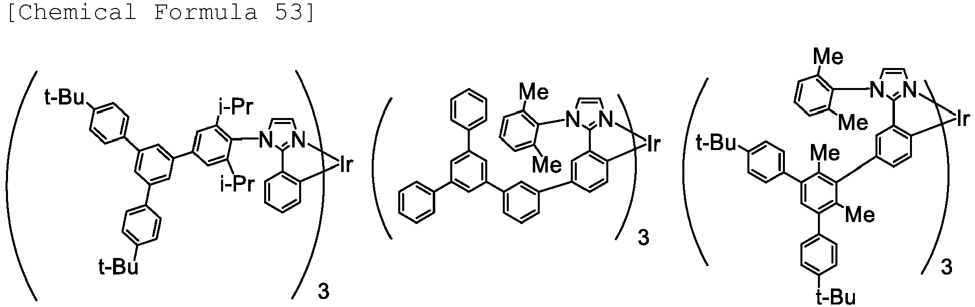

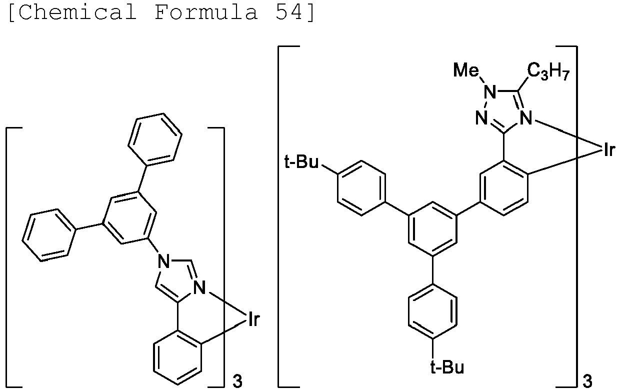

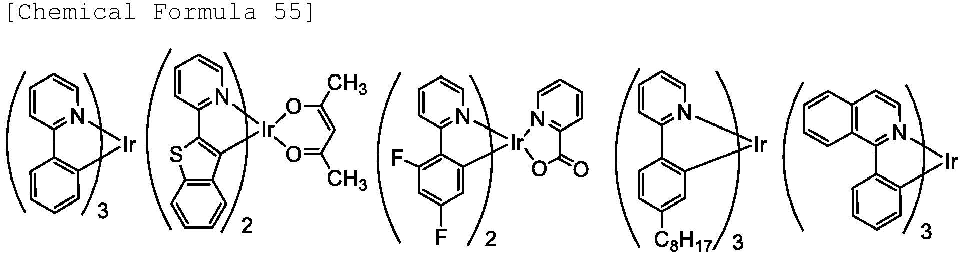

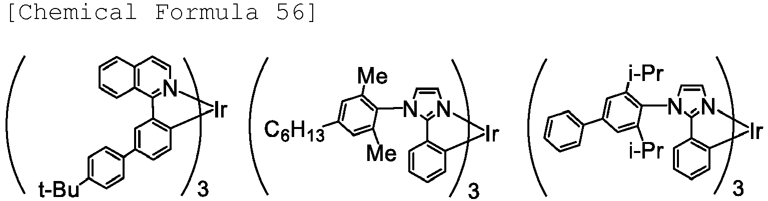

The phosphorescent compound represented by formula (1) can be synthesized according to methods disclosed in, for example, "Journal of the American Chemical Society, Vol. 107, 1431-1432 (1985)", "Journal of the American Chemical Society, Vol. 106, 6647-6653 (1984)", JP 2004-530254 W, JP 2008-179617 A, JP 2011-105701 A, JP 2007-504272 W, WO 2006/121811, JP 2013-147450 A and JP 2014-224101 A.

### [Compound Represented by Formula (H)]

The molecular weight of a compound represented by formula (H) is usually 1 × 10² to 5 × 10⁴, preferably 2 × 10² to 1 × 10⁴, more preferably 3 × 10² to 5 × 10³, still more preferably 4 × 10² to 2.5 × 10³, and particularly preferably 5 × 10² to 1.5 × 10³.

n^{H1} is preferably an integer of 0 or more and 3 or less, more preferably an integer of 0 or more and 2 or less, still more preferably 0 or 1, and particularly preferably 0, because it is easy to synthesize the compound.

n^{H2} is preferably an integer of 1 or more and 7 or less, more preferably an integer of 1 or more and 5 or less, still more preferably an integer of 1 or more and 3 or less, particularly preferably 1 or 2, and especially preferably 1, because the light emitting device according to the embodiment of the present invention is excellent in luminance life.

### [Group Represented by Formula (H1-1)]

In the ring R^{H1} and the ring R^{H2}, the number of carbon atoms of the monocyclic aromatic hydrocarbon ring, excluding the number of carbon atoms of a substituent, is preferably 6.

In the ring R^{H1} and the ring R^{H2}, the monocyclic aromatic hydrocarbon ring is preferably a benzene ring optionally having a substituent.

In the ring R^{H1} and the ring R^{H2}, the number of carbon atoms of the fused-ring aromatic hydrocarbon ring, excluding the number of carbon atoms of a substituent, is usually 7 to 60, preferably 9 to 30, and more preferably 10 to 18.

The fused-ring aromatic hydrocarbon ring in the ring R^{H1} and the ring R^{H2} includes, for example, a naphthalene ring, an anthracene ring, a phenanthrene ring, a dihydrophenanthrene ring, a naphthacene ring, a fluorene ring, a spirobifluorene ring, an indene ring, a pyrene ring, a perylene ring and a chrysene ring, and is preferably a naphthalene ring, an anthracene ring, a phenanthrene ring, a dihydrophenanthrene ring, a fluorene ring or a spirobifluorene ring, more preferably a naphthalene ring, a fluorene ring or a spirobifluorene ring, still more preferably a fluorene ring or a spirobifluorene ring, and particularly preferably a fluorene ring, and these rings each optionally have a substituent.

In the ring R^{H1} and the ring R^{H2}, the number of carbon atoms of the monocyclic aromatic heterocyclic ring, excluding the number of carbon atoms of a substituent, is preferably 2 to 5, and more preferably 3 to 5.

The monocyclic aromatic heterocyclic ring in the ring R^{H1} and the ring R^{H2} includes, for example, a pyrrole ring, a diazole ring, a triazole ring, a pyridine ring, a diazabenzene ring and a triazine ring, and is preferably a pyridine ring or a diazabenzene ring, and these rings each optionally have a substituent.

In the ring R^{H1} and the ring R^{H2}, the number of carbon atoms of the fused-ring aromatic heterocyclic ring, excluding the number of carbon atoms of a substituent, is usually 2 to 60, preferably 4 to 30, and more preferably 6 to 20.

The fused-ring aromatic heterocyclic ring in the ring R^{H1} and the ring R^{H2} includes, for example, an azanaphthalene ring, a diazanaphthalene ring, a triazanaphthalene ring, an indole ring, a carbazole ring, an azacarbazole ring, a diazacarbazole ring, a dibenzofuran ring, a dibenzothiophene ring, a phenoxazine ring, a phenothiazine ring, an acridine ring, a 9,10-dihydroacridine ring, an acridone ring, a phenazine ring and a 5,10-dihydrophenazine ring, and is preferably an azanaphthalene ring, a diazanaphthalene ring, a carbazole ring, an azacarbazole ring, a diazacarbazole ring, a dibenzofuran ring, a dibenzothiophene ring, a phenoxazine ring, a phenothiazine ring, a 9,10-dihydroacridine ring or a 5,10-dihydrophenazine ring, more preferably a carbazole ring, an azacarbazole ring, a diazacarbazole ring, a dibenzofuran ring, a dibenzothiophene ring, a phenoxazine ring, a phenothiazine ring, a 9,10-dihydroacridine ring or a 5,10-dihydrophenazine ring, still more preferably a carbazole ring, a dibenzofuran ring or a dibenzothiophene ring, and particularly preferably a carbazole ring, and these rings each optionally have a substituent.

The substituent which the ring R^{H1} and the ring R^{H2} optionally have is preferably an alkyl group, a cycloalkyl group, an alkoxy group, a cycloalkoxy group, an aryl group, an aryloxy group, a monovalent heterocyclic group, a substituted amino group or a halogen atom, more preferably an alkyl group, a cycloalkyl group, an alkoxy group, a cycloalkoxy group, an aryl group, a monovalent heterocyclic group or a substituted amino group, still more preferably an alkyl group, a cycloalkyl group, an aryl group, a monovalent heterocyclic group or a substituted amino group, particularly preferably an alkyl group, a cycloalkyl group or a group represented by formula (D-A), formula (D-B) or formula (D-C), and especially preferably an alkyl group or a cycloalkyl group, and these groups each optionally have a substituent.

The substituent which the substituent which the ring R^{H1} and the ring R^{H2} optionally have optionally further has is preferably an alkyl group, a cycloalkyl group, an alkoxy group, a cycloalkoxy group, an aryl group, an aryloxy group, a monovalent heterocyclic group, a substituted amino group or a halogen atom, more preferably an alkyl group, a cycloalkyl group, an alkoxy group, a cycloalkoxy group, an aryl group, a monovalent heterocyclic group or a substituted amino group, still more preferably an alkyl group, a cycloalkyl group, an aryl group or a monovalent heterocyclic group, particularly preferably an alkyl group, a cycloalkyl group or an aryl group, and especially preferably an alkyl group or a cycloalkyl group, and these groups each optionally have a substituent.

The examples and the preferable range of the aryl group, the monovalent heterocyclic group and the substituted amino group in the substituent which the ring R^{H1} and the ring R^{H2} optionally have are the same as the examples and the preferable range of the aryl group, the monovalent heterocyclic group and the substituted amino group as the substituent which the ring L¹ and the ring L² optionally have, respectively.

The examples and the preferable range of the aryl group, the monovalent heterocyclic group and the substituted amino group in the substituent which the substituent which the ring R^{H1} and the ring R^{H2} optionally have optionally further has are the same as the examples and the preferable range of the aryl group, the monovalent heterocyclic group and the substituted amino group as the substituent which the ring L¹ and the ring L² optionally have, respectively.

Regarding the combination of the ring R^{H1} and the ring R^{H2}, it is preferable that one is a fused-ring aromatic hydrocarbon ring or a fused-ring aromatic heterocyclic ring, and the other is a monocyclic aromatic hydrocarbon ring or a monocyclic aromatic heterocyclic ring, it is more preferable that one is a fused-ring aromatic hydrocarbon ring or a fused-ring aromatic heterocyclic ring, and the other is a monocyclic aromatic hydrocarbon ring, and it is still more preferable that one is a fused-ring aromatic hydrocarbon ring, and the other is a monocyclic aromatic hydrocarbon ring.

X^{H1} is preferably a single bond, an oxygen atom or a sulfur atom, and more preferably a single bond.

R^{XH1} is preferably an alkyl group, a cycloalkyl group, an aryl group or a monovalent heterocyclic group, more preferably an aryl group or a monovalent heterocyclic group, and still more preferably an aryl group, and these groups each optionally have a substituent.

R^{XH1'} is preferably an alkyl group, a cycloalkyl group, an aryl group, a monovalent heterocyclic group or a substituted amino group, more preferably an alkyl group, a cycloalkyl group, an aryl group or a monovalent heterocyclic group, still more preferably an alkyl group, a cycloalkyl group or an aryl group, and particularly preferably an alkyl group or a cycloalkyl group, and these groups each optionally have a substituent.

The examples and the preferable range of the substituent which the ring R^{XH1} and the ring R^{XH1'} optionally have are the same as the examples and the preferable range of the substituent which the substituent which the ring R^{H1} and the ring R^{H2} optionally have optionally further has.

The group represented by formula (H1-1) is preferably a group represented by formula (H1-1B), a group represented by formula (H1-1C) or a group represented by formula (H1-1D), more preferably a group represented by formula (H1-1B) or a group represented by formula (H1-1C), and still more preferably a group represented by formula (H1-1B).

X^{H2} and X^{H3} represent preferably a single bond, a group represented by -N(R^{XH2})- or a group represented by - C(R^{XH2'})₂-, and more preferably a single bond or a group represented by -C(R^{XH2'})₂-.

Of X^{H 2} and X^{H 3}, at least one is preferably a single bond, and it is more preferable that X^{H 3} is a single bond.

Of X^{H2} and X^{H 3}, when at least one is a single bond, the other is preferably an oxygen atom, a sulfur atom, a group represented by -N(R^{X H 2})- or a group represented by - C(R^{X H 2'})₂-, more preferably a group represented by -N(R^{X H 2})- or a group represented by -C(R^{X H 2'})₂-, and still more preferably a group represented by -C(R^{X H 2'})₂-.

The examples and the preferable range of R^{X H 2} are the same as the examples and the preferable range of R^{X H 1}.

The examples and the preferable range of R^{X H 2'} are the same as the examples and the preferable range of R^{X H 1'}.

The examples and the preferable range of the substituent which R^{X H 2} and R^{X H 2'} optionally have are the same as the examples and the preferable range of the substituent which the substituent which the ring R^{H 1} and the ring R^{H 2} optionally have optionally further has.

Z^{H 1}, Z^{H 2}, Z^{H 3}, Z^{H 4}, Z^{H 5}, Z^{H 6}, Z^{H 7}, Z^{H 8}, Z^{H 9}, Z^{H 10}, Z^{H 11} and Z^{H 12} represent preferably a carbon atom

R^{H 1}, R^{H2}, R^{H 3}, R^{H 4}, R^{H 5}, R^{H 6}, R^{H 7}, R^{H 8}, R^{H 9}, R^{H 10}, R^{H 11} and R^{H 12} represent preferably a hydrogen atom, an alkyl group, a cycloalkyl group, an alkoxy group, a cycloalkoxy group, an aryl group, a monovalent heterocyclic group or a substituted amino group, more preferably a hydrogen atom, an alkyl group, a cycloalkyl group, an aryl group, a monovalent heterocyclic group or a substituted amino group, still more preferably a hydrogen atom, an alkyl group, a cycloalkyl group or a group represented by formula (D-A), formula (D-B) or formula (D-C), particularly preferably a hydrogen atom, an alkyl group or a cycloalkyl group, and especially preferably a hydrogen atom, and these groups each optionally have a substituent.

The examples and the preferable range of the substituent which R^{H 1}, R^{H2}, R^{H 3}, R^{H 4}, R^{H 5}, R^{H 6}, R^{H 7}, R^{H 8}, R^{H 9}, R^{H 10}, R^{H 11} and R^{H 12} optionally have are the same as the examples and the preferable range of the substituent which the substituent which the ring R^{H 1} and the ring R^{H 2} optionally have optionally further has.

R^{H 1} and R^{H 2}, R^{H 3} and R^{H 4} , R^{H 5} and R^{H 6}, R^{H 6} and R^{H 7}, R^{H 7} and R^{H 8}, R^{H 9} and R^{H 10}, R^{H 10} and R^{H 11}, and R^{H 11} and R^{H 12} each may be combined together to form a ring together with the carbon atoms to which they are attached, but it is preferable that no ring is formed.

L^{H 1} is preferably an alkylene group, a cycloalkylene group, an arylene group or a divalent heterocyclic group, more preferably an arylene group or a divalent heterocyclic group, and still more preferably an arylene group, and these groups each optionally have a substituent.

The arylene group represented by L^{H 1} is preferably a phenylene group, a naphthalenediyl group, a fluorenediyl group, a phenanthrenediyl group or a dihydrophenanthrenediyl group, more preferably a group represented by formula (A-1) to formula (A-9), formula (A-19) or formula (A-20), still more preferably a group represented by formula (A-1) to formula (A-3), particularly preferably a group represented by formula (A-1) or (A-2), and especially preferably a group represented by formula (A-2), and these groups each optionally have a substituent.

The divalent heterocyclic group represented by L^{H 1} is preferably a group represented by formula (AA-1) to formula (AA-34), more preferably a group represented by formula (AA-1) to formula (AA-6), a group represented by formula (AA-10) to formula (AA-21) or a group represented by formula (AA-24) to formula (AA-34), still more preferably a group represented by formula (AA-1) to formula (AA-4), a group represented by formula (AA-10) to formula (AA-15) or a group represented by formula (AA-29) to formula (AA-34), and particularly preferably a group represented by formula (AA-2), formula (AA-4), formula (AA-10), formula (AA-12) or formula (AA-14).

The examples and the preferable range of the substituent which L^{H 1} optionally has are the same as the examples and the preferable range of the substituent which the ring R^{H 1} and the ring R^{H 2} optionally have.

The examples and the preferable range of the substituent which the substituent which L^{H 1} optionally has optionally further has are the same as the examples and the preferable range of the substituent which the substituent which the ring R^{H 1} and the ring R^{H 2} optionally have optionally further has.

R^{H 1'} is preferably an aryl group or a monovalent heterocyclic group, and more preferably an aryl group, and these groups each optionally have a substituent.

The examples and the preferable range of the substituent which R^{H 1'} optionally has are the same as the examples and the preferable range of the substituent which the substituent which the ring R^{H 1} and the ring R^{H 2} optionally have optionally further has.

In Ar^{H 2}, the number of carbon atoms of the aromatic hydrocarbon group, excluding the number of carbon atoms of a substituent, is usually 6 to 60, preferably 6 to 30, and more preferably 6 to 18.

In Ar^{H 2}, examples of the aromatic hydrocarbon group include a group obtained by removing from a benzene ring, a naphthalene ring, an anthracene ring, a phenanthrene ring, a dihydrophenanthrene ring, a naphthacene ring, a fluorene ring, a spirobifluorene ring, an indene ring, a pyrene ring, a perylene ring, a chrysene ring or a ring in which these rings are fused one or more hydrogen atoms linked directly to carbon atoms constituting the ring, preferably a group obtained by removing from a benzene ring, a naphthalene ring, a phenanthrene ring, a dihydrophenanthrene ring, a fluorene ring, a spirobifluorene ring or a ring in which these rings are fused one or more hydrogen atoms linked directly to carbon atoms constituting the ring, more preferably a group obtained by removing from a benzene ring, a naphthalene ring, a phenanthrene ring, a dihydrophenanthrene ring, a fluorene ring or a spirobifluorene ring one or more hydrogen atoms linked directly to carbon atoms constituting the ring, still more preferably a group obtained by removing from a benzene ring, a fluorene ring or a spirobifluorene ring one or more hydrogen atoms linked directly to carbon atoms constituting the ring, and particularly preferably a group obtained by removing from a benzene ring one or more hydrogen atoms linked directly to carbon atoms constituting the ring, and these groups each optionally have a substituent.

In Ar^{H 2}, the number of carbon atoms of the aromatic heterocyclic group, excluding the number of carbon atoms of a substituent, is usually 1 to 60, preferably 2 to 40, more preferably 3 to 20, and still more preferably 3 to 10.

In Ar^{H 2}, examples of the aromatic heterocyclic group include a group obtained by removing from a pyrrole ring, a furan ring, a thiophene ring, an oxadiazole ring, a thiadiazole ring, a thiazole ring, an oxazole ring, an isothiazole ring, an isooxazole ring, a benzoxazole ring, a benzothiadiazole ring, a benzothiazole ring, a benzoxazole ring, a pyridine ring, a diazabenzene ring, a triazine ring, an azanaphthalene ring, a diazanaphthalene ring, a triazanaphthalene ring, a tetraazanaphthalene ring, an azaanthracene ring, a diazaanthracene ring, a triazaanthracene ring, a tetraazaanthracene ring, an azaphenanthrene ring, a diazaphenanthrene ring, a triazaphenanthrene ring, a tetraazaphenanthrene ring, a dibenzofuran ring, a dibenzothiophene ring, a dibenzosilole ring, a dibenzophosphole ring, a carbazole ring, an azacarbazole ring, a diazacarbazole ring, a phenoxazine ring, a phenothiazine ring or a ring in which an aromatic ring is fused to these heterocyclic rings one or more hydrogen atoms linked directly to carbon atoms or heteroatoms constituting the ring, preferably a group obtained by removing from a pyridine ring, a diazabenzene ring, a triazine ring, an azanaphthalene ring, a diazanaphthalene ring, an azaanthracene ring, a diazaanthracene ring, an azaphenanthrene ring, a diazaphenanthrene ring, a dibenzofuran ring, a dibenzothiophene ring, a carbazole ring, an azacarbazole ring or a diazacarbazole ring one or more hydrogen atoms linked directly to carbon atoms or heteroatoms constituting the ring (of these groups, preferred is a group obtained by removing one or more hydrogen atoms linked directly to carbon atoms constituting the ring), more preferably a group obtained by removing from a pyridine ring, a diazabenzene ring, a triazine ring, a quinoline ring, an isoquinoline ring, a quinazoline ring, a quinoxaline ring, an acridine ring, a phenazine ring, a phenanthroline ring, a dibenzofuran ring, a dibenzothiophene ring, a carbazole ring, an azacarbazole ring or a diazacarbazole ring one or more hydrogen atoms linked directly to carbon atoms or heteroatoms constituting the ring (of these groups, preferred is a group obtained by removing one or more hydrogen atoms linked directly to carbon atoms constituting the ring), still more preferably a group obtained by removing from a pyridine ring, a diazabenzene ring, a triazine ring, a quinoline ring, an isoquinoline ring, a quinazoline ring, a quinoxaline ring, a dibenzofuran ring, a dibenzothiophene ring or a carbazole ring one or more hydrogen atoms linked directly to carbon atoms or heteroatoms constituting the ring (of these groups, preferred is a group obtained by removing one or more hydrogen atoms linked directly to carbon atoms constituting the ring), particularly preferably a group obtained by removing from a pyridine ring, a pyrimidine ring or a triazine ring one or more hydrogen atoms linked directly to carbon atoms constituting the ring, and especially preferably a group obtained by removing from a triazine ring one or more hydrogen atoms linked directly to carbon atoms constituting the ring, and these groups each optionally have a substituent.

Ar^{H 2} is preferably a group obtained by removing from a benzene ring, a fluorene ring, a spirobifluorene ring, a pyridine ring, a diazabenzene ring, a triazine ring, a quinoline ring, an isoquinoline ring, a quinazoline ring, a quinoxaline ring, a dibenzofuran ring, a dibenzothiophene ring or a carbazole ring one or more hydrogen atoms linked directly to carbon atoms or heteroatoms constituting the ring, more preferably a group obtained by removing from a benzene ring, a fluorene ring, a spirobifluorene ring, a pyridine ring, a diazabenzene ring, a triazine ring, a quinoline ring, an isoquinoline ring, a quinazoline ring, a quinoxaline ring, a dibenzofuran ring, a dibenzothiophene ring or a carbazole ring one or more hydrogen atoms linked directly to carbon atoms constituting the ring, still more preferably a group obtained by removing from a benzene ring, a pyridine ring, a pyrimidine ring or a triazine ring one or more hydrogen atoms linked directly to carbon atoms constituting the ring, particularly preferably a group obtained by removing from a benzene ring or a triazine ring one or more hydrogen atoms linked directly to carbon atoms constituting the ring, and especially preferably a group obtained by removing from a triazine ring one or more hydrogen atoms linked directly to carbon atoms constituting the ring, these groups each optionally have a substituent, because the light emitting device according to the embodiment of the present invention is excellent in luminance life.

The substituent which Ar^{H 2} optionally has (which is different from the below-mentioned group represented by formula (1H'), the same shall apply hereinafter) is preferably an alkyl group, a cycloalkyl group, an alkoxy group, a cycloalkoxy group, an aryl group, an aryloxy group, a monovalent heterocyclic group, a substituted amino group or a halogen atom, more preferably an alkyl group, a cycloalkyl group, an alkoxy group, a cycloalkoxy group, an aryl group, a monovalent heterocyclic group or a substituted amino group, still more preferably an alkyl group, a cycloalkyl group, an aryl group, a monovalent heterocyclic group or a substituted amino group, particularly preferably an alkyl group, a cycloalkyl group or a group represented by formula (D-A), formula (D-B) or formula (D-C), especially preferably a group represented by formula (D-A), formula (D-B) or formula (D-C), and especially more preferably a group represented by formula (D-A), and these groups each optionally have a substituent.

The examples and the preferable range of the aryl group, the monovalent heterocyclic group and the substituted amino group in the substituent which Ar^{H 2} optionally has are the same as the examples and the preferable range of the aryl group, the monovalent heterocyclic group and the substituted amino group in the substituent which the ring L¹ and the ring L² optionally have, respectively.

The examples and the preferable range of the aryl group, the monovalent heterocyclic group and the substituted amino group in the substituent which the substituent which Ar^{H 2} optionally has optionally further has are the same as the examples and the preferable range of the aryl group, the monovalent heterocyclic group and the substituted amino group in the substituent which the ring L¹ and the ring L² optionally have, respectively.

The examples and the preferable range of the substituent which the substituent which Ar^{H 2} optionally has optionally further has are the same as the examples and the preferable range of the substituent which the substituent which the ring R^{H 1} and the ring R^{H 2} optionally have optionally further has.

Since the light emitting device according to the embodiment of the present invention is more excellent in luminance life, the compound represented by formula (H) is preferably a compound represented by formula (H'-1) to formula (H'-14), more preferably a compound represented by formula (H'-1) to formula (H'-5), still more preferably a compound represented by formula (H'-4) or formula (H'-5), and particularly preferably a compound represented by formula (H'-4) : 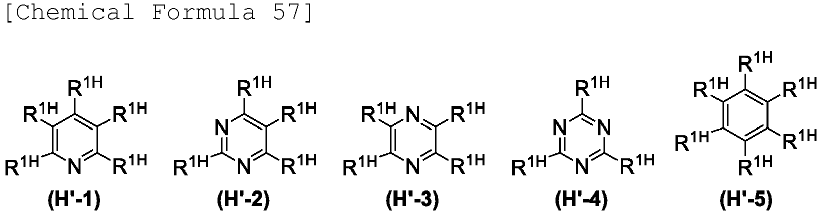 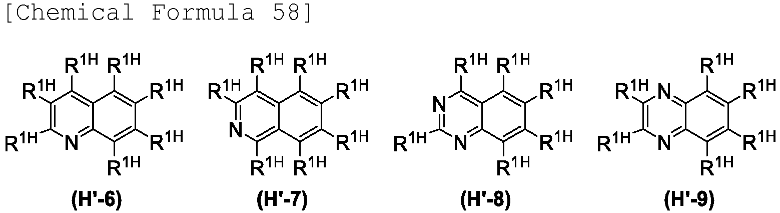 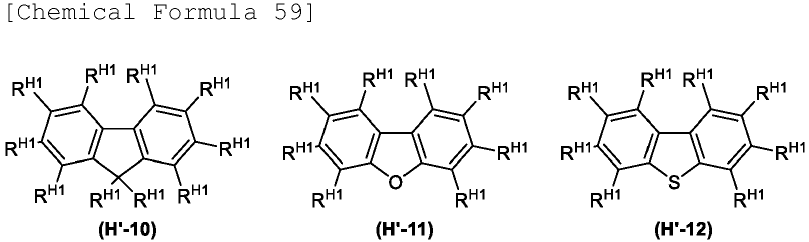 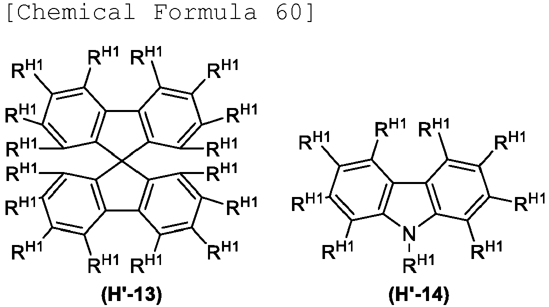 wherein R^{1 H} represents a hydrogen atom, an alkyl group, a cycloalkyl group, an alkoxy group, a cycloalkoxy group, an aryl group, an aryloxy group, a monovalent heterocyclic group, a substituted amino group, a halogen atom or a group represented by formula (1H'), and these groups each optionally have a substituent, the plurality of R^{1 H} may be the same or different, and at least one of the plurality of R^{1 H} is a group represented by formula (1H').

Of the plurality of R^{1 H}, R^{1 H} whose number is n^{H 2} preferably represents a group represented by formula (1H'): 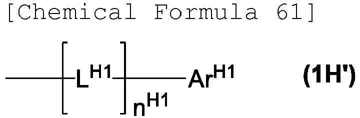 wherein L^{H 1}, n^{H 1} and Ar^{H 1} represent the same meaning as defined above.

R^{1 H} is preferably a hydrogen atom, an alkyl group, a cycloalkyl group, an alkoxy group, a cycloalkoxy group, an aryl group, a monovalent heterocyclic group, a substituted amino group or a group represented by formula (1H'), more preferably a hydrogen atom, an alkyl group, a cycloalkyl group, an aryl group, a monovalent heterocyclic group, a substituted amino group or a group represented by formula (1H'), still more preferably a hydrogen atom, an alkyl group, a cycloalkyl group or a group represented by formula (D-A), formula (D-B), formula (D-C) or formula (1H'), particularly preferably a hydrogen atom or a group represented by formula (D-A), formula (D-B), formula (D-C) or formula (1H'), and especially preferably a hydrogen atom or a group represented by formula (D-A) or formula (1H'), and these groups each optionally have a substituent.

The examples and the preferable range of the aryl group, the monovalent heterocyclic group and the substituted amino group in R^{1 H} are the same as the examples and the preferable range of the aryl group, the monovalent heterocyclic group and the substituted amino group in the substituent which the ring L¹ and the ring L² optionally have, respectively.

The examples and the preferable range of the substituent which R^{1 H} optionally has are the same as the examples and the preferable range of the substituent which the substituent which the ring R^{H 1} and the ring R^{H 2} optionally have optionally further has.

The compound represented by formula (H'-1) to formula (H'-14) includes, for example, a compound represented by formula (H"-1) to formula (H"-33), preferably a compound represented by formula (H"-1) to formula (H"-21), more preferably a compound represented by formula (H"-1) to formula (H"-11), still more preferably a compound represented by formula (H"-1) to formula (H"-8), and particularly preferably a compound represented by formula (H"-8): 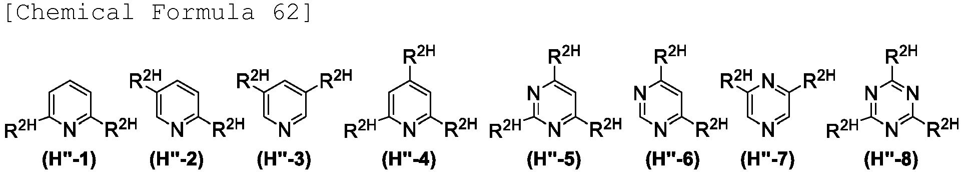 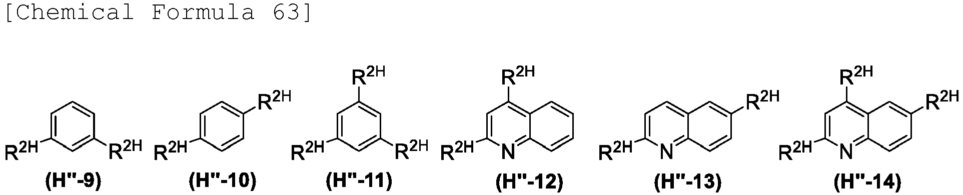 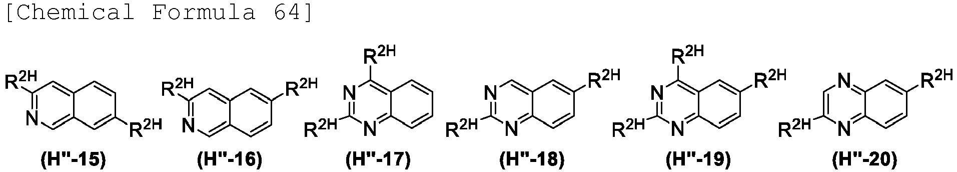 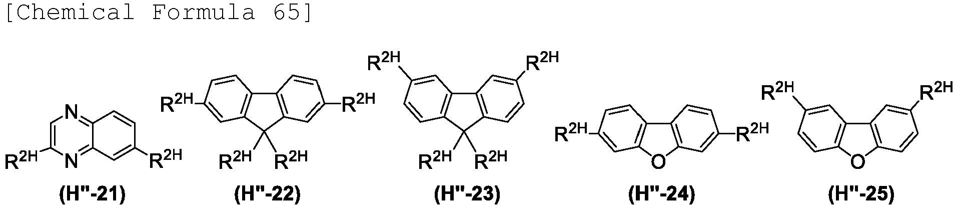 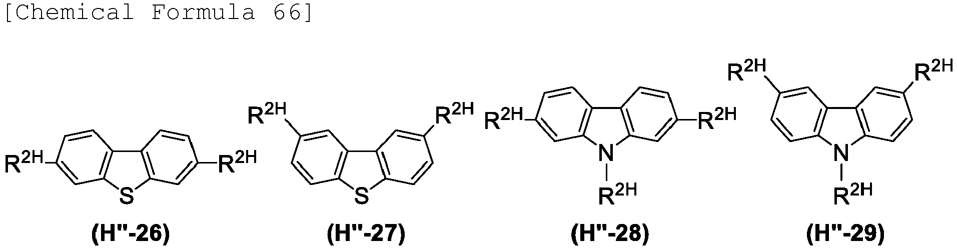 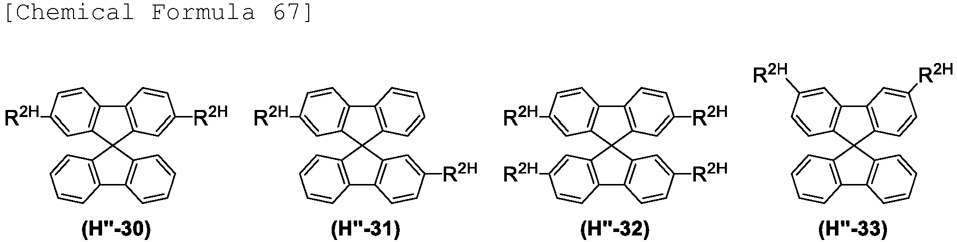 wherein R^{2 H} represents an alkyl group, a cycloalkyl group, an aryl group, a monovalent heterocyclic group, a substituted amino group or a group represented by formula (1H'), and these groups each optionally have a substituent, the plurality of R^{2 H} may be the same of different, and at least one of the plurality of R^{2 H} is a group represented by formula (1H') .

Of the plurality of R^{2 H}, R^{2 H} whose number is n^{H 2} represents preferably a group represented by formula (1H').

R^{2 H} is preferably an alkyl group, a cycloalkyl group, a group represented by formula (D-A), formula (D-B), formula (D-C) or formula (1H'), more preferably a group represented by formula (D-A), formula (D-B), formula (D-C) or formula (1H'), and still more preferably a group represented by formula (D-A) or formula (1H'), and these groups each optionally have a substituent.

The examples and the preferable range of the aryl group, the monovalent heterocyclic group and the substituted amino group in R^{2 H} are the same as the examples and the preferable range of the aryl group, the monovalent heterocyclic group and the substituted amino group in the substituent which the ring L¹ and the ring L² optionally have, respectively.

The examples and the preferable range of the substituent which R^{2 H} optionally has are the same as the examples and the preferable range of the substituent which the substituent which the ring R^{H 1} and the ring R^{H 2} optionally have optionally further has.

The compound represented by formula (H) include, for example, compounds represented by the following formulas.

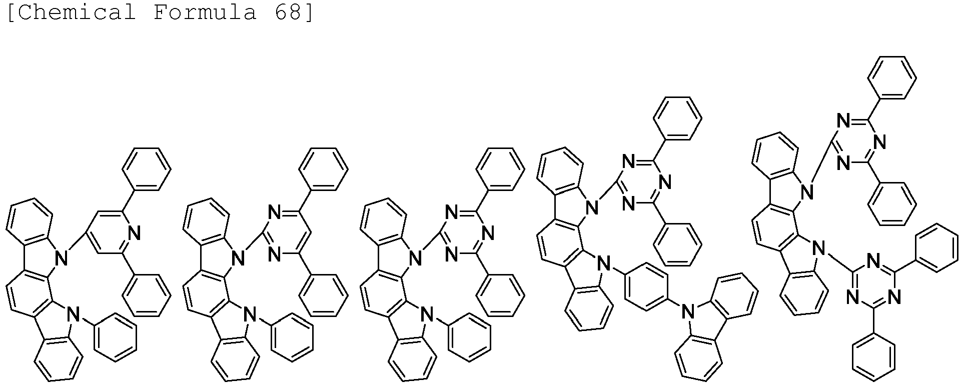

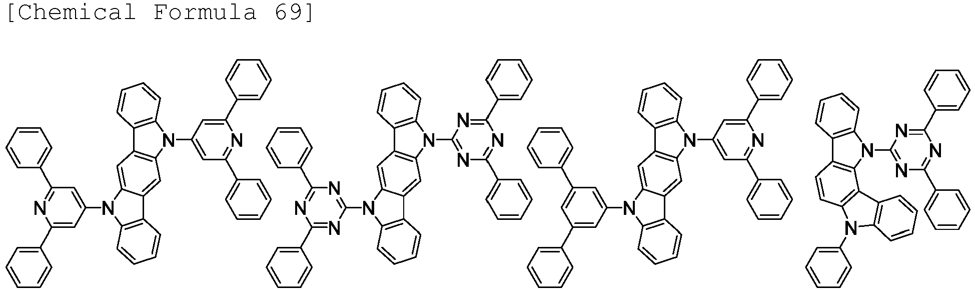

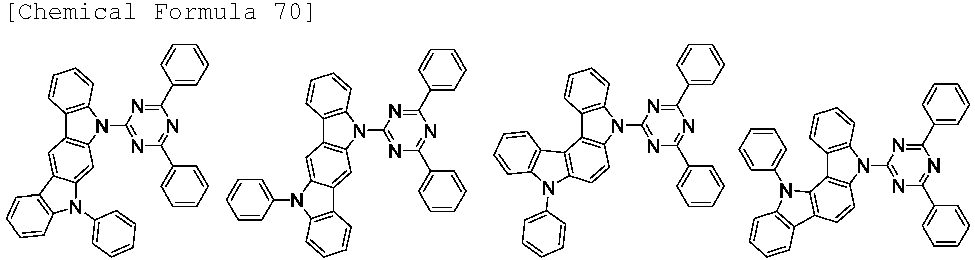

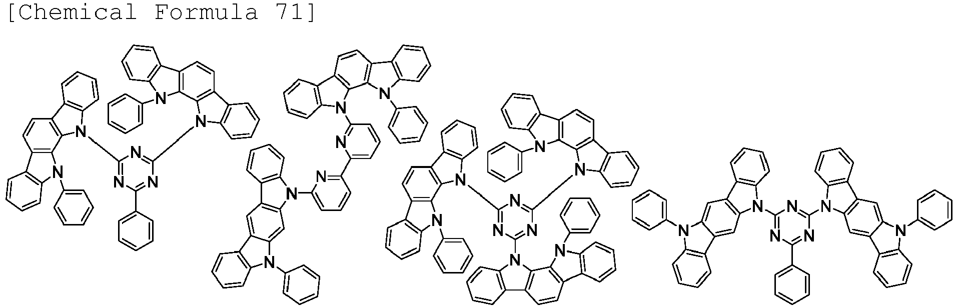

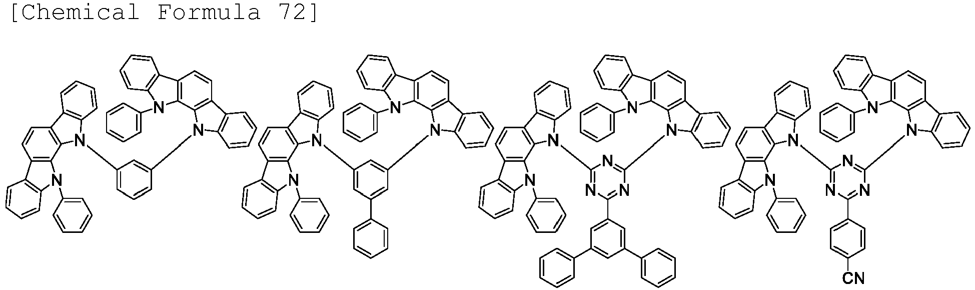

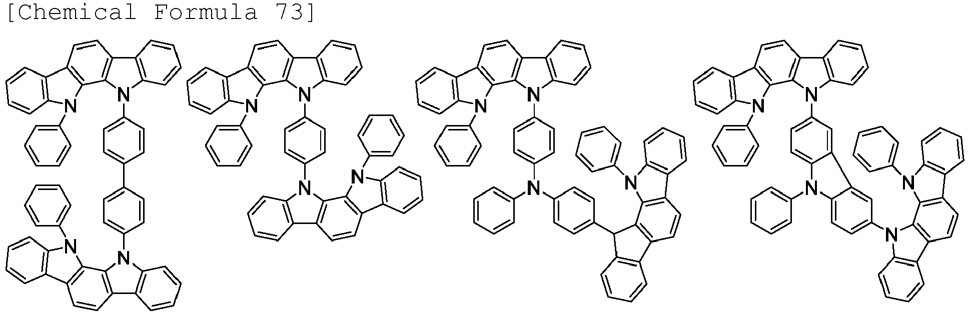

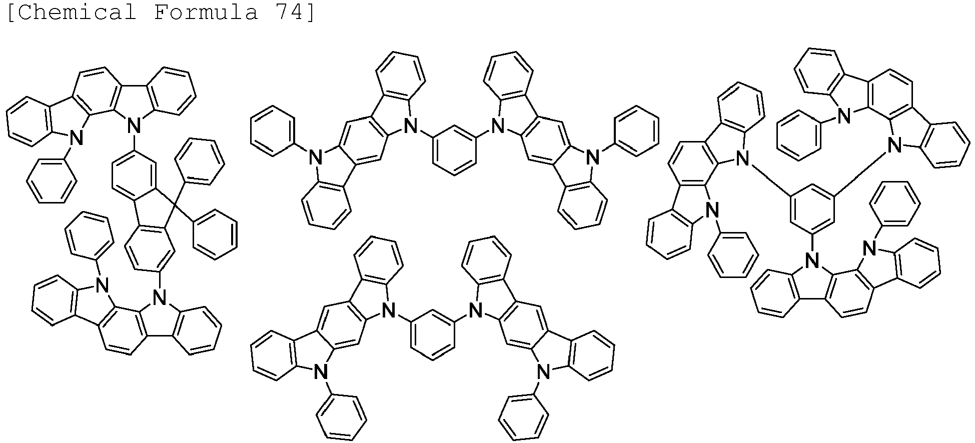

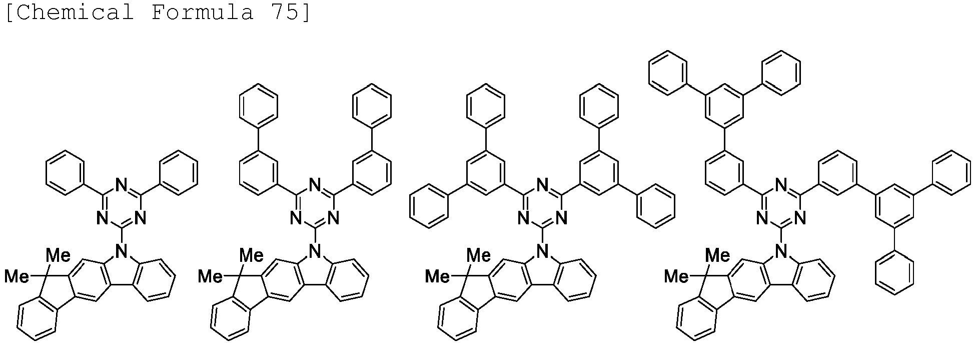

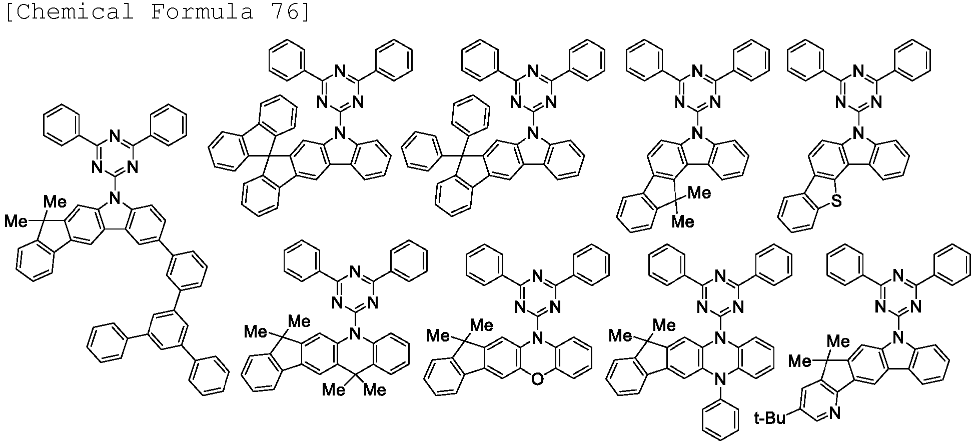

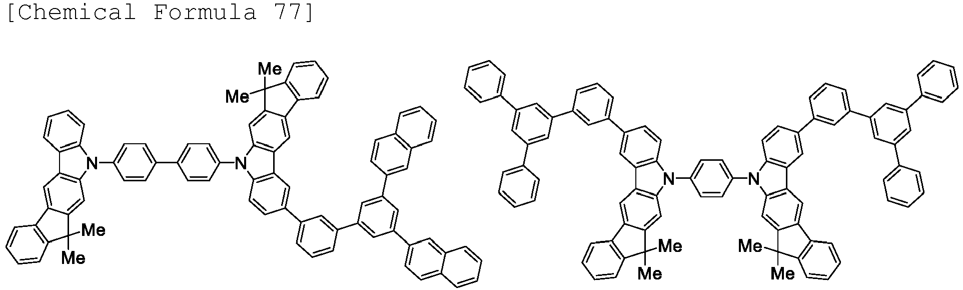

The compound represented by formula (H) is available from Aldrich, Luminescence Technology Corp. The compound can be synthesized according to methods disclosed in, for example, WO 2007/063754, WO 2008/056746, WO 2011/032686, WO 2012/096263, JP 2009-227663 A and JP 2010-275255 A.

### [Composition Ratio of First Organic Layer]

A first organic layer is a layer comprising a phosphorescent compound represented by formula (1) and a compound represented by formula (H).

In the first organic layer, a phosphorescent compound represented by formula (1) may be contained alone. Since it is possible to adjust luminescent color of the light emitting device according to the embodiment of the present invention, two or more phosphorescent compounds represented by formula (1) may be contained. In the first organic layer, a compound represented by formula (H) may be contained alone or two or more compounds may be contained.

In the first organic layer, the amount of the phosphorescent compound represented by formula (1) is usually 0.01 to 95 parts by weight when the total amount of the phosphorescent compound represented by formula (1) and the compound represented by formula (H) is 100 parts by weight, and is preferably 0.1 to 80 parts by weight, more preferably 1 to 65 parts by weight, still more preferably 3 to 50 parts by weight, and particularly preferably 5 to 40 parts by weight, because the light emitting device according to the embodiment of the present invention is more excellent in luminance life.

The first organic layer may also be a layer comprising a composition comprising a phosphorescent compound represented by formula (1), a compound represented by formula (H), and at least one material selected from the group consisting of a hole transporting material, a hole injection material, an electron transporting material, an electron injection material, a light emitting material and an antioxidant (hereinafter also referred to as "first composition"). In the first composition, the light emitting material is different from the phosphorescent compound represented by formula (1). In the first composition, the hole transporting material, the hole injection material, the light emitting material, the electron transporting material and the electron injection material are different from the compound represented by formula (H).

### [Hole Transporting Material]

The hole transporting material is classified into a low molecular weight compound and a polymer compound, and is preferably a polymer compound. The hole transporting material optionally has a crosslinkable group.

The polymer compound includes, for example, polyvinylcarbazole and derivatives thereof; polyarylene having an aromatic amine structure in the side chain or main chain and derivatives thereof. The polymer compound may also be a compound in which an electron accepting portion is linked. The electron accepting portion includes, for example, fullerene, tetrafluorotetracyanoquinodimethane, tetracyanoethylene, trinitrofluorenone and the like, and preferably fullerene.

In the first composition, the amount of the hole transporting material mixed is usually 1 to 400 parts by weight, and preferably 5 to 150 parts by weight when the total amount of the phosphorescent compound represented by formula (1) and the compound represented by formula (H) is 100 parts by weight.

The hole transporting material may be used alone or two or more hole transporting materials may be used in combination.

### [Electron Transporting Material]

The electron transporting material is classified into a low molecular weight compound and a polymer compound. The electron transporting material optionally has a crosslinkable group.

The low molecular weight compound includes, for example, a phosphorescent compound having 8-hydroxyquinoline as a ligand, oxadiazole, anthraquinodimethane, benzoquinone, naphthoquinone, anthraquinone, tetracyanoanthraquinodimethane, fluorenone, diphenyldicyanoethylene, diphenoquinone and derivatives thereof.

The polymer compound includes, for example, polyphenylene, polyfluorene and derivatives thereof. These polymer compounds may be doped with metal.

In the first composition, the amount of the electron transporting material mixed is usually 1 to 400 parts by weight, and preferably 5 to 150 parts by weight when the total amount of the phosphorescent compound represented by formula (1) and the compound represented by formula (H) is 100 parts by weight.

The electron transporting material may be used alone or two or more electron transporting materials may be used in combination.

### [Hole Injection Material and Electron Injection Material]

The hole injection material and the electron injection material are each classifies into a low molecular weight compound and a polymer compound. The hole injection material and the electron injection material optionally have a crosslinkable group.

The low molecular weight compound includes, for example, metal phthalocyanines such as copper phthalocyanine; carbon; oxides of metals such as molybdenum and tungsten; and metal fluorides such as lithium fluoride, sodium fluoride, cesium fluoride and potassium fluoride.

The polymer compound includes, for example, polyaniline, polythiophene, polypyrrole, polyphenylenevinylene, polythienylenevinylene, polyquinoline and polyquinoxaline, and derivatives thereof; and conductive polymers such as a polymer comprising an aromatic amine structure in the side chain or main chain.

In the first composition, the amounts of the hole injection material and the electron injection material mixed are each usually 1 to 400 parts by weight, and preferably 5 to 150 parts by weight when the total amount of the phosphorescent compound represented by formula (1) and the compound represented by formula (H) is 100 parts by weight.

The electron injection material and the hole injection material may each be used alone or two or more electron injection materials and hole injection materials may be used in combination.

### [Ion Doping]

When the hole injection material or the electron injection material comprises a conductive polymer, the electric conductivity of the conductive polymer is preferably 1 × 10⁻⁵ S/cm to 1 × 10³ S/cm. To adjust the electric conductivity of the conductive polymer in the above range, the conductive polymer can be doped with a suitable amount of ions.

The type of ions to be doped is anions in the case of the hole injection material and cations in the case of the electron injection material. The anions includes, for example, polystyrenesulfonate ions, alkylbenzenesulfonate ions and camphorsulfonate ions. The cations includes, for example, lithium ions, sodium ions, potassium ions and tetrabutylammonium ions.

The ions to be doped may be used alone or two or more ions may be used.

### [Light Emitting Material]

The light emitting material is classified into a low molecular weight compound and a polymer compound. The light emitting material optionally has a crosslinkable group.

The low molecular weight compound includes, for example, naphthalene and derivatives thereof, anthracene and derivatives thereof, perylene and derivatives thereof, and triplet light emitting complexes having iridium, platinum or europium as the central metal.

The polymer compound includes, for example, polymer compounds having a phenylene group, a naphthalenediyl group, a fluorenediyl group, a phenanthrenediyl group, dihydrophenanthrenediyl group, a group represented by formula (X) mentioned below, a carbazolediyl group, a phenoxazinediyl group, a phenothiazinediyl group, an anthracenediyl group, a pyrenediyl group and the like.

The light emitting material preferably comprises a triplet light emitting complex.

The triplet light emitting complex includes, for example, metal complexes mentioned below.

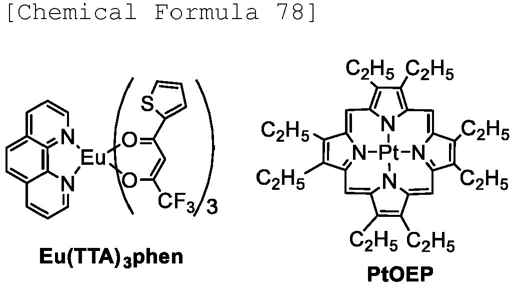

In the first composition, the amount of the light emitting material mixed is usually 1 to 400 parts by weight, and preferably 5 to 150 parts by weight when the total amount of the phosphorescent compound represented by formula (1) and the compound represented by formula (H) is 100 parts by weight.

The light emitting material may be used alone or two or more light emitting materials may be used in combination.

### [Antioxidant]

The antioxidant may be a compound which is soluble in the same solvent as for the phosphorescent compound represented by formula (1) and the compound represented by formula (H) and does not disturb light emission and charge transportation, and the examples thereof include phenol-based antioxidants and phosphorus-based antioxidants.

In the first composition, the amount of the antioxidant mixed is usually 0.001 to 10 parts by weight when the total amount of the phosphorescent compound represented by formula (1) and the compound represented by formula (H) is 100 parts by weight.

The antioxidant may be used alone or two or more antioxidants may be used in combination.

### [First Ink]

The composition comprising a phosphorescent compound represented by formula (1), a compound represented by formula (H), and a solvent (hereinafter also referred to as "first ink") can be suitably used in wet process such as a spin coating method, a casting method, a micro gravure coating method, a gravure coating method, a bar coating method, a roll coating method, a wire bar coating method, a dip coating method, a spray coating method, a screen printing method, a flexographic printing method, an offset printing method, an ink-jet printing method, a capillary coating method and a nozzle coating method.

The viscosity of the first ink may be adjusted depending on the type of the printing method, and when applying to an ink-jet printing method in which a solution goes through a discharge apparatus, the viscosity is preferably 1 to 20 mPa·s at 25°C because clogging during discharging and flight bending are less likely to occur.

The solvent contained in the first ink is a solvent capable of dissolving or uniformly dispersing solid components in the ink. The solvent includes, for example, chlorine-based solvents such as 1,2-dichloroethane, 1,1,2-trichloroethane, chlorobenzene and o-dichlorobenzene; ether-based solvents such as THF, dioxane, anisole and 4-methylanisole; aromatic hydrocarbon-based solvents such as toluene, xylene, mesitylene, ethylbenzene, n-hexylbenzene and cyclohexylbenzene; aliphatic hydrocarbon-based solvents such as cyclohexane, methylcyclohexane, n-pentane, n-hexane, n-heptane, n-octane, n-nonane, n-decane, n-dodecane and bicyclohexyl; ketone-based solvents such as acetone, methyl ethyl ketone, cyclohexanone and acetophenone; ester-based solvents such as ethyl acetate, butyl acetate, ethylcellosolve acetate, methyl benzoate and phenyl acetate; polyhydric alcohol-based solvents such as ethylene glycol, glycerin and 1,2-hexanediol; alcohol-based solvents such as isopropyl alcohol and cyclohexanol; sulfoxide-based solvents such as dimethyl sulfoxide; and amide-based solvents such as N-methyl-2-pyrrolidone and N,N-dimethylformamide.

These solvents may be used alone or two or more solvents may be used in combination.

In the first ink, the amount of the solvent mixed is usually 1,000 to 100,000 parts by weight, and preferably 2,000 to 20,000 parts by weight when the total amount of the phosphorescent compound represented by formula (1) and the compound represented by formula (H) is 100 parts by weight.

### <Second Organic Layer>

The second organic layer is a layer comprising a crosslinked body of a crosslinkable material.

### [Crosslinkable Material]

The crosslinked body of a crosslinkable material is obtained by being brought into a state where the crosslinkable material is crosslinked by the above-mentioned methods and conditions.

The crosslinkable material may be a low molecular weight compound or a polymer compound, and is preferably a low molecular weight compound having at least one crosslinkable group selected from Group A of crosslinkable group (hereinafter also referred to as "low molecular weight compound of second organic layer") or a polymer compound comprising a crosslinkable constitutional unit having at least one crosslinkable group selected from Group A of crosslinkable group (hereinafter, referred to also as "polymer compound of second organic layer"), and more preferably a polymer compound comprising a crosslinkable constitutional unit having at least one crosslinkable group selected from Group A of crosslinkable group, because the light emitting device according to the embodiment of the present invention is more excellent in luminance life.

The crosslinkable group selected from Group A of crosslinkable group is preferably a crosslinkable group represented by formula (XL-1) to formula (XL-4), formula (XL-7) to formula (XL-10) or formula (XL-14) to formula (XL-17), more preferably a crosslinkable group represented by formula (XL-1), formula (XL-3), formula (XL-9), formula (XL-10), formula (XL-16) or formula (XL-17), still more preferably a crosslinkable group represented by formula (XL-1), formula (XL-16) or formula (XL-17), particularly preferably a crosslinkable group represented by formula (XL-1) or formula (XL-17), and especially preferably a crosslinkable group represented by formula (XL-17), because the light emitting device of the present invention is more excellent in luminance life.

The crosslinkable group selected from Group A of crosslinkable group is preferably a group represented by formula (XL-2) to formula (XL-15) or formula (XL-17), more preferably a crosslinkable group represented by formula (XL-2) to formula (XL-4), formula (XL-7) to formula (XL-10), formula (XL-14), formula (XL-15) or formula (XL-17), still more preferably a crosslinkable group represented by formula (XL-3), formula (XL-9), formula (XL-10) or formula (XL-17), and particularly preferably a crosslinkable group represented by formula (XL-17), because the crosslinkable material is more excellent in crosslinkability.

### [Polymer Compound of Second Organic Layer]

The constitutional unit having at least one crosslinkable group selected from Group A of crosslinkable group contained in the polymer compound of the second organic layer is preferably a constitutional unit represented by formula (2) or a constitutional unit represented by formula (2'), and may be constitutional units represented by the following formulas.

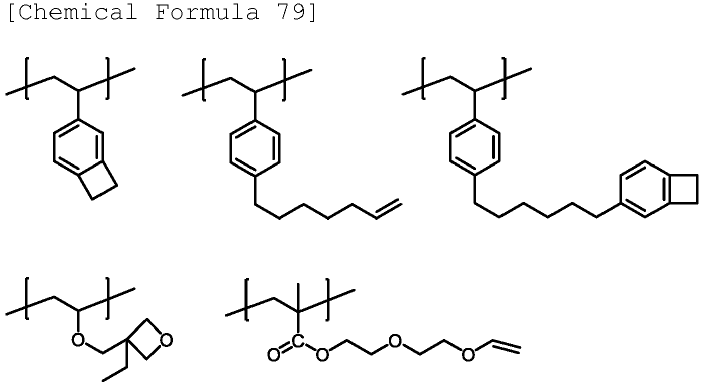

### [Constitutional Unit Represented by Formula (2)]

nA is preferably an integer of 0 to 3, more preferably an integer of 0 to 2, still more preferably 0 or 1, and particularly preferably 0, because the light emitting device according to the embodiment of the present invention is more excellent in luminance life.

n is preferably 2 because the light emitting device according to the embodiment of the present invention is more excellent in luminance life.

Ar³ is preferably an aromatic hydrocarbon group optionally having a substituent because the light emitting device according to the embodiment of the present invention is more excellent in luminance life.

The number of carbon atoms of the aromatic hydrocarbon group represented by Ar³, excluding the number of carbon atoms of a substituent, is usually 6 to 60, preferably 6 to 30, and more preferably 6 to 18.

The arylene group moiety obtained by removing n substituents of the aromatic hydrocarbon group represented by Ar³ is preferably a group represented by formula (A-1) to formula (A-20), more preferably a group represented by formula (A-1), formula (A-2), formula (A-6) to formula (A-10), formula (A-19) or formula (A-20), and still more preferably a group represented by formula (A-1), formula (A-2), formula (A-7), formula (A-9) or formula (A-19), and these groups each optionally have a substituent.

The number of carbon atoms of the heterocyclic group represented by Ar³, excluding the number of carbon atoms of a substituent, is usually 2 to 60, preferably 3 to 30, and more preferably 4 to 18.

The divalent heterocyclic moiety obtained by removing n substituents of the heterocyclic group represented by Ar³ is preferably a group represented by formula (AA-1) to formula (AA-34).

The aromatic hydrocarbon group and the heterocyclic group represented by Ar³ each optionally have a substituent, and the substituent is preferably an alkyl group, a cycloalkyl group, an alkoxy group, a cycloalkoxy group, an aryl group, an aryloxy group, a halogen atom, a monovalent heterocyclic group and a cyano group.

The alkylene group represented by L^{A}, excluding the number of carbon atoms of a substituent, is usually 1 to 20, preferably 1 to 15, and more preferably 1 to 10. The cycloalkylene group represented by L^{A}, excluding the number of carbon atoms of a substituent, is usually 3 to 20.

The alkylene group and cycloalkylene group each optionally have a substituent, and examples thereof include a methylene group, an ethylene group, a propylene group, a butylene group, a hexylene group, a cyclohexylene group and an octylene group.

The alkylene group and the cycloalkylene group represented by L^{A} each optionally have a substituent. The substituent which the alkylene group and the cycloalkylene group optionally have is preferably an alkyl group, a cycloalkyl group, an alkoxy group, a cycloalkoxy group, a halogen atom or a cyano group, and these groups each optionally further have a substituent.

The arylene group represented by L^{A} optionally has a substituent. The arylene group is preferably a phenylene group or a fluorenediyl group, and more preferably a m-phenylene group, a p-phenylene group, a fluorene-2,7-diyl group and a fluorene-9,9-diyl group. The substituent which the arylene group optionally has is preferably an alkyl group, a cycloalkyl group, an alkoxy group, a cycloalkoxy group, an aryl group, an aryloxy group, a monovalent heterocyclic group, a halogen atom, a cyano group, or a crosslinkable group selected from Group A of crosslinkable group, and these groups each optionally have a substituent.

The divalent heterocyclic group represented by L^{A} is preferably a group represented by formula (AA-1) to formula (AA-34) .

L^{A} is preferably an arylene group or an alkylene group, and more preferably a phenylene group, a fluorenediyl group or an alkylene group, and these groups each optionally have a substituent, because it becomes easy to produce a polymer compound of the second organic layer.

The crosslinkable group represented by X is a group represented by formula (XL-2) to formula (XL-15) or formula (XL-17), more preferably a crosslinkable group represented by formula (XL-2) to formula (XL-4), formula (XL-7) to formula (XL-10), formula (XL-14), formula (XL-15) or formula (XL-17), still more preferably a crosslinkable group represented by formula (XL-3), formula (XL-9), formula (XL-10) or formula (XL-17), and particularly preferably a crosslinkable group represented by formula (XL-17), because the polymer compound of the second organic layer is excellent in crosslinkability.

The crosslinkable group represented by X is preferably a crosslinkable group represented by formula (XL-1) to formula (XL-4), formula (XL-7) to formula (XL-10) or formula (XL-14) to formula (XL-17), more preferably a crosslinkable group represented by formula (XL-1), formula (XL-3), formula (XL-9), formula (XL-10), formula (XL-16) or formula (XL-17), still more preferably a crosslinkable group represented by formula (XL-1), formula (XL-16) or formula (XL-17), particularly preferably a crosslinkable group represented by formula (XL-1) or formula (XL-17), and especially preferably a crosslinkable group represented by formula (XL-17), because the light emitting device according to the embodiment of the present invention is more excellent in luminance life.

The content of the constitutional unit represented by formula (2) is preferably 0.5 to 80 mol%, more preferably 3 to 50 mol%, and still more preferably 5 to 20 mol%, based on the total content of the constitutional units contained in the polymer compound of the second organic layer, because the polymer compound of the second organic layer is excellent in stability and crosslinkability.

The polymer compound of the second organic layer may comprise the constitutional unit represented by formula (2) alone or two or more constitutional units.

### [Constitutional Unit Represented by Formula (2')]

mA is preferably an integer of 0 to 3, more preferably an integer of 0 to 2, still more preferably 0 or 1, and particularly preferably 0, because the light emitting device according to the embodiment of the present invention is more excellent in luminance life.

m is preferably 1 or 2, and more preferably 2, because the light emitting device according to the embodiment of the present invention is more excellent in luminance life.

c is preferably 0 because it becomes easy to produce the polymer compound of the second organic layer and the light emitting device according to the embodiment of the present invention is more excellent in luminance life.

Ar⁵ is preferably an aromatic hydrocarbon group optionally having a substituent because the light emitting device according to the embodiment of the present invention is more excellent in luminance life.

The definition and examples of the arylene group moiety obtained by removing m substituents of the aromatic hydrocarbon group represented by Ar⁵ are the same as the definition and examples of the arylene group represented by Ar^{X2} in formula (X) mentioned below.

The definition and examples of the divalent heterocyclic group moiety obtained by removing m substituents of the aromatic hydrocarbon group represented by Ar⁵ are the same as the definition and examples of the divalent heterocyclic group moiety represented by Ar^{X2} in formula (X) mentioned below.

The definition and examples of the divalent group obtained by removing m substituents of the group in which at least one aromatic hydrocarbon ring and at least one heterocyclic ring are bonded directly to each other represented by Ar⁵ are the same as the definition and examples of the divalent group in which at least one arylene group and at least one divalent heterocyclic group are bonded directly to each other represented by Ar^{X2} in formula (X) mentioned below.

Ar⁴ and Ar⁶ represent preferably an arylene group optionally having a substituent, because the light emitting device according to the embodiment of the present invention is more excellent in luminance life.

The definition and examples of the arylene group represented by Ar⁴ and Ar⁶ are the same as the definition and examples of the arylene group represented by Ar^{X1} and Ar^{X3} in formula (X) mentioned below.

The definition and examples of the divalent heterocyclic group represented by Ar⁴ and Ar⁶ are the same as the definition and examples of the divalent heterocyclic group represented by Ar^{X1} and Ar^{X3} in formula (X) mentioned below.

The groups represented by Ar⁴, Ar⁵ and Ar⁶ each optionally have a substituent, and the substituent includes an alkyl group, a cycloalkyl group, an alkoxy group, a cycloalkoxy group, an aryl group, an aryloxy group, a halogen atom, a monovalent heterocyclic group and a cyano group.

The definition and examples of the alkylene group, the cycloalkylene group, the arylene group and the divalent heterocyclic group represented by K^{A} are the same as the definition and examples of the alkylene group, the cycloalkylene group, the arylene group and the divalent heterocyclic group represented by L^{A}, respectively.

K^{A} is preferably a phenylene group or a methylene group, because it becomes easy to produce the polymer compound of the second organic layer.

The definition and examples of the crosslinkable group represented by X' are the same as the definition and examples of the crosslinkable group represented by X mentioned above.

The content of the constitutional unit represented by formula (2') is preferably 0.5 to 50 mol%, more preferably 3 to 30 mol%, and still more preferably 5 to 20 mol%, based on the total content of the constitutional units contained in the polymer compound of the second organic layer, because the polymer compound of the second organic layer is excellent in stability and the polymer compound of the second organic layer is excellent in crosslinkability.

The polymer compound of the second organic layer may comprise the constitutional unit represented by formula (2') alone or two or more constitutional units.

### [Preferred Aspect of Constitutional Unit Represented by Formula (2) or (2')]

The constitutional unit represented by formula (2) includes, for example, a constitutional unit represented by formula (2-1) to formula (2-30) and the constitutional unit represented by formula (2') includes, for example, a constitutional unit represented by formula (2'-1) to formula (2'-9). Of these constitutional units, a constitutional unit represented by formula (2-1) to formula (2-30) is preferable, a constitutional unit represented by formula (2-1) to formula (2-15), formula (2-19), formula (2-20), formula (2-23), formula (2-25) or formula (2-30) is more preferable, and a constitutional unit represented by formula (2-1) to formula (2-9) or formula (2-30) is still more preferable, because the polymer compound of the second organic layer is excellent in crosslinkability.

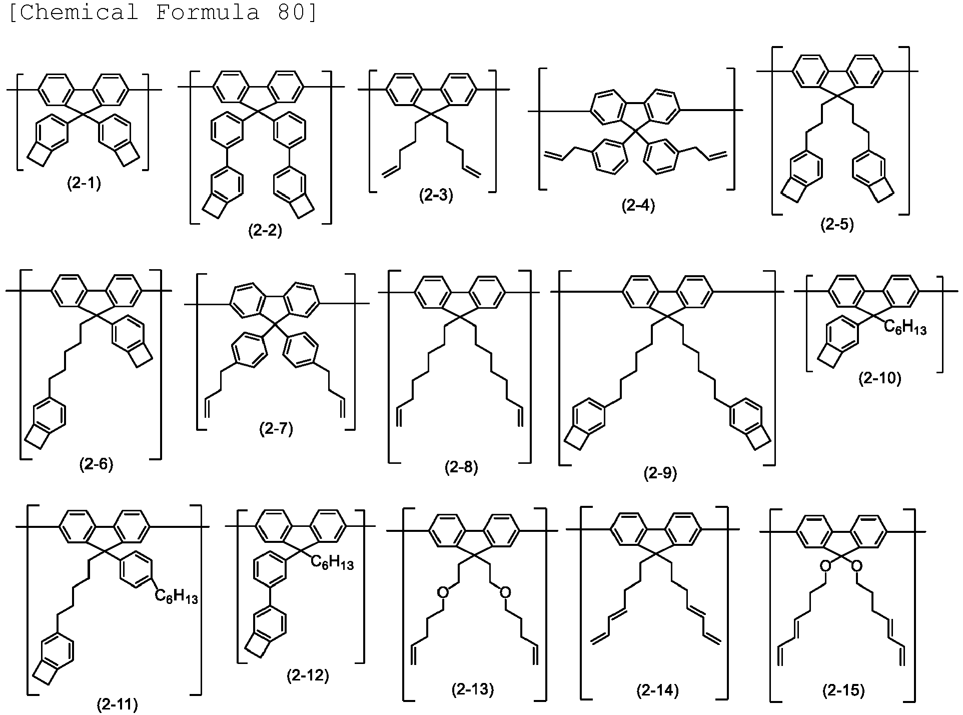

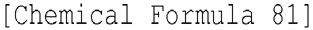

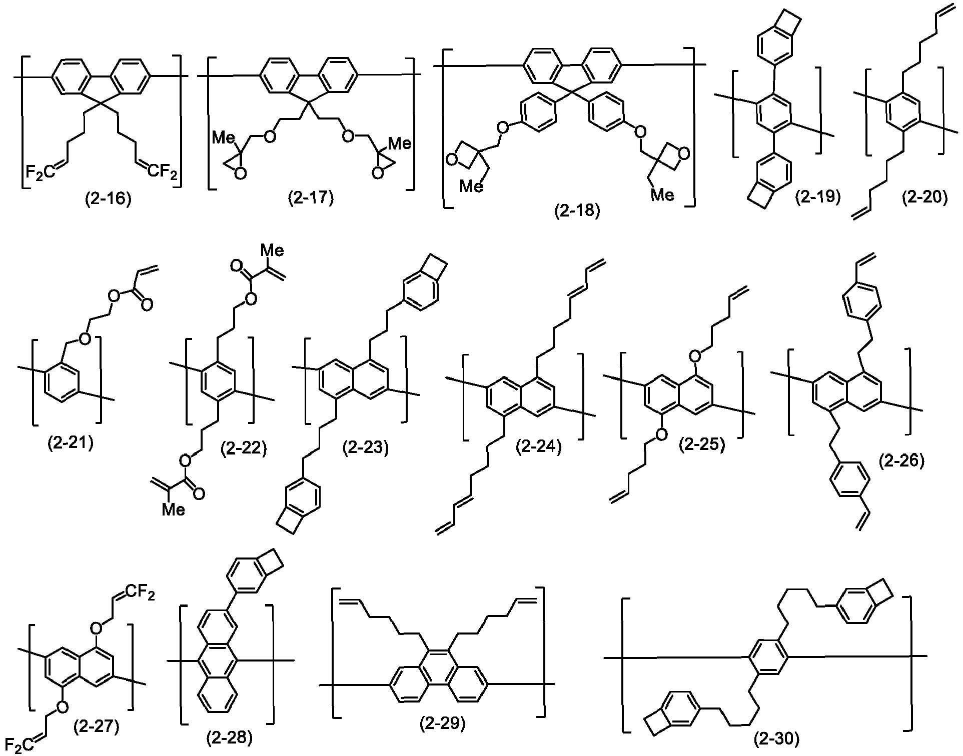

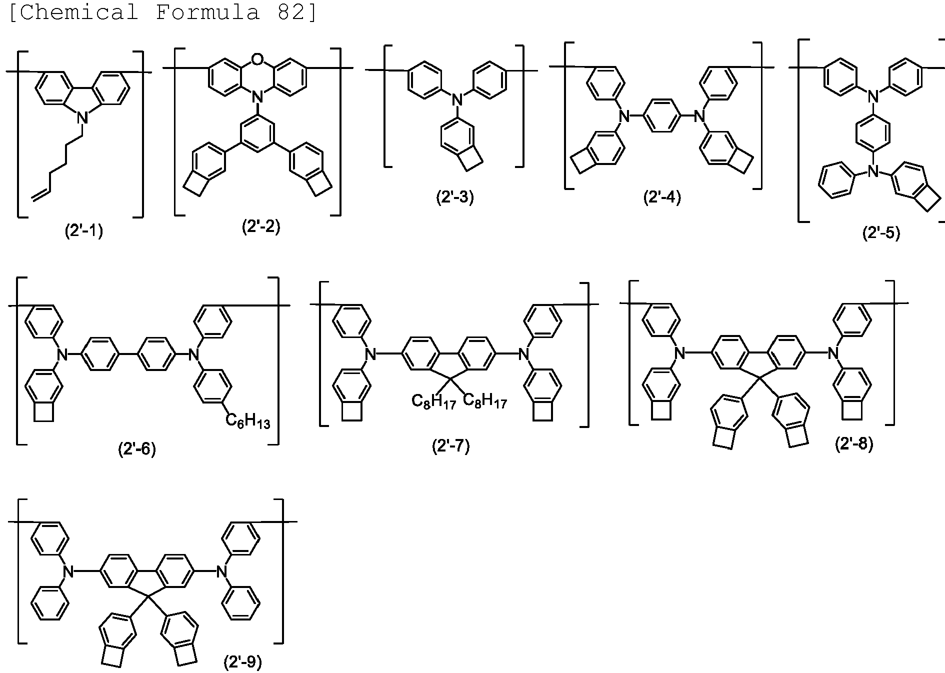

### [Other Constitutional Units]

It is preferable that the polymer compound of the second organic layer further comprises a constitutional unit represented by formula (X), because the hole transportability is excellent. It is preferable that the polymer compound of the second organic layer further comprises a constitutional unit represented by formula (Y), because the light emitting device according to the embodiment of the present invention is more excellent in luminance life.

Since the polymer compound of the second organic layer is excellent in hole transportability and the light emitting device according to the embodiment of the present invention is more excellent in luminance life, the polymer compound of the second organic layer further comprises a constitutional unit represented by formula (X) and a constitutional unit represented by formula (Y): 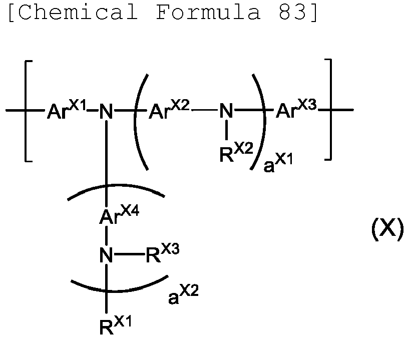 wherein
a^{X1} and a^{X2} each independently represent an integer of 0 or more,
Ar^{X1} and Ar^{X3} each independently represent an arylene group or a divalent heterocyclic group, and these groups each optionally have a substituent,
Ar^{X2} and Ar^{X4} each independently represent an arylene group, a divalent heterocyclic group, or a divalent group in which at least one arylene group and at least one divalent heterocyclic ring group are bonded directly to each other, and these groups each optionally have a substituent, and when a plurality of Ar^{X2} and Ar^{X4} are present, they may be the same or different at each occurrence, and
R^{x1}, R^{X2} and R^{X3} each independently represent a hydrogen atom, an alkyl group, a cycloalkyl group, an aryl group or a monovalent heterocyclic group, and these groups each optionally have a substituent, and when a plurality of R^{X2} and R^{X3} are present, they may be the same or different at each occurrence.

a^{X1} preferably an integer of 2 or less, and more preferably 1, because the light emitting device according to the embodiment of the present invention is more excellent in luminance life.

a^{X2} is preferably an integer of 2 or less, and more preferably 0, because the light emitting device according to the embodiment of the present invention is more excellent in luminance life.

R^{X1}, R^{X2} and R^{X3} represent preferably an alkyl group, a cycloalkyl group, an aryl group or a monovalent heterocyclic group, and more preferably an aryl group, and these groups each optionally have a substituent.

The arylene group represented by Ar^{X1} and Ar^{X3} is more preferably a group represented by formula (A-1) or formula (A-9), and still more preferably a group represented by formula (A-1), and these groups each optionally have a substituent.

The divalent heterocyclic group represented by Ar^{X1} and Ar^{X3} is more preferably a group represented by formula (AA-1), formula (AA-2) or formula (AA-7) to formula (AA-26), and these groups each optionally have a substituent.

Ar^{X1} and Ar^{X3} represent preferably an arylene group optionally having a substituent.

The arylene group represented by Ar^{X2} and Ar^{X4} is more preferably a group represented by formula (A-1), formula (A-6), formula (A-7), formula (A-9) to formula (A-11) or formula (A-19), and these groups each optionally have a substituent.

The more preferable range of the divalent heterocyclic group represented by Ar^{X2} and Ar^{X4} is the same as the more preferable range of the divalent heterocyclic group represented by Ar^{X1} and Ar^{X3}.

The more preferable range and the further preferable range of the arylene group and the divalent heterocyclic group in the divalent group in which at least one arylene group and at least one divalent heterocyclic group are bonded directly to each other represented by Ar^{X2} and Ar^{X4} are the same as the more preferable range and the further preferable range of the arylene group and the divalent heterocyclic group represented by Ar^{X1} and Ar^{X3}, respectively.

The divalent group in which at least one arylene group and at least one divalent heterocyclic group are bonded directly to each other represented by Ar^{X2} and Ar^{X4} includes, for example, groups represented by the following formulas, and they each optionally have a substituent: 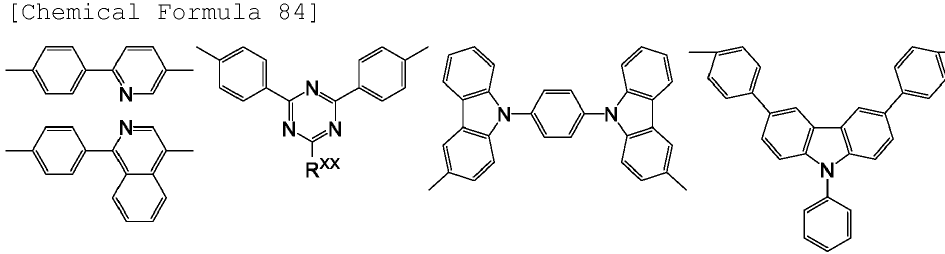 wherein R^{XX} represents a hydrogen atom, an alkyl group, a cycloalkyl group, an aryl group or a monovalent heterocyclic group, and these groups each optionally have a substituent.

R^{XX} is preferably an alkyl group, a cycloalkyl group or an aryl group, and these groups each optionally have a substituent.

Ar^{X2} and Ar^{X4} represent preferably an arylene group optionally having a substituent.

The substituent which the group represented by Ar^{X1} to Ar^{X4} and R^{X1} to R^{X3} optionally has is preferably an alkyl group, a cycloalkyl group or an aryl group, and these groups each optionally have a substituent.

The constitutional unit represented by formula (X) is preferably a constitutional unit represented by formula (X-1) to formula (X-7), more preferably a constitutional unit represented by formula (X-3) to formula (X-7), and still more preferably a constitutional unit represented by formula (X-3) to formula (X-6): 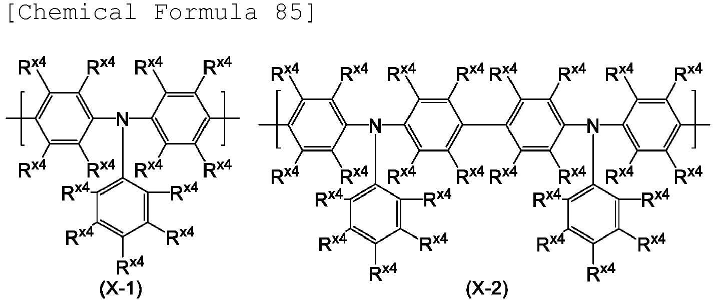 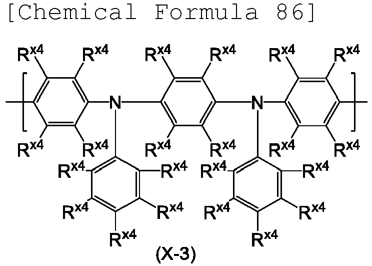 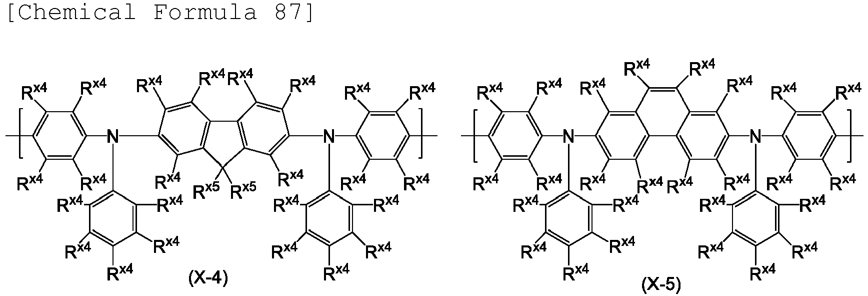 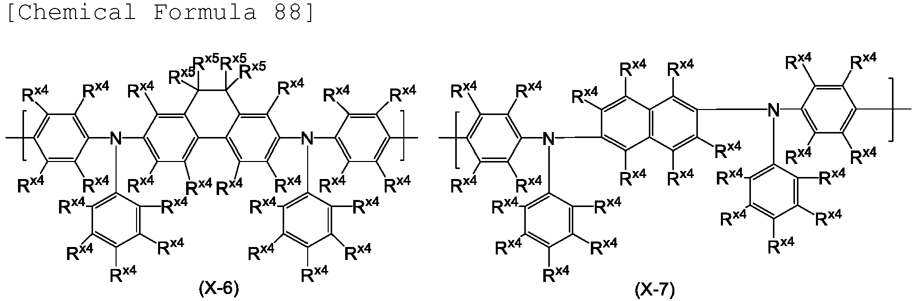 wherein R^{X4} and R^{X5} each independently represent a hydrogen atom, an alkyl group, a cycloalkyl group, an alkoxy group, a cycloalkoxy group, an aryl group, an aryloxy group, a halogen atom, a monovalent heterocyclic group or a cyano group, and these groups each optionally have a substituent, the plurality of R^{X4} may be the same or different, the plurality of R^{X5} may be the same or different, and adjacent R^{X5} may be combined together to form a ring together with the carbon atoms to which they are attached.

The content of the constitutional unit represented by formula (X) is preferably 0.1 to 90 mol%, more preferably 1 to 70 mol%, and still more preferably 10 to 50 mol%, based on the total content of the constitutional units contained in the polymer compound of the second organic layer, because the hole transportability is excellent.

The constitutional unit represented by formula (X) includes, for example, a constitutional unit represented by formula (X1-1) to formula (X1-19), and preferably a constitutional unit represented by formula (X1-6) to formula (X1-14).

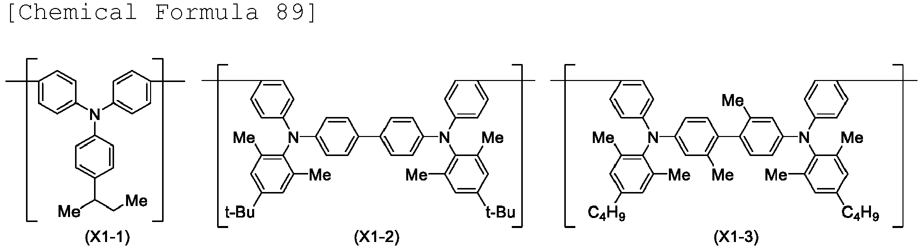

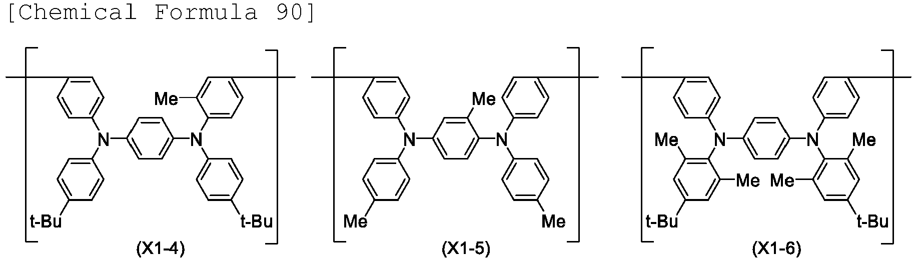

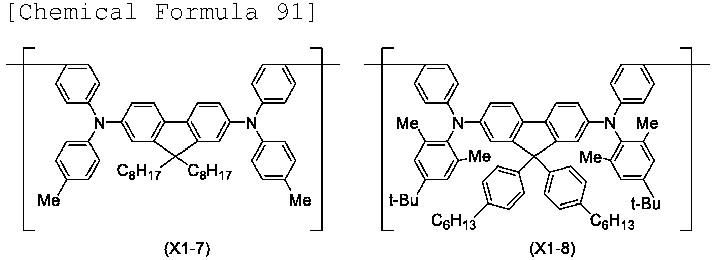

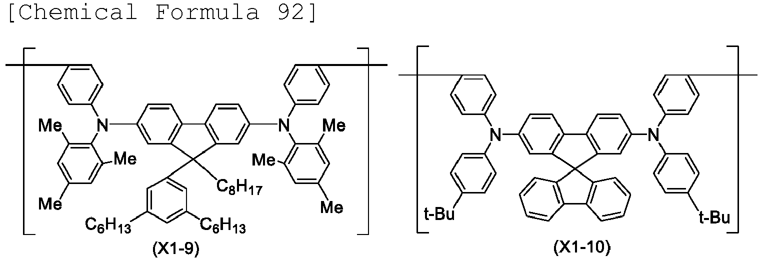

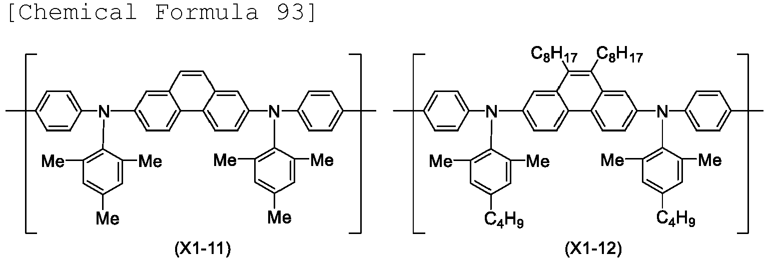

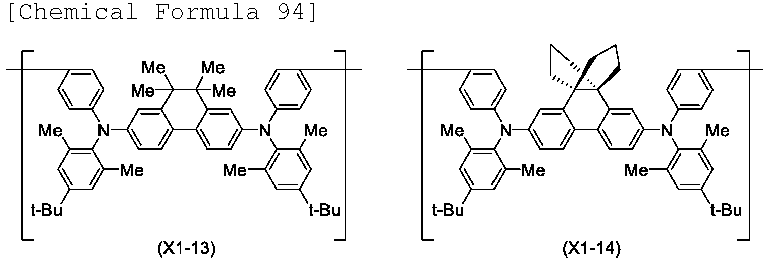

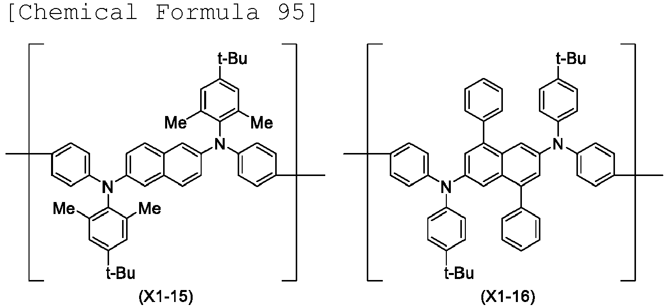

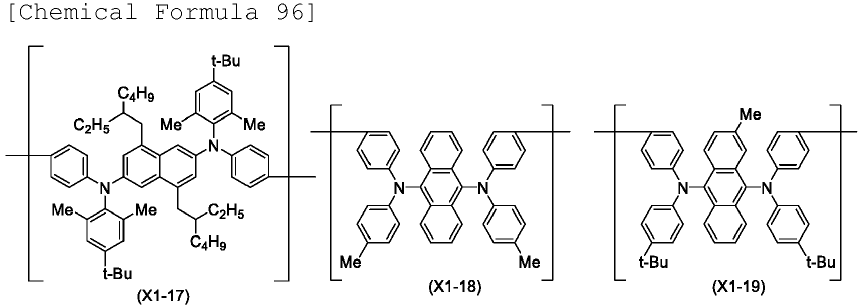

The polymer compound of the second organic layer may comprise the constitutional unit represented by formula (X) alone or two or more constitutional units:

[Chemical Formula 97] **-[-Ar^{Y1}-]- (Y)**

wherein Ar^{Y1} represents an arylene group, a divalent heterocyclic ring group, or a divalent group in which at least one arylene group and at least one divalent heterocyclic ring group are bonded directly to each other, and these groups each optionally have a substituent.

The arylene group represented by Ar^{Y1} is more preferably a group represented by formula (A-1), formula (A-6), formula (A-7), formula (A-9) to formula (A-11), formula (A-13) or formula (A-19), and still more preferably a group represented by formula (A-1), formula (A-7), formula (A-9) or formula (A-19), and these groups each optionally have a substituent.

The divalent heterocyclic group represented by Ar^{Y1} is more preferably a group represented by formula (AA-4), formula (AA-10), formula (AA-13), formula (AA-15), formula (AA-18) or formula (AA-20), and still more preferably a group represented by formula (AA-4), formula (AA-10), formula (AA-18) or formula (AA-20), and these groups each optionally have a substituent.

The more preferable range and the still more preferable range of the arylene group and the divalent heterocyclic group in the divalent group in which at least one arylene group and at least one divalent heterocyclic group are bonded directly to each other represented by Ar^{Y1} are the same as the more preferable range and the still more preferable range of the arylene group and the divalent heterocyclic group represented by Ar^{Y1} mentioned above, respectively.

The divalent group in which at least one arylene group and at least one divalent heterocyclic group are bonded directly to each other represented by Ar^{Y1} includes the same groups as the divalent group in which at least one arylene group and at least one divalent heterocyclic group are bonded directly to each other represented by Ar^{X2} and Ar^{X4} in formula (X).

The substituent which the group represented by Ar^{Y1} optionally has is preferably an alkyl group, a cycloalkyl group or an aryl group, and these groups each optionally have a substituent.

The constitutional unit represented by formula (Y) includes, for example, constitutional units represented by formulas (Y-1) to (Y-7), and is preferably a constitutional unit represented by formula (Y-1) or formula (Y-2) from the viewpoint of the luminance life of the light emitting device according to the embodiment of the present invention, preferably a constitutional unit represented by formula (Y-3) or formula (Y-4) from the viewpoint of the electron transportability of the polymer compound of the second organic layer, and preferably a constitutional unit represented by formula (Y-5) to formula (Y-7) from the viewpoint of the hole transportability of the polymer compound of the second organic layer: 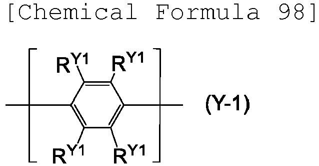 wherein R^{Y1} represents a hydrogen atom, an alkyl group, a cycloalkyl group, an alkoxy group, a cycloalkoxy group, an aryl group or a monovalent heterocyclic group, and these groups each optionally have a substituent, the plurality of R^{Y1} may be the same or different, and adjacent R^{Y1} may be combined together to form a ring together with the carbon atoms to which they are attached.

R^{Y1} is preferably a hydrogen atom, an alkyl group, a cycloalkyl group or an aryl group, and these groups each optionally have a substituent.

The constitutional unit represented by formula (Y-1) is preferably a constitutional unit represented by formula (Y-1') : 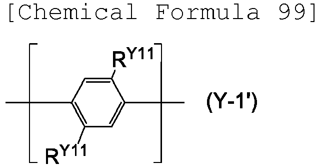 wherein R^{Y11} represents an alkyl group, a cycloalkyl group, an alkoxy group, a cycloalkoxy group, an aryl group or a monovalent heterocyclic group, and these groups each optionally have a substituent, and the plurality of R^{Y11} may be the same or different.

R^{Y11} is preferably an alkyl group, a cycloalkyl group or an aryl group, and more preferably an alkyl group or a cycloalkyl group, and these groups each optionally have a substituent: 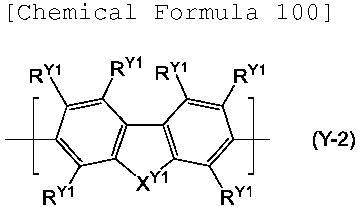 wherein
R^{Y1} represents the same meaning as defined above, and
X^{Y1} represents a group represented by -C(R^{Y2})₂-, - C(R^{Y2})=C(R^{Y2})- or -C(R^{Y2})₂-C(R^{Y2})₂ R^{Y2} represents a hydrogen atom, an alkyl group, a cycloalkyl group, an alkoxy group, a cycloalkoxy group, an aryl group or a monovalent heterocyclic group, and these groups each optionally have a substituent, the plurality of R^{Y2} may be the same or different, and R^{Y2} may be combined together to form a ring together with the carbon atoms to which they are attached.

R^{Y2} is preferably an alkyl group, a cycloalkyl group, an aryl group or a monovalent heterocyclic group, and more preferably an alkyl group, a cycloalkyl group or an aryl group, and these groups each optionally have a substituent.

Regarding the combination of two R^{Y2} in the group represented by -C(R^{Y2})₂- in X^{Y1}, it is preferable that the both are an alkyl group or a cycloalkyl group, the both are an aryl group, the both are a monovalent heterocyclic group, or one is an alkyl group or a cycloalkyl group and the other is an aryl group or a monovalent heterocyclic group, it is more preferable that one is an alkyl group or cycloalkyl group and the other is an aryl group, and these groups each optionally have a substituent. The two groups R^{Y2} may be combined together to form a ring together with the atoms to which they are attached, and when the groups R^{Y2} form a ring, the group represented by -C(R^{Y2})₂- is preferably a group represented by formula (Y-A1) to (Y-A5), more preferably a group represented by formula (Y-A4), and these groups each optionally have a substituent.

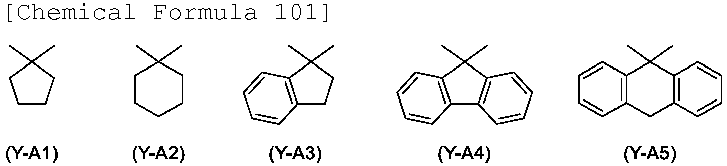

Regarding the combination of two R^{Y2} in the group represented by -C(R^{Y2})=C(R^{Y2})- in X^{Y1}, it is preferable that the both are an alkyl group or a cycloalkyl group, or one is an alkyl group or a cycloalkyl group and the other is an aryl group, and these groups each optionally have a substituent

Four R^{Y2} in the group represented by -C(R^{Y2})₂-C(R^{Y2})₂- in X^{Y1} are preferably an alkyl group or a cycloalkyl group optionally having a substituent. The plurality of R^{Y2} may be combined together to form a ring together with the atoms to which they are attached, and when the groups R^{Y2} form a ring, the group represented by -C(R^{Y2})₂-C(R^{Y2})₂- is preferably a group represented by formula (Y-B1) to (Y-B5), more preferably a group represented by formula (Y-B3), and these groups each optionally have a substituent: 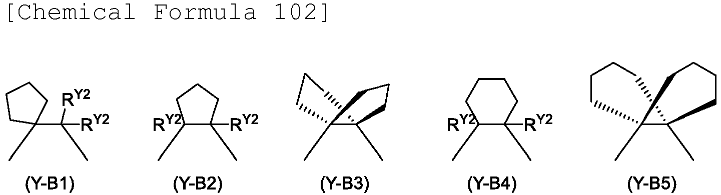 wherein R^{Y2} represents the same meaning as defined above.

The constitutional unit represented by formula (Y-2) is preferably a constitutional unit represented by formula (Y-2') : 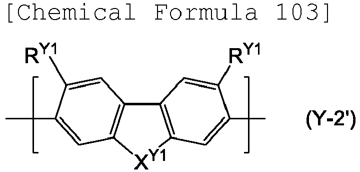 wherein R^{Y1} and X^{Y1} represent the same meaning as defined above: 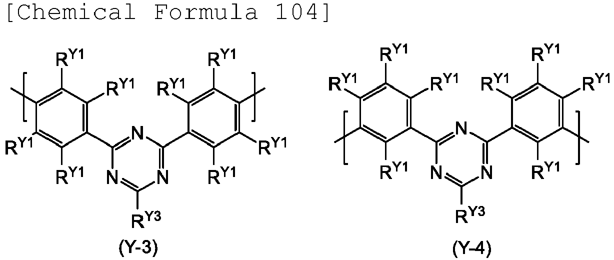 wherein
R^{Y1} represents the same meaning as defined above, and
R^{Y3} represents a hydrogen atom, an alkyl group, a cycloalkyl group, an alkoxy group, a cycloalkoxy group, an aryl group or a monovalent heterocyclic group, and these groups each optionally have a substituent.

R^{Y3} is preferably an alkyl group, a cycloalkyl group, an alkoxy group, a cycloalkoxy group, an aryl group or a monovalent heterocyclic group, and more preferably an aryl group, and these groups each optionally have a substituent: 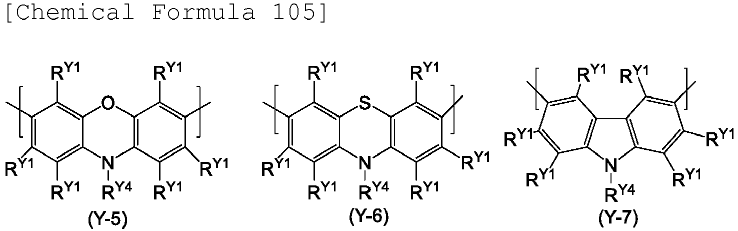 wherein
R^{Y1} represents the same meaning as defined above, and
R^{Y4} represents a hydrogen atom, an alkyl group, a cycloalkyl group, an alkoxy group, a cycloalkoxy group, an aryl group or a monovalent heterocyclic group, and these groups each optionally have a substituent.

R^{Y4} is preferably an alkyl group, a cycloalkyl group, an alkoxy group, a cycloalkoxy group, an aryl group or a monovalent heterocyclic group, and more preferably an aryl group, and these groups each optionally have a substituent.

The constitutional unit represented by formula (Y) includes, for example, a constitutional unit represented by formula (Y-11) to formula (Y-56), and is preferably a constitutional unit represented by formula (Y-11) to formula (Y-55).

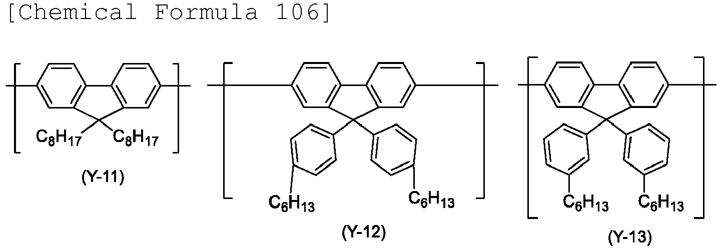

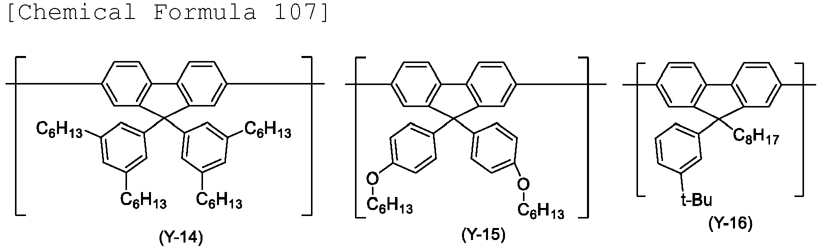

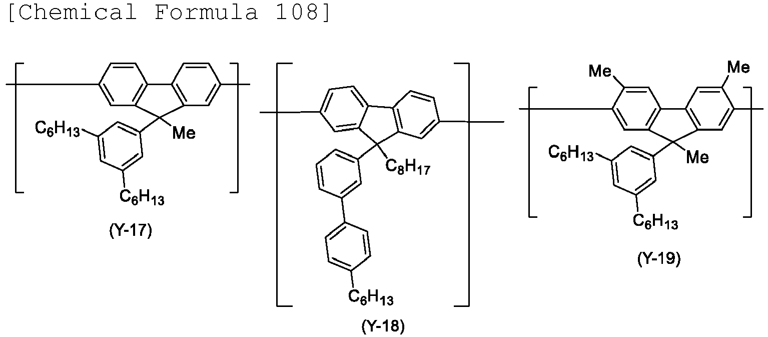

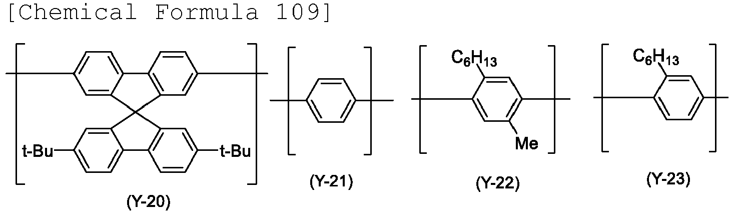

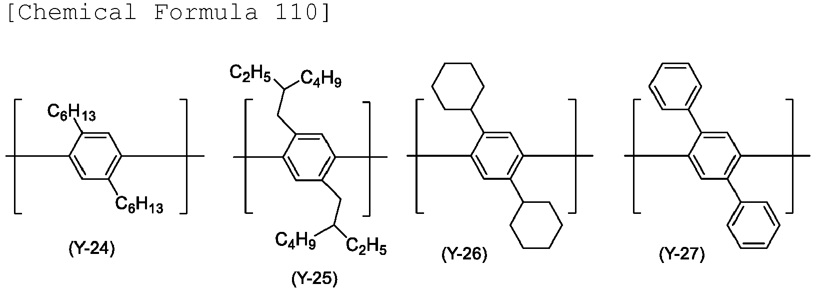

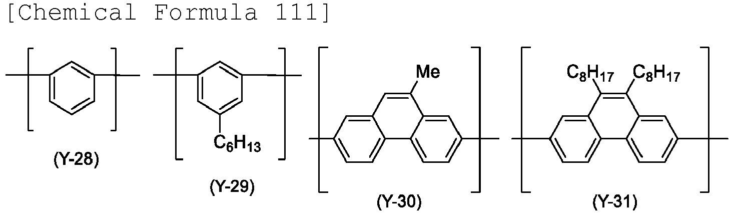

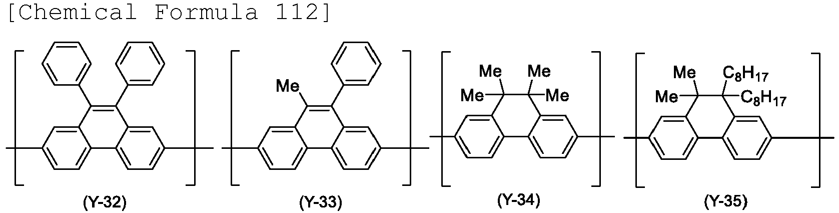

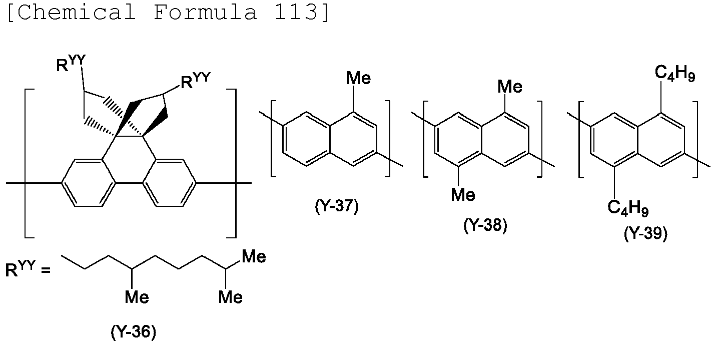

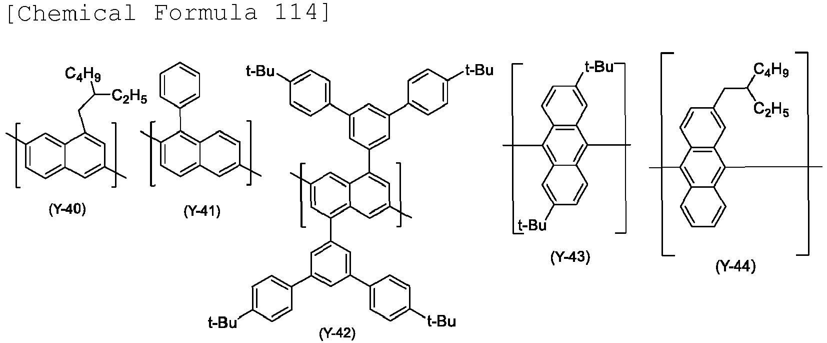

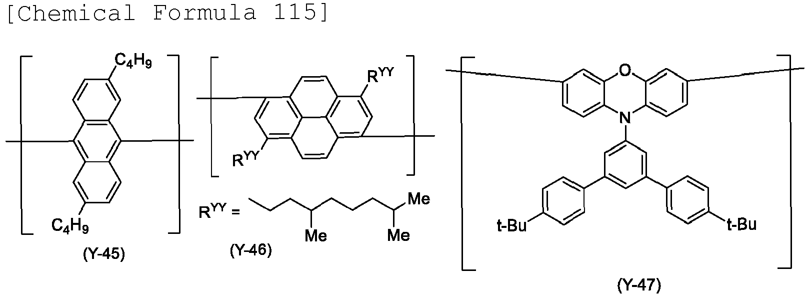

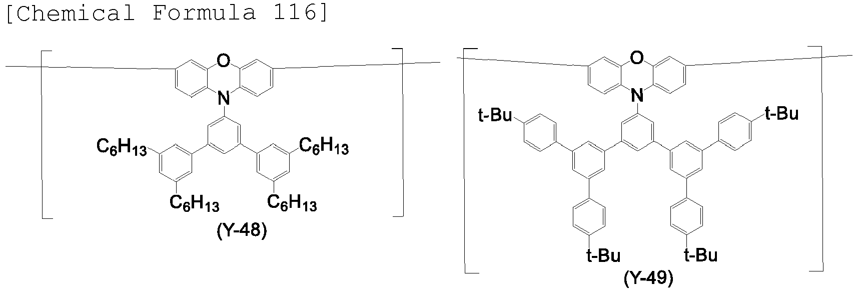

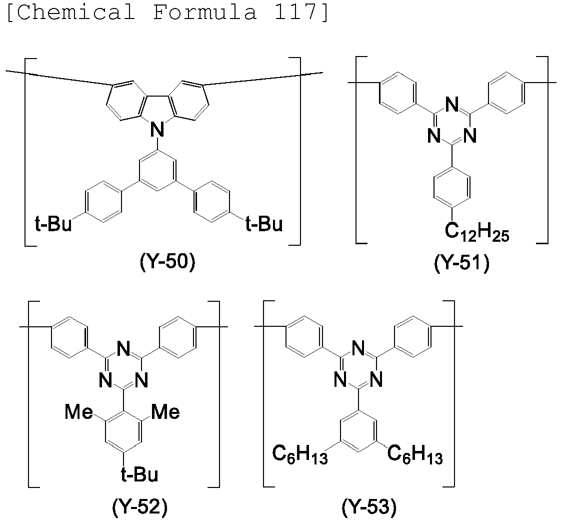

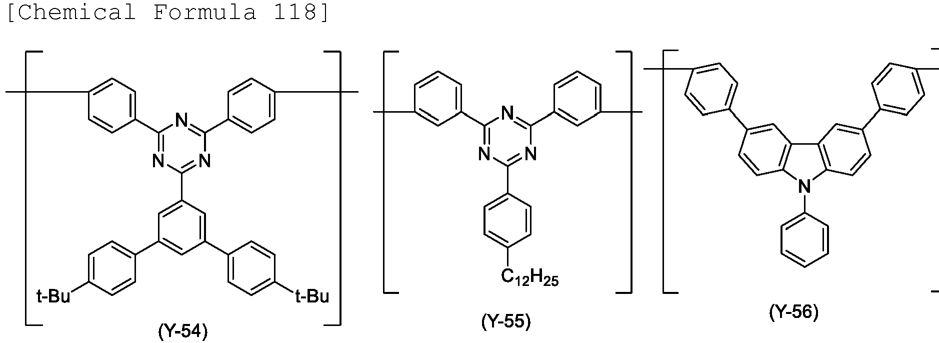

The content of the constitutional unit represented by formula (Y) in which Ar^{Y1} is an arylene group is preferably 0.5 to 80 mol%, and more preferably 30 to 60 mol%, based on the total content of constitutional units contained in the polymer compound of the second organic layer, because the light emitting device according to the embodiment of the present invention is more excellent in luminance life.

The content of the constitutional unit represented by formula (Y) in which Ar^{Y1} is a divalent heterocyclic group or a divalent group in which at least one arylene group and at least one divalent heterocyclic group are bonded directly to each other is preferably 0.5 to 40 mol%, and more preferably 3 to 30 mol%, based on the total content of constitutional units contained in the polymer compound of the second organic layer, because the polymer compound of the second organic layer is excellent in charge transportability.

The polymer compound of the second organic layer may comprise the constitutional unit represented by formula (Y) alone or two or more constitutional units.

The polymer compound of the second organic layer includes, for example, polymer compounds P-1 to P-8 shown in Table 1. "Other constitutional unit" means a constitutional unit other than constitutional units represented by formula (2), formula (2'), formula (X) and formula (Y).

**[Table 1]**

| Polymer compound | Constitutional unit and molar ratio thereof | | | | |
|---|---|---|---|---|---|
| | Formula (2) | Formula (2') | Formula (X) | Formula (Y) | Others |
| | p' | q' | r' | s' | t' |
| P-1 | 0.1 to 99.9 | 0.1 to 99.9 | 0 | 0 | 0 to 30 |
| P-2 | 0.1 to 99.9 | 0 | 0.1 to 99.9 | 0 | 0 to 30 |
| P-3 | 0.1 to 99.9 | 0 | 0 | 0.1 to 99.9 | 0 to 30 |
| P-4 | 0 | 0.1 to 99.9 | 0.1 to 99.9 | 0 | 0 to 30 |
| P-5 | 0 | 0.1 to 99.9 | 0 | 0.1 to 99.9 | 0 to 30 |
| P-6 | 0.1 to 99.8 | 0.1 to 99.8 | 0.1 to 99.8 | 0 | 0 to 30 |
| P-7 | 0.1 to 99.8 | 0.1 to 99.8 | 0 | 0.1 to 99.8 | 0 to 30 |
| P-8 | 0.1 to 99.7 | 0.1 to 99.7 | 0.1 to 99.7 | 0.1 to 99.7 | 0 to 30 |

[In the table, p', q', r', s' and t' represent the molar ratio of each constitutional unit. p'+q'+r'+s'+t' = 100 and 70 ≤ p'+q'+r'+s' ≤ 100.]

The examples and preferable range of constitutional units represented by formula (2), formula (2'), formula (X) and formula (Y) in the polymer compounds P-1 to P-8 are as mentioned above.

The polystyrene-equivalent number average molecular weight of the polymer compound of the second organic layer is preferably 5 × 10³ to 1 × 10⁶, more preferably 1 × 10⁴ to 5 × 10⁵, and still more preferably 1.5 × 10⁴ to 1 × 10⁵.

### [Method for Producing Polymer Compound of Second Organic Layer]

The polymer compound of the second organic layer can be produced using known polymerization methods disclosed in Chem. Rev., Vol. 109, pp.897-1091 (2009) and the like, and the known polymerization methods include, for example, polymerization methods by a coupling reaction using a transition metal catalyst, such as the Suzuki reaction, the Yamamoto reaction, the Buchwald reaction, the Stille reaction, the Negishi reaction and the Kumada reaction.

In the above-mentioned polymerization methods, the method of charging monomers includes a method in which the total amount of monomers is charged in a mass into the reaction system, a method in which monomers are partially charged and reacted and then the remaining monomers are charged in a mass continuously or dividedly, a method in which monomers are charged continuously or dividedly and the like.

The transition metal catalyst includes a palladium catalyst and a nickel catalyst.

The post treatment of the polymerization reaction is performed by using known methods, for example, a method in which water-soluble impurities are removed by liquid separation, a method in which the reaction solution after the polymerization reaction is added to a lower alcohol such as methanol, followed by filtration of the resulting precipitate and further drying, alone or in combination. When a polymer host has low purity, the polymer host can be purified by usual methods, for example, crystallization, reprecipitation, continuous extraction using a Soxhlet extractor, column chromatography and the like.

### [Low Molecular Weight Compound of Second Organic Layer]

The low molecular weight compound of the second organic layer is preferably a low molecular weight compound represented by formula (3): 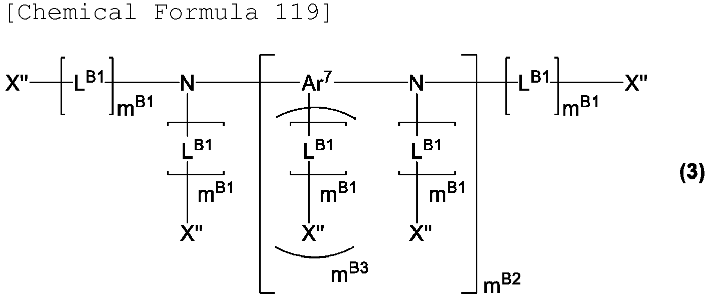 wherein
m^{B1}, m^{B2} and m^{B3} each independently represent an integer of 0 or more, the plurality of m^{B1} may be the same or different, and when a plurality of m^{B3} are present, they may be the same or different,
Ar⁷ represents an aromatic hydrocarbon group, a heterocyclic group, or a group in which at least one aromatic hydrocarbon ring and at least one heterocyclic ring are bonded directly to each other, and these groups each optionally have a substituent, and when a plurality of Ar⁷ are present, they may be the same or different,
L^{B1} represents an alkylene group, a cycloalkylene group, an arylene group, a divalent heterocyclic group, a group represented by -N(R"')-, an oxygen atom or a sulfur atom, these groups each optionally have a substituent, R"' represents a hydrogen atom, an alkyl group, a cycloalkyl group, an aryl group or a monovalent heterocyclic group, and these groups each optionally have a substituent, and when a plurality of L^{B1} are present, they may be the same or different, and
X" represents a crosslinkable group, a hydrogen atom, an alkyl group, a cycloalkyl group, an aryl group or a monovalent heterocyclic group, and these groups each optionally have a substituent, the plurality of X" may be the same or different, and at least one of the plurality of X" is a crosslinkable group.

m^{B1} is usually an integer of 0 to 10, preferably an integer of 0 to 5, more preferably an integer of 0 to 2, still more preferably 0 or 1, and particularly preferably 0, because it becomes easy to synthesize a crosslinkable material.

m^{B2} is usually an integer of 0 to 10, preferably an integer of 0 to 5, more preferably an integer of 0 to 3, still more preferably 1 or 2, and particularly preferably 1, because it becomes easy to synthesize a crosslinkable material and the light emitting device according to the embodiment of the present invention is more excellent in luminance life.

m^{B3} is usually an integer of 0 to 5, preferably an integer of 0 to 4, more preferably an integer of 0 to 2, and still more preferably 0, because it becomes easy to synthesize a crosslinkable material.

The definition and examples of the arylene group moiety obtained by removing m^{B3} substituents of the aromatic hydrocarbon group represented by Ar⁷ are the same as the definition and examples of the arylene group represented by Ar^{X2} in formula (X) mentioned above.

The definition and examples of the divalent heterocyclic group moiety obtained by removing m^{B3} substituents of the heterocyclic group represented by Ar⁷ are the same as the definition and examples of the divalent heterocyclic group moiety represented by Ar^{X2} in formula (X) mentioned above.

The definition and examples of the divalent group obtained by removing m^{B3} substituents of the group in which at least one aromatic hydrocarbon ring and at least one heterocyclic ring are bonded directly to each other represented by Ar⁷ are the same as the definition and examples of the divalent group in which at least one arylene group and at least one divalent heterocyclic group are bonded directly to each other represented by Ar^{X2} in formula (X) mentioned above.

The definition and examples of the substituent which Ar⁷ optionally has are the same as the definition and examples of the substituent which the group represented by Ar^{X2} in formula (X) mentioned above.

Ar⁷ is preferably an aromatic hydrocarbon group because the light emitting device according to the embodiment of the present invention is excellent in luminance life, and this aromatic hydrocarbon group optionally has a substituent.

The definition and examples of the alkylene group, the cycloalkylene group, the arylene group and the divalent heterocyclic group represented by L^{B1} are the same as the definition and examples of the alkylene group, the cycloalkylene group, the arylene group and the divalent heterocyclic group represented by L^{A} mentioned above, respectively.

L^{B1} is preferably an alkylene group, an arylene group or an oxygen atom, more preferably an alkylene group or an arylene group, still more preferably a phenylene group, a fluorenediyl group or an alkylene group, particularly preferably a phenylene group or an alkylene group, and these groups each optionally have a substituent, because it becomes easy to synthesize a crosslinkable material.

X" is preferably a crosslinkable group represented by any one of formulas (XL-1) to (XL-17), an aryl group or a monovalent heterocyclic group, more preferably a crosslinkable group represented by formula (XL-1), (XL-3), (XL-7) to (XL-10), (XL-16) or (XL-17) or an aryl group, still more preferably a crosslinkable group represented by formula (XL-1), (XL-16) or (XL-17), a phenyl group, a naphthyl group or a fluorenyl group, particularly preferably a crosslinkable group represented by formula (XL-16) or (XL-17), a phenyl group or a naphthyl group, and especially preferable a crosslinkable group represented by formula (XL-16) or a naphthyl group, and these groups each optionally have a substituent.

The crosslinkable material includes, for example, low molecular weight compounds represented by formulas (3-1) to (3-16), and the crosslinkable materials are preferably low molecular weight compounds represented by formulas (3-1) to (3-10), and more preferably low molecular weight compounds represented by formulas (3-5) to (3-9).

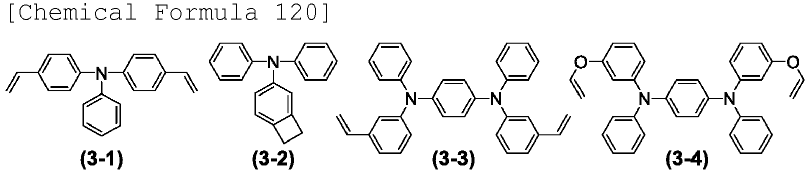

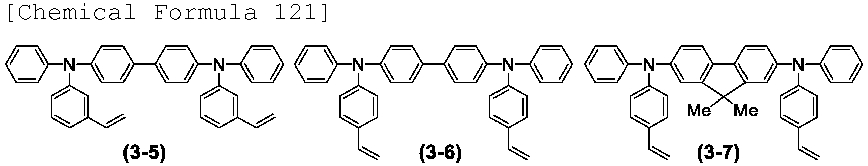

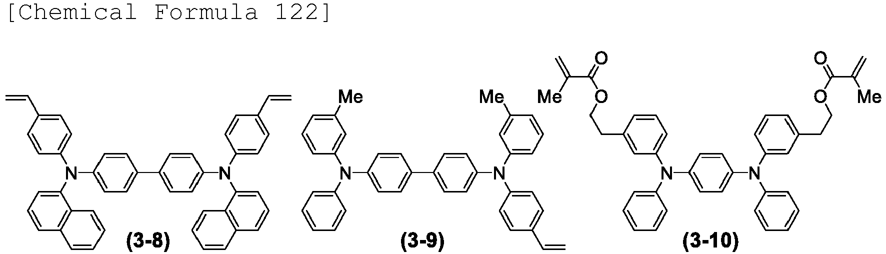

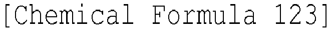

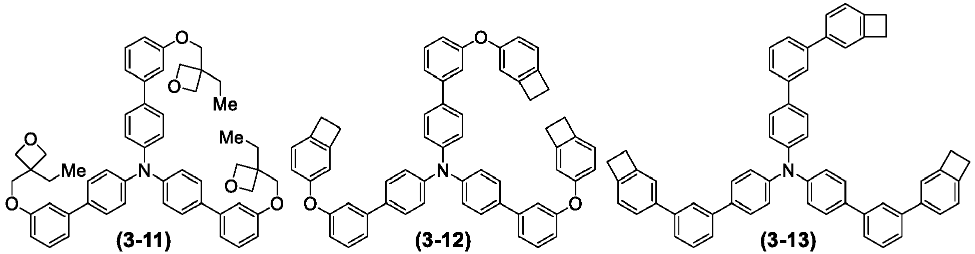

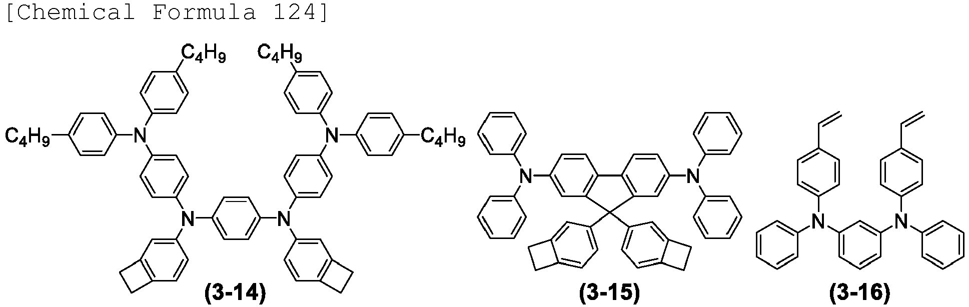

The low molecular weight compound of the second organic layer is available from Aldrich, Luminescence Technology Corp., American Dye Source and the like. The low molecular weight compound can be synthesized according to methods disclosed, for example, in WO 1997/033193, WO 2005/035221 and WO 2005/049548.

In the second organic layer, a crosslinked body of a crosslinkable material may be contained alone or two or more thereof may be contained.

### [Second Composition]

The second organic layer may be a layer comprising a composition comprising a crosslinked body of a crosslinkable material and at least one material selected from the group consisting of a hole transporting material, a hole injection material, an electron transporting material, an electron injection material, a light emitting material and an antioxidant (hereinafter also referred to as "second composition").

The examples and the preferable range of the hole transporting material, the electron transporting material, the hole injection material, the electron injection material and the light emitting material contained in the second composition are the same as are the same as the examples and the preferable range of the hole transporting material, the electron transporting material, the hole injection material, the electron injection material and the light emitting material contained in the first composition. In the second composition, the amounts of the hole transporting material, the electron transporting material, the hole injection material, the electron injection material and the light emitting material are each usually 1 to 400 parts by weight, and preferably 5 to 150 parts by weight, when the amount of the crosslinked body of the crosslinkable material is 100 parts by weight.

The examples and the preferable range of the antioxidant contained in the second composition are the same as the examples and the preferable range of the antioxidant contained in the first composition. In the second composition, the amount of the antioxidant is usually 0.001 to 10 parts by weight when the amount of the crosslinked body of the crosslinkable material is 100 parts by weight.

### [Second Ink]

A second composition comprising a crosslinkable material and a solvent (hereinafter also referred to as "second ink") can be suitably used for wet process described in the item of the first ink. The preferable range of the viscosity of the second ink is the same as the preferable range of the viscosity of the first ink. The examples and the preferable range of the solvent contained in the second ink are the same as the examples and the preferable range of the solvent contained in the first ink.

In the second ink, the amount of the solvent mixed is usually 1,000 to 100,000 parts by weight, and preferably 2,000 to 20,000 parts by weight, when the amount of the crosslinkable material is 100 parts by weight.

### <Layer Constitution of Light Emitting Device>

The light emitting device according to the embodiment of the present invention may comprise layers other than the anode, the cathode, the first organic layer and the second organic layer.

In the light emitting device according to the embodiment of the present invention, the first organic layer is usually a light emitting layer (hereinafter referred to as "first light emitting layer").

In the light emitting device according to the embodiment of the present invention, the second organic layer is usually a hole transporting layer, a second light emitting layer or an electron transporting layer, preferably a hole transporting layer or a second light emitting layer, and more preferably a hole transporting layer.

In the light emitting device according to the embodiment of the present invention, it is preferable that the first organic layer and the second organic layer are adjacent to each other because the light emitting device according to the embodiment of the present invention is more excellent in luminance life. In the light emitting device according to the embodiment of the present invention, the second organic layer is preferably a layer disposed between the anode and the first organic layer, more preferably a hole transporting layer or a second light emitting layer disposed between the anode and the first organic layer, and still more preferably a hole transporting layer disposed between the anode and the first organic layer, because the light emitting device according to the embodiment of the present invention is more excellent in luminance life.

In the light emitting device according to the embodiment of the present invention, when the second organic layer is a hole transporting layer disposed between the anode and the first organic layer, it is preferable that the light emitting device further comprises a hole injection layer between the anode and the second organic layer, because the light emitting device according to the embodiment of the present invention is more excellent in luminance life. When the second organic layer is a hole transporting layer disposed between the anode and the first organic layer, the light emitting device further comprises at least one layer of an electron injection layer and an electron transporting layer between the cathode and the first organic layer, because the light emitting device according to the embodiment of the present invention is more excellent in luminance life.

In the light emitting device according to the embodiment of the present invention, when the second organic layer is a second light emitting layer disposed between the anode and the first organic layer, it is preferable that the light emitting device further comprises at least one layer of a hole injection layer and a hole transporting layer between the anode and the second organic layer, because the light emitting device according to the embodiment of the present invention is more excellent in luminance life. When the second organic layer is a second light emitting layer disposed between the anode and the first organic layer, it is preferable that the light emitting device further comprises at least one layer of an electron injection layer and an electron transporting layer between the cathode and the first organic layer, because the light emitting device according to the embodiment of the present invention is more excellent in luminance life.

In the light emitting device according to the embodiment of the present invention, when the second organic layer is a second light emitting layer disposed between the cathode and the first organic layer, it is preferable that the light emitting device further comprises at least one layer of a hole injection layer and a hole transporting layer between the anode and the first organic layer, because the light emitting device according to the embodiment of the present invention is more excellent in luminance life. When the second organic layer is a second light emitting layer disposed between the cathode and the first organic layer, it is preferable that the light emitting device further comprises at least one layer of an electron injection layer and an electron transporting layer between the cathode and the second organic layer, because the light emitting device according to the embodiment of the present invention is more excellent in luminance life.

In the light emitting device according to the embodiment of the present invention, when the second organic layer is an electron transporting layer disposed between the cathode and the first organic layer, it is preferable that the light emitting device further comprises at least one layer of a hole injection layer and a hole transporting layer between the anode and the first organic layer, because the light emitting device according to the embodiment of the present invention is more excellent in luminance life. When the second organic layer is an electron transporting layer disposed between the cathode and the first organic layer, it is preferable that the light emitting device further comprises an electron injection layer between the cathode and the second organic layer, because the light emitting device according to the embodiment of the present invention is more excellent in luminance life.

Specific layer constitution of the light emitting device according to the embodiment of the present invention includes, for example, layer constitutions represented by the following (D1) to (D15). The light emitting device according to the embodiment of the present invention usually comprises a substrate, and an anode may be laminated on the substrate first, or a cathode may be laminated on the substrate first.
(D1) anode/second light emitting layer (second organic layer)/first light emitting layer (first organic layer)/cathode
(D2) anode/hole transporting layer (second organic layer)/first light emitting layer (first organic layer)/cathode
(D3) anode/hole injection layer/second light emitting layer (second organic layer)/first light emitting layer (first organic layer)/cathode
(D4) anode/hole injection layer/second light emitting layer (second organic layer)/first light emitting layer (first organic layer)/electron transporting layer/cathode
(D5) anode/hole injection layer/second light emitting layer (second organic layer)/first light emitting layer (first organic layer)/electron injection layer/cathode
(D6) anode/hole injection layer/second light emitting layer (second organic layer)/first light emitting layer (first organic layer)/electron transporting layer/electron injection layer/cathode
(D7) anode/hole injection layer/hole transporting layer (second organic layer)/first light emitting layer (first organic layer)/cathode
(D8) anode/hole injection layer/hole transporting layer (second organic layer)/first light emitting layer (first organic layer)/electron transporting layer/cathode
(D9) anode/hole injection layer/hole transporting layer (second organic layer)/first light emitting layer (first organic layer)/electron injection layer/cathode
(D10) anode/hole injection layer/hole transporting layer (second organic layer)/first light emitting layer (first organic layer)/electron transporting layer/electron injection layer/cathode
(D11) anode/hole injection layer/hole transporting layer/second light emitting layer (second organic layer)/first light emitting layer (first organic layer)/electron transporting layer/electron injection layer/cathode
(D12) anode/hole injection layer/hole transporting layer (second organic layer)/first light emitting layer (first organic layer)/second light emitting layer/electron transporting layer/electron injection layer/cathode
(D13) anode/hole injection layer/hole transporting layer/first light emitting layer (first organic layer)/second light emitting layer (second organic layer)/electron transporting layer/electron injection layer/cathode
(D14) anode/hole injection layer/hole transporting layer/first light emitting layer (first organic layer)/electron transporting layer (second organic layer)/electron injection layer/cathode
(D15) anode/hole injection layer/hole transporting layer (second organic layer)/second light emitting layer/first light emitting layer (first organic layer)/electron transporting layer/electron injection layer/cathode

In the above-mentioned (D1) to (D15), "/" means that layers therebefore and thereafter are laminated while being adjacent to each other. Specifically, "second light emitting layer (second organic layer)/first light emitting layer (first organic layer)" means that a second light emitting layer (second organic layer) and a first light emitting layer (first organic layer) are laminated while being adjacent to each other.

Since the light emitting device according to the embodiment of the present invention is more excellent in luminance life, layer constitution represented by (D3) to (D12) is preferable and layer constitution represented by (D7) to (D10) is more preferable.

In the light emitting device according to the embodiment of the present invention, if necessary, two or more layers of an anode, a hole injection layer, a hole transporting layer, a second light emitting layer, an electron transporting layer, an electron injection layer and a cathode may be provided, respectively.

When a plurality of anodes, hole injection layers, hole transporting layers, second light emitting layers, electron transporting layers, electron injection layers and cathodes are present, they may be the same or different at each occurrence.

The thickness of the anode, the hole injection layer, the hole transporting layer, the first light emitting layer, the second light emitting layer, the electron transporting layer, the electron injection layer and the cathode is usually 1 nm to 1 µm, preferably 2 nm to 500 nm, and still more preferably 5 nm to 150 nm.

In the light emitting device according to the embodiment of the present invention, the order and the number of layers to be laminated and the thickness of each layer may be adjusted in consideration of the light emission efficiency and the device life of the light emitting device.

### [Second Light Emitting Layer]

The second light emitting layer is usually a layer comprising a second organic layer or a light emitting material. When the second light emitting layer is a layer comprising the light emitting material, the light emitting material contained in the second light emitting layer includes, for example, a light emitting material which the above-mentioned first composition optionally comprises. The second light emitting layer may comprise a light emitting material alone or two or more light emitting materials.

When the light emitting device according to the embodiment of the present invention comprises the second light emitting layer and the below-mentioned hole transporting layer and the below-mentioned electron transporting layer are not second organic layers, the second light emitting layer is preferably a second organic layer.

### [Hole Transporting Layer]

The hole transporting layer is usually a layer comprising a second organic layer or a hole transporting material. When the hole transporting layer is a layer comprising a hole transporting material, the hole transporting material includes, for example, a hole transporting material which the above-mentioned first composition optionally comprises. The hole transporting layer may comprise a hole transporting material alone or two or more hole transporting materials.

When the light emitting device according to the embodiment of the present invention comprises the hole transporting layer and the above-mentioned second light emitting layer and the above-mentioned electron transporting layer are not second organic layers, the hole transporting layer is preferably a second organic layer.

### [Electron Transporting Layer]

The electron transporting layer is usually a second organic layer or a layer comprising an electron transporting material, and preferably a layer comprising an electron transporting material. When the electron transporting layer is a layer comprising an electron transporting material, the electron transporting material contained in the electron transporting layer includes, for example, an electron transporting material which the above-mentioned first composition optionally comprises.

When the light emitting device according to the embodiment of the present invention comprises the electron transporting layer and the electron transporting layer is not a second organic layer, the electron transporting material contained in the electron transporting layer is preferably a polymer compound comprising at least one constitutional unit selected from the group consisting of a constitutional unit represented by formula (ET-1) and a constitutional unit represented by formula (ET-2) (hereinafter also referred to as "polymer compound of electron transporting layer": 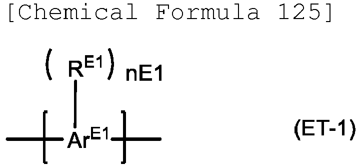 wherein
nE1 represents an integer of 1 or more,
Ar^{E1} represents an aromatic hydrocarbon group or a heterocyclic group, and these groups each optionally have a substituent other than R^{E1}, and
R^{E1} represents a group represented by formula (ES-1), and when a plurality of R^{E1} are present, they may be the same or different:

-R^{E3}-{(Q^{E1})_{nE3}-Y^{E1}(ME¹)_{aE1}(Z^{E1})_{bE1}}_{mE1} (ES-1)

wherein
nE3 represents an integer of 0 or more, aE1 represents an integer of 1 or more, bE1 represents an integer of 0 or more and mE1 represents an integer of 1 or more, when a plurality of nE3, aE1 and bE1 are present, they may be the same or different at each occurrence, and when R^{E3} is a single bond, mE1 is 1, and aE1 and bE1 are selected so that the charge of a group represented by formula (ES-1) becomes 0,
R^{E3} represents a single bond, a hydrocarbon group, a heterocyclic group or -O-R^{E3}, (R^{E3'} represents a hydrocarbon group or a heterocyclic group), and these groups each optionally have a substituent,
Q^{E1} represents an alkylene group, a cycloalkylene group, an arylene group, an oxygen atom or a sulfur atom, these groups each optionally have a substituent, and when a plurality of Q^{E1} are present, they may be the same or different,
Y^{E1} represents CO₂⁻, SO₃⁻, SO₂⁻ or PO₃²⁻, and when a plurality of Y^{E1} are present, they may be the same or different,
M^{E1} represents an alkali metal cation, an alkali earth metal cation or an ammonium cation, and this ammonium cation optionally have a substituent, and when a plurality of M^{E1} are present, they may be the same or different, and
Z^{E1} represents F-, Cl⁻, Br-, I-, OH-, B(R^{E4})₄-, R^{E4}SO₃⁻ , R^{E4}COO⁻, NO₃⁻, SO₄²⁻ , HSO₄⁻, PO₄³⁻, HPO₄²⁻, H₂PO₄⁻, BF₄⁻ or PF₆⁻, R^{E4} represents an alkyl group, a cycloalkyl group or an aryl group, and these groups each optionally have a substituent, and when a plurality of Z^{E1} are present, they may be the same or different.

nE1 is usually an integer of 1 to 4, and preferably 1 or 2.

The aromatic hydrocarbon group or the heterocyclic group represented by Ar^{E1} is preferably a group obtained by removing from a 1,4-phenylene group, a 1,3-phenylene group, a 1,2-phenylene group, a 2,6-naphthalenediyl group, a 1,4-naphthalenediyl group, a 2,7-fluorenediyl group, a 3,6-fluorenediyl group, a 2,7-phenanthrenediyl group or a 2,7-carbazolediyl group nE1 hydrogen atoms bonding directly to atoms constituting its ring, and optionally has a substituent other than R^{E1}.

The substituent other than R^{E1} which Ar^{E1} optionally has includes a halogen atom, a cyano group, an alkyl group, a cycloalkyl group, an aryl group, a monovalent heterocyclic group, an alkoxy group, a cycloalkoxy group, an aryloxy group, an amino group, a substituted amino group, an alkenyl group, a cycloalkenyl group, an alkynyl group, a cycloalkynyl group, a carboxyl group and a group represented by formula (ES-3):

-O-(C_{n'}H_{2n'}O)ₙₓ-C_{m'}H_{2m'+1} (ES-3)

wherein n', m' and nx each independently represent an integer of 1 or more.

nE3 is usually an integer of 0 to 10, preferably an integer of 0 to 8, and more preferably an integer of 0 to 2.

aE1 is usually an integer of 1 to 10, preferably an integer of 1 to 5, more preferably 1 or 2.

bE1 is usually an integer of 0 to 10, preferably an integer of 0 to 4, and more preferably 0 or 1.

mE1 is usually an integer of 1 to 5, preferably 1 or 2, and more preferably 1.

When R^{E3} is -O-R^{E3'}, the group represented by formula (ES-1) is a group represented by the following formula.

-O-R^{E3'}-{(Q^{E1})_{nE3}-Y^{E1}(M^{E1})_{aE1}(Z^{E1})_{bE1}}_{mE1}

R^{E3} is preferably a hydrocarbon group or a heterocyclic group, more preferably an aromatic hydrocarbon group or an aromatic heterocyclic group, and still more preferably an aromatic hydrocarbon group.

The substituent which R^{E3} optionally has includes an alkyl group, a cycloalkyl group, an aryl group, a monovalent heterocyclic group and a group represented by formula (ES-3), and is preferably a group represented by formula (ES-3).

Q^{E1} is preferably an alkylene group, an arylene group or an oxygen atom, and more preferably an alkylene group or an oxygen atom.

Y^{E1} is preferably CO₂⁻, SO₂⁻ or PO₃²⁻, and more preferably CO₂⁻.

The alkali metal cation represented by M^{E1} includes, for example, Li⁺, Na⁺, K⁺, Rb⁺ and Cs⁺, and is preferably K⁺, Rb⁺ or Cs⁺, and more preferably Cs⁺.

The alkaline earth metal cation represented by M^{E1} includes, for example, Be²⁺, Mg²⁺, Ca²⁺, Sr²⁺ and Ba²⁺, and is preferably Mg²⁺, Ca²⁺, Sr²⁺ or Ba²⁺, and more preferably Ba²⁺.

M^{E1} is preferably an alkali metal cation or an alkaline earth metal cation, and more preferably an alkali metal cation.

Z^{E1} is preferably F⁻, Cl⁻, Br-, I-, OH-, B(R^{E4})₄⁻, R^{E4}SO₃⁻, R^{E4}COO⁻ or NO₃⁻, and preferably, F⁻, Cl⁻, Br⁻, I⁻, OH⁻, R^{E4}SO₃⁻ or R^{E4}COO⁻. R^{E4} is preferably an alkyl group.

The group represented by the formula (ES-1) includes, for example, groups represented by the following formulas: 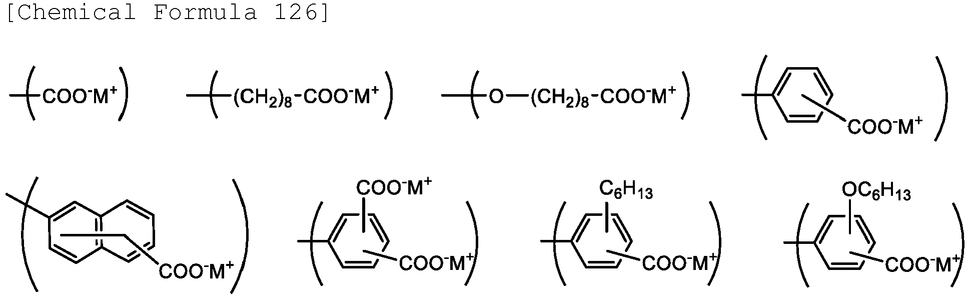 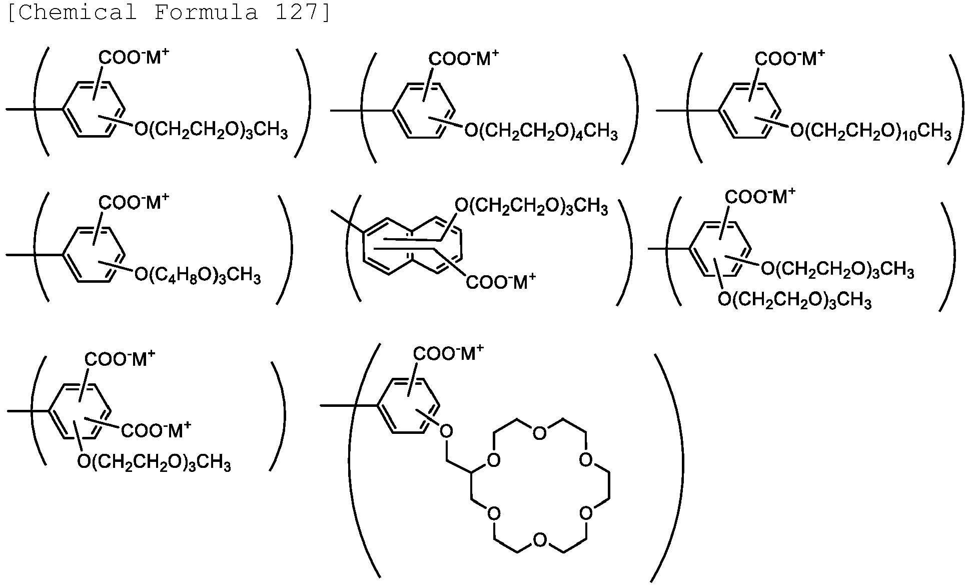 wherein M⁺ represents Li⁺, Na⁺, K⁺, Cs⁺ or N(CH₃)₄⁺, and when a plurality of M⁺ are present, they may be the same or different: 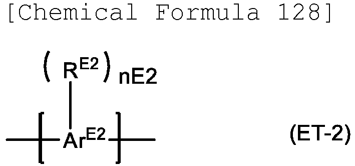 wherein
nE2 represents an integer of 1 or more,
Ar^{E2} represents an aromatic hydrocarbon group or a heterocyclic group, and these groups each optionally have a substituent other than R^{E2}, and
R^{E2} represents a group represented by the formula (ES-2), and when a plurality of R^{E2} are present, they may be the same or different.

-R^{E5}-{(Q^{E2})_{nE4}-Y^{E2}(M^{E2})_{aE2}(Z^{E2})_{bE2}}_{mE2} (ES-2)

wherein
nE4 represents an integer of 0 or more, aE2 represents an integer of 1 or more, bE2 represents an integer of 0 or more, and mE2 represents an integer of 1 or more, and when a plurality of nE4, aE2 and bE2 are present, they may be the same or different at each occurrence, and when R^{E5} is a single bond, mE2 is 1, and aE2 and bE2 are selected so that the charge of a group represented by formula (ES-2) becomes 0,
R^{E5} represents a single bond, a hydrocarbon group, a heterocyclic group or -O-R^{E5'} (R^{E5'} represents a hydrocarbon group or a heterocyclic group), and these groups each optionally have a substituent,
Q^{E2} represents an alkylene group, a cycloalkylene group, an arylene group, an oxygen atom or a sulfur atom, these groups each optionally have a substituent, and when a plurality of Q^{E2} are present, they may be the same or different,
Y^{E2} represents -C⁺R^{E6}₂, -N⁺R^{E6}₃, -P⁺R^{E6}₃, -S⁺R^{E6}₂ or - I⁺R^{E6}₂, R^{E6} represents a hydrogen atom, an alkyl group, a cycloalkyl group or an aryl group, and these groups each optionally have a substituent, and the plurality of R^{E6} may be the same or different, and when a plurality of Y^{E2} are present, they may be the same or different,
M^{E2} represents F⁻, Cl⁻, Br⁻, I⁻, OH⁻, B(R^{E7})₄⁻, R^{E7}SO₃⁻, R^{E7}COO⁻, BF₄⁻, SbCl₆⁻ or SbF₆⁻, R^{E7} represents an alkyl group, a cycloalkyl group or an aryl group, and these groups each optionally have a substituent, and when a plurality of M^{E2} are present, they may be the same or different, and
Z^{E2} represents an alkali metal cation or an alkali earth metal cation, and when a plurality of Z^{E2} are present, they may be the same or different.

nE2 is usually an integer of 1 to 4, and preferably 1 or 2.

The aromatic hydrocarbon group or heterocyclic group represented by Ar^{E2} is preferably a group obtained by removing from a 1,4-phenylene group, a 1,3-phenylene group, a 1,2-phenylene group, a 2,6-naphthalenediyl group, a 1,4-naphthalenediyl group, a 2,7-fluorenediyl group, a 3,6-fluorenediyl group, 2a ,7-phenanthrenediyl group or a 2,7-carbazolediyl group nE2 hydrogen atoms bonding directly to atoms constituting its ring, and optionally has a substituent other than R^{E2}.

The substituent other than R^{E2} which Ar^{E2} optionally has is the same as the substituent other than R^{E1} which Ar^{E1} optionally has.

nE4 is usually an integer of 0 to 10, preferably an integer of 0 to 8, and more preferably an integer of 0 to 2.

aE2 is usually an integer of 1 to 10, preferably an integer of 1 to 5, and more preferably 1 or 2.

bE2 is usually an integer of 0 to 10, preferably an integer of 0 to 4, and more preferably 0 or 1.

mE2 is usually an integer of 1 to 5, preferably 1 or 2, and more preferably 1.

When R^{E5} is -O-R^{E5'}, the group represented by formula (ES-2) is a group represented by the following formula.

-O-R^{E5'}-{(Q^{E1})_{nE3}-Y^{E1}(M^{E1})_{aE1}(Z^{E1})_{bE1}}_{mE1}

R^{E5} is preferably a hydrocarbon group or a heterocyclic group, more preferably an aromatic hydrocarbon group or an aromatic heterocyclic group, and still more preferably an aromatic hydrocarbon group.

The substituent which R^{E5} optionally has includes an alkyl group, a cycloalkyl group, an aryl group, a monovalent heterocyclic group and a group represented by formula (ES-3), and is preferably a group represented by formula (ES-3).

Q^{E 2} is preferably an alkylene group, an arylene group or an oxygen atom, and more preferably an alkylene group or an oxygen atom.

Y^{E2} is preferably -C⁺R^{E6}₂, -N⁺R^{E6}₃, -P⁺R^{E6}₃ or -S⁺R^{E6}₂, and more preferably -N⁺R^{E6}₃. R^{E6} is preferably a hydrogen atom, an alkyl group or an aryl group, and more preferably a hydrogen atom or an alkyl group.

M^{E2} is preferably F⁻, Cl⁻, Br-, I-, B(R^{E7})₄⁻, R^{E7}SO₃⁻, R^{E7}COO⁻, BF₄⁻ or SbF⁶⁻, and more preferably Br-, I-, B(R^{E7})₄⁻, R^{E7}COO⁻ or SbF⁶⁻. R^{E7} is preferably an alkyl group.

The alkali metal cation represented by Z^{E2} includes, for example, Li⁺, Na⁺, K⁺, Rb⁺ and Cs⁺, and is preferably Li⁺, Na⁺ or K⁺ .

The alkaline earth metal cation represented by Z^{E2} includes, for example, Be²⁺, Mg²⁺, Ca²⁺, Sr²⁺ and Ba²⁺, and is preferably Mg²⁺ or Ca²⁺.

Z^{E2} is preferably an alkali metal cation.

The group represented by the formula (ES-2) includes, for example, groups represented by the following formulas: 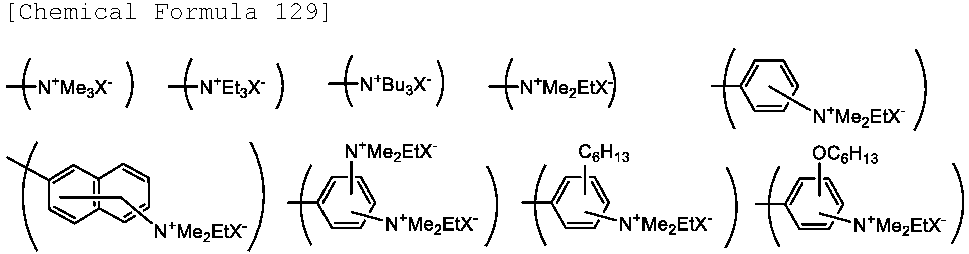 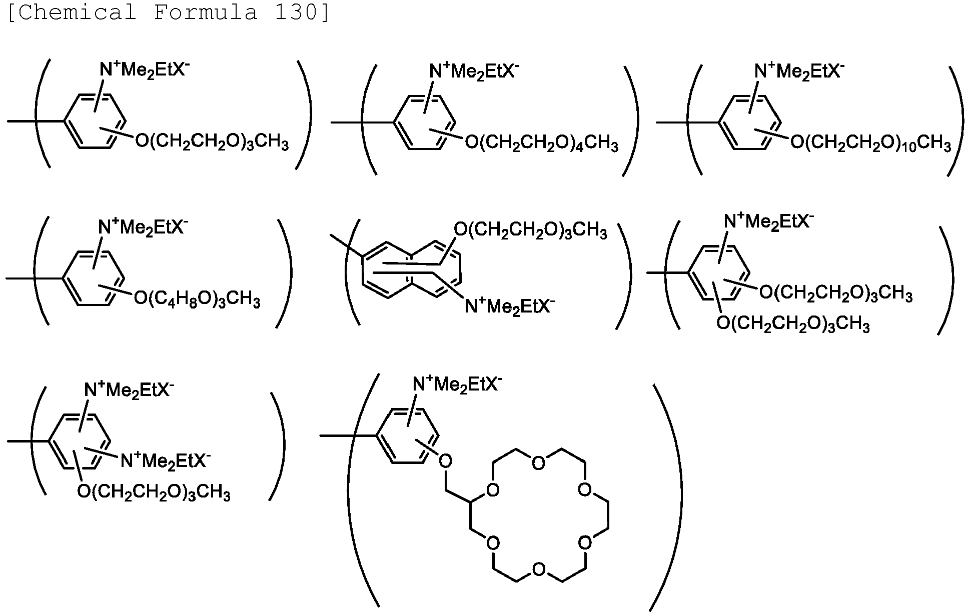 wherein X⁻ represents F⁻, Cl⁻ , Br⁻, I⁻, B(C₆H₅)₄⁻, CH₃COO⁻ or CF₃SO₃⁻, and when a plurality of X⁻ are present, they may be the same or different.

The constitutional unit represented by formula (ET-1) and formula (ET-2) includes, for example, constitutional units represented by formula (ET-31) to formula (ET-38) mentioned below.

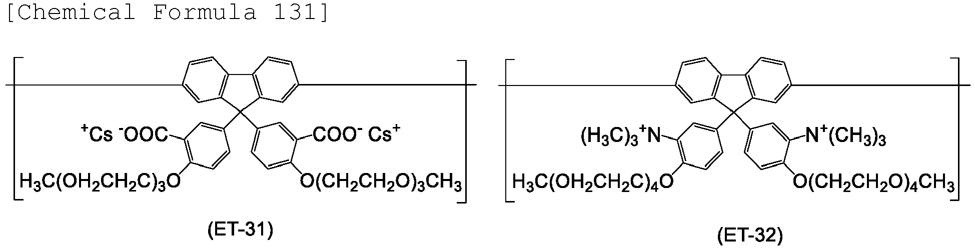

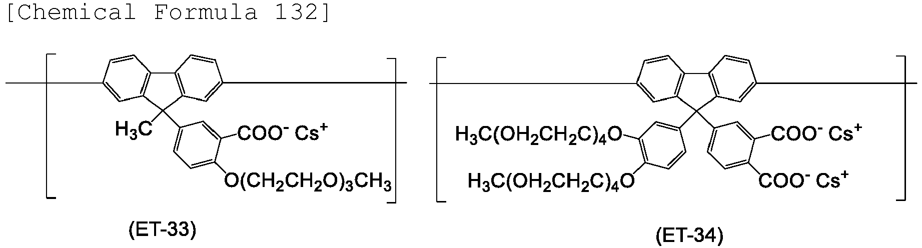

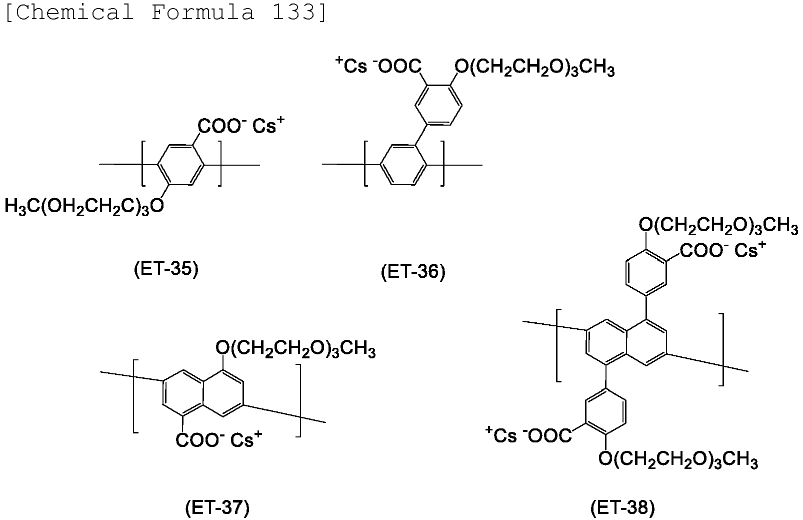

The polymer compound of the electron transporting layer can be synthesized, for example, according to methods disclosed in JP 2009-239279 A, JP 2012-033845 A, JP 2012-216821 A, JP 2012-216822 A and JP 2012-216815 A.

When a material used for formation of a hole injection layer mentioned below, a material used for formation of a hole transporting layer, a material used for formation of a first light emitting layer, a material used for formation of a second light emitting layer, a material used for formation of an electron transporting layer and a material used for formation of an electron injection layer mentioned below are dissolved in a solvent used for formation of a layer adjacent to a hole injection layer, a hole transporting layer, a first light emitting layer, a second light emitting layer, an electron transporting layer and an electron injection layer, respectively, in fabrication of a light emitting device, it is preferable to avoid dissolution of the material in the solvent. The method of avoiding dissolution of the material is preferably i) a method of using a material having a crosslinkable group, or ii) a method of making a difference in solubility between adjacent layers. In the method i), a layer is formed using a material having a crosslinkable group and then the crosslinkable group is crosslinked, thus making it possible to insolubilize the layer.

When an electron transporting layer is laminated on a first light emitting layer or a second light emitting layer by utilizing a difference in solubility, the electron transporting layer can be laminated by using a solution having low solubility in the first light emitting layer or the second light emitting layer.

The solvent used when an electron transporting layer is laminated on a first light emitting layer or a second light emitting layer by utilizing a difference in solubility is preferably water, alcohols, ethers, esters, nitrile compounds, nitro compounds, fluorinated alcohols, thiols, sulfides, sulfoxides, thioketones, amides, carboxylic acids and the like. Specific examples of the solvent include methanol, ethanol, 2-propanol, 1-butanol, tert-butyl alcohol, acetonitrile, 1,2-ethanediol, N,N-dimethylformamide, dimethyl sulfoxide, acetic acid, nitromethane, propylene carbonate, pyridine, carbon disulfide, and a mixed solvent thereof. When the mixed solvent is used, it may be a mixed solvent of at least one solvent of water, alcohols, ethers, esters, nitrile compounds, nitro compounds, fluorinated alcohols, thiols, sulfides, sulfoxides, thioketones, amides, carboxylic acids and the like, and at least one solvent of chlorine-based solvents, aromatic hydrocarbon-based solvents, aliphatic hydrocarbon-based solvents and ketone-based solvents.

### [Hole Injection Layer and Electron Injection Layer]

The hole injection layer is a layer comprising a hole injection material. The hole injection material contained in the hole injection layer includes, for example, a hole injection material which the above-mentioned first composition optionally comprises. The hole injection layer may comprise a hole injection material alone or two or more hole injection materials.

The electron injection layer is a layer comprising an electron injection material. The electron injection material contained in the electron injection layer includes, for example, an electron injection material which the above-mentioned first composition optionally comprises. The electron injection layer may comprise an electron injection material alone or two or more electron injection materials.

### [Substrate/Electrode]

The substrate in the light emitting device may be a substrate which can form an electrode and does not chemically change in forming an organic layer, and for example, substrates made of glass, plastic, silicon and the like. When an opaque substrate is used, an electrode farthest from the substrate is preferably transparent or semitransparent.

The material of an anode includes, for example, conductive metal oxides and semitransparent metals, preferably, indium oxide, zinc oxide, tin oxide; conductive compounds such as indium tin oxide (ITO) and indium zinc oxide; composites of Ag, palladium and copper (APC); NESA, gold, platinum, silver and copper.

The material of a cathode includes, for example, metals such as lithium, sodium, potassium, rubidium, cesium, beryllium, magnesium, calcium, strontium, barium, aluminum, zinc and indium; alloys of two or more metals; alloys of at least one metal and at least one of silver, copper, manganese, titanium, cobalt, nickel, tungsten and tin; and graphite and graphite intercalation compounds. The alloy includes, for example, a magnesium-silver alloy, a magnesium-indium alloy, a magnesium-aluminum alloy, an indium-silver alloy, a lithium-aluminum alloy, a lithium-magnesium alloy, a lithium-indium alloy and a calcium-aluminum alloy.

In the light emitting device according to embodiments of the present invention, at least one of an anode and a cathode is usually transparent or semitransparent, and it is preferable that an anode is transparent or semitransparent.

The method of forming an anode and a cathode includes, for example, a vacuum vapor deposition method, a sputtering method, an ion plating method, a plating method and a laminate method.

### [Method for Producing Light Emitting Device]

In the light emitting device according to the embodiment of the present invention, the method of forming the respective layers such as a first light emitting layer, a second light emitting layer, a hole transporting layer, an electron transporting layer, a hole injection layer and an electron injection layer includes, for example, a vacuum vapor-deposition method from a powder and a method by film formation from a solution or a molten state, and includes, for example, a method by film formation from a solution or a molten state when a polymer compound is used.

The first light emitting layer, the second light emitting layer, the hole transporting layer, the electron transporting layer, the hole injection layer and the electron injection layer can be formed by wet process such as a spin coat method and an ink-jet printing method, using a first ink, a second ink, and an ink each containing the above-mentioned light emitting material, hole transporting material, electron transporting material, hole injection material and electron injection material.

### [Applications of Light Emitting Device]

To obtain planar light emission using a light emitting device, a planar anode and a planar cathode are disposed so as to overlap with each other. To obtain patterned light emission, it is possible to employ a method in which a mask with a patterned window is placed on a surface of a planer light emitting device, a method in which an extremely thick layer intended to be a non-light emitting is formed, thereby having the layer substantially no-light emitting, or a method in which an anode, a cathode or both electrodes are formed in a patterned shape. By forming a pattern with any of these methods and disposing some electrodes so as to switch ON/OFF independently, a segment type display capable of displaying numbers and letters and the like is provided. To produce a dot matrix display, both an anode and a cathode are formed in a stripe shape and disposed so as to cross orthogonally with each other. Partial color display and multi-color display are made possible by a method in which a plurality of polymer compounds each showing a different luminescent color are printed separately or a method in which a color filter or a fluorescence conversion filter is used. The dot matrix display can be passively driven and also can be actively driven combined with TFT. These displays can be used in computers, television sets, portable terminals and the like. The planar light emitting device can be suitably used as a planer light source for backlight of a liquid crystal display or as a planar light source for illumination. If a flexible substrate is used, it can also be used as a curved light source and a curved display.

### Examples

Embodiments of the present invention will be described in detail by way of Examples, but the present invention is not limited to these Examples.

In Examples, the polystyrene-equivalent number average molecular weight (Mn) and the polystyrene-equivalent weight average molecular weight (Mw) of a polymer compound were determined by the following size exclusion chromatography (SEC) using tetrahydrofuran as a mobile phase. Measurement conditions of each SEC are as mentioned below.

A polymer compound to be measured was dissolved in tetrahydrofuran at the concentration of about 0.05% by weight and 10 µL of the solution was injected into SEC. A mobile phase was allowed to flow at a flow rate of 2.0 mL/minute. As the column, PLgel MIXED-B (manufactured by Polymer Laboratories Ltd.) was used. As the detector, an UV-VIS detector (tradename: SPD-10Avp, manufactured by Shimadzu Corporation) was used.

### <Synthesis Example G1> Synthesis of Phosphorescent Compounds G1 to G5

A phosphorescent compound G1 was synthesized based on the method disclosed in WO 2004/026886.

A phosphorescent compound G2 was synthesized based on the method disclosed in WO 2011/032626.

A phosphorescent compound G3 was synthesized according to the method disclosed in WO 2009/131255.

A phosphorescent compound G4 was synthesized based on the method disclosed in JP 2014-224101 A.

A phosphorescent compound G5 was synthesized according to the method disclosed in JP 2014-224101 A.

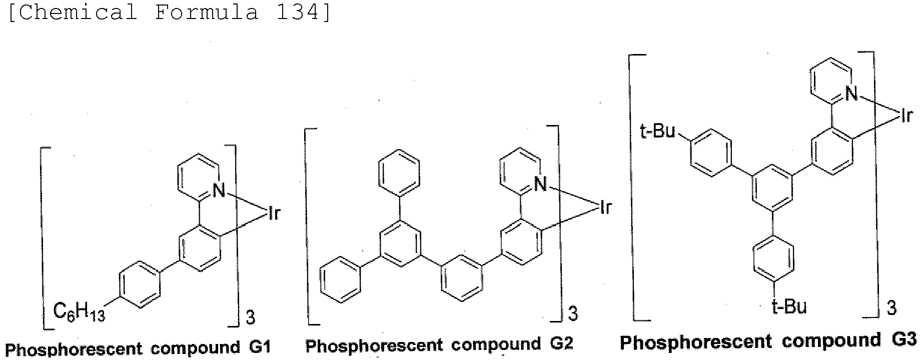

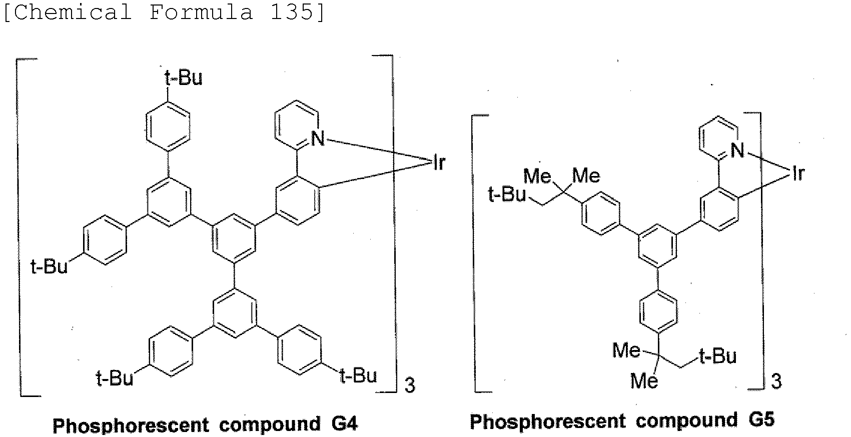

### <Synthesis Example R1> Synthesis of Phosphorescent Compounds R1 to R3

A phosphorescent compound R1 was synthesized based on the method disclosed in JP 2006-188673 A.

A phosphorescent compound R2 was synthesized according to the method disclosed in JP 2008-179617 A.

A phosphorescent compound R3 was synthesized according to the method disclosed in JP 2011-105701 A.

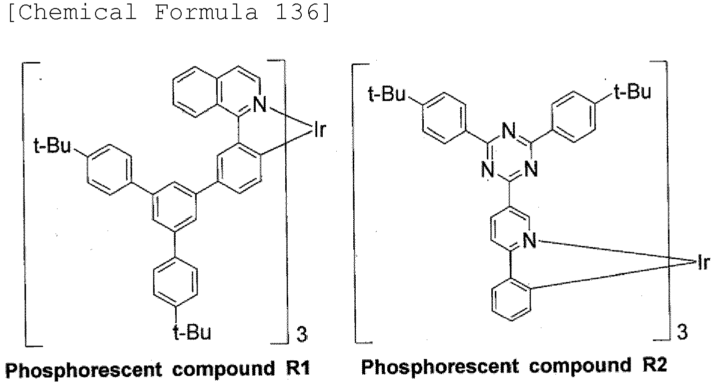

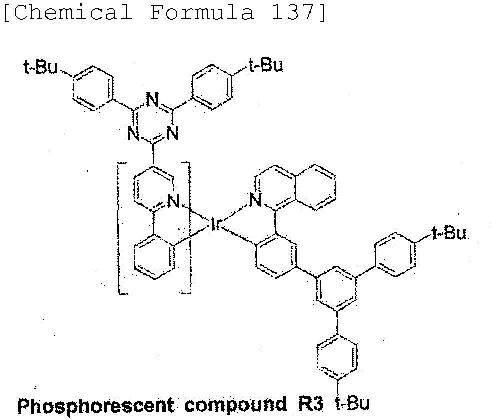

### <Synthesis Example H1> Synthesis and Obtainment of Compounds H1 to H6

A compound H1 was synthesized based on the method disclosed in WO 2010/136109.

Compounds H2 and H3 were purchased from Luminescense Technology Corp.

A compound H4 was synthesized according to the method disclosed in JP 2010-189630 A.

A compound H5 was synthesized based on the method disclosed in WO 2011/070963.

A compound H6 was synthesized according to the method disclosed in JP 2015-110751 A.

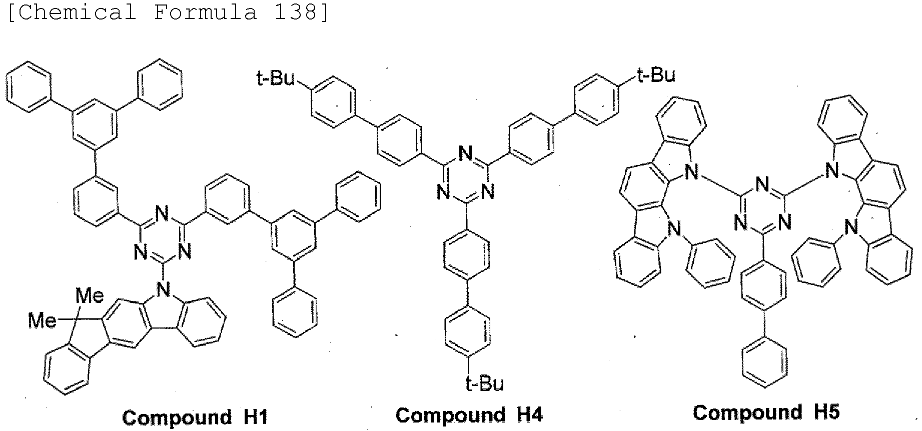

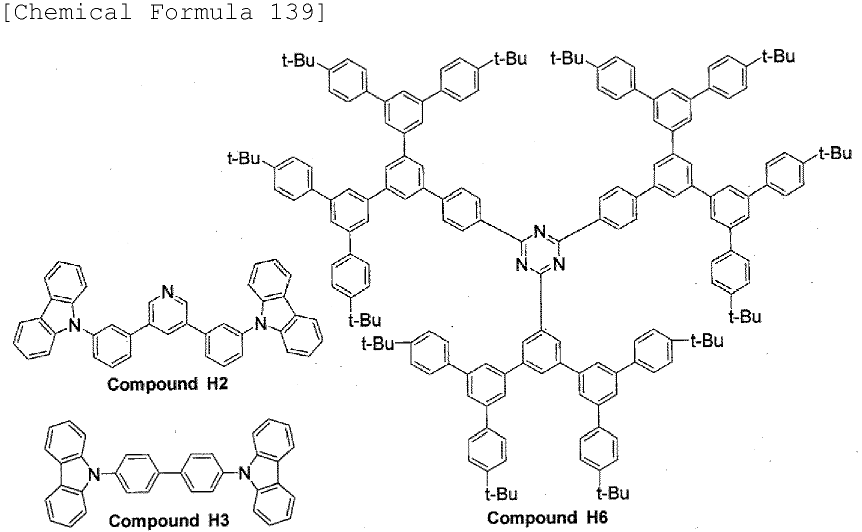

### <Synthesis Example M1> Synthesis of Compounds M1 to M8

A compound M1 was synthesized according to the method disclosed in JP 2011-174062 A.

A compound M2, a compound M7 and a compound M8 were synthesized according to the method disclosed in WO 2002/045184.

A compound M3 was synthesized according to the method disclosed in WO 2005/049546.

A compound M4 was synthesized according to the method disclosed in JP 2008-106241 A.

A compound M5 was synthesized according to the method disclosed in JP 2010-189630 A.

A compound M6 was synthesized according to the method disclosed in WO 2011/049241.

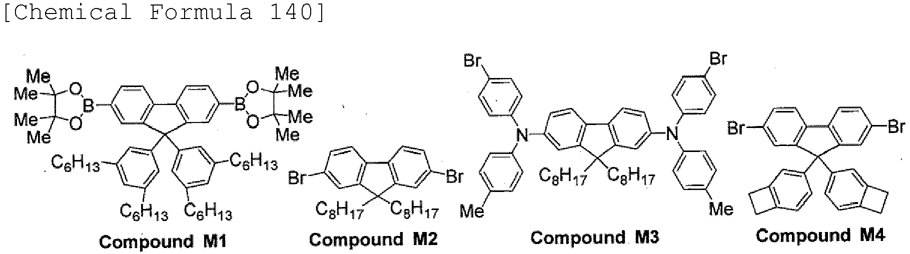

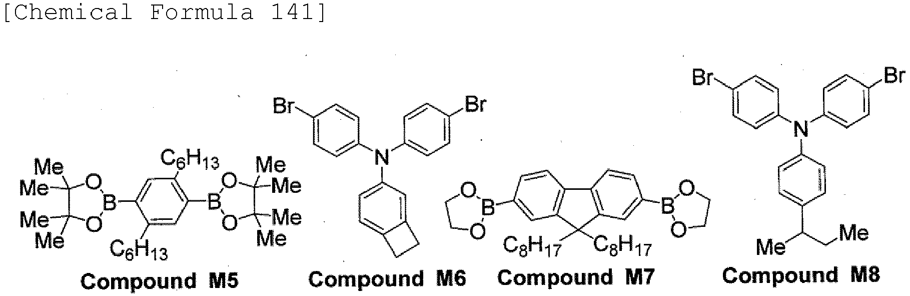

### <Synthesis Example HTL1> Synthesis of Polymer Compound HTL-1

A polymer compound HTL-1 was synthesized by the Suzuki coupling reaction according to the method disclosed in WO 2013/146806 using the compound M5, the compound M3 and the compound M6. The polymer compound HTL-1 had Mn of 1.9 × 10⁴ and Mw of 9.9 × 10⁴.

The polymer compound HTL-1 is a copolymer constituted of a constitutional unit derived from the compound M5, a constitutional unit derived from the compound M3 and a constitutional unit derived from the compound M6 at a molar ratio of 50:42.5:7.5, according to the theoretical values calculated from the amounts of the charging raw materials.

### <Synthesis Example HTL2> Synthesis of Polymer Compound HTL-2

(Step 1) After replacing the atmosphere in a reaction vessel with an inert gas atmosphere, the compound M1 (2.69 g), the compound M2 (0.425 g), the compound M3 (1.64 g), the compound M4 (0.238 g), dichlorobis(triphenylphosphine)palladium (2.1 mg) and toluene (62 ml) were added, followed by heating to 105°C.

(Step 2) An aqueous 20% by weight tetraethylammonium hydroxide aqueous solution (10 ml) was added dropwise to the resulting reaction solution, and the mixture was refluxed for 4.5 hours.

(Step 3) After the reaction, phenylboronic acid (36.8 mg) and dichlorobis(triphenylphosphine)palladium (2.1 mg) were added thereto, and the mixture was refluxed for 16.5 hours.

(Step 4) Thereafter, an aqueous sodium diethyldithiacarbamate solution was added thereto, followed by stirring at 80°C for 2 hours. After cooling, the resulting reaction solution was washed twice with water, twice with an aqueous 3% by weight acetic acid solution, and twice with water, and then the resulting solution was added dropwise in methanol to form a precipitate. The resulting precipitate was dissolved in toluene and then purified by passing through an alumina column and a silica gel column in this order. The resulting solution was added dropwise in methanol and, after stirring, the resulting precipitate was collected by filtration and then dried to obtain 3.12 g of a polymer compound HTL-2. The polymer compound HTL-2 had Mn of 7.8 × 10⁴ and Mw of 2.6 × 10⁵ .

The polymer compound HTL-2 is a copolymer constituted of a constitutional unit derived from the compound M1, a constitutional unit derived from the compound M2, a constitutional unit derived from the compound M3 and a constitutional unit derived from the compound M4 at a molar ratio of 50:12.5:30:7.5, according to the theoretical values calculated from the amounts of the charging raw materials.

### <Synthesis Example HTL3> Synthesis of Polymer Compound HTL-3

In the same manner as in the synthesis of the polymer compound HTL-2, except that (Step 1) in the synthesis of the polymer compound HTL-2 was changed to the following (Step 1-1), (Step 2) was changed to the following (Step 2-1), and (Step 3) was changed to the following (Step 3-1), 3.00 g of a polymer compound HTL-3 was obtained.

(Step 1-1) After replacing the atmosphere in a reaction vessel with an inert gas atmosphere, the compound M5 (1.74 g), the compound M3 (3.19 g), dichlorobis(triphenylphosphine)palladium (2.5 mg) and toluene (40 mL) were added, followed by heating to 80°C.

(Step 2-1) An aqueous 20% by weight tetraethylammonium hydroxide aqueous solution (12 mL) was added dropwise to the resulting reaction solution, and the mixture was refluxed for 8 hours.

(Step 3-1) After the reaction, phenylboronic acid (0.427 g) and dichlorobis(triphenylphosphine)palladium (2.5 mg) were added thereto, and the mixture was refluxed for 17 hours.

The polymer compound HTL-3 had Mn of 4.5 × 10⁴ and Mw of 1.5 × 10⁵.

The polymer compound HTL-3 is a copolymer constituted of a constitutional unit derived from the compound M5 and a constitutional unit derived from the compound M3 at a molar ratio of 50:50, according to the theoretical values calculated from the amounts of the charging raw materials.

### <Synthesis Example HTL4> Synthesis of Polymer Compound HTL-4

The polymer compound HTL-4 was synthesized according to the method disclosed in WO 2011/049241 using the compound M7, the compound M8 and the compound M6. The polymer compound HTL-4 had Mn of 8.9 × 10⁴ and Mw of 4.2 × 10⁵.

The polymer compound HTL-4 is a copolymer constituted of a constitutional unit derived from the compound M7, a constitutional unit derived from the compound M8 and a constitutional unit derived from the compound M6 at a molar ratio of 50:42.5:7.5, according to the theoretical values calculated from the amounts of the charging raw materials.

### <Synthesis Example HTL5> Synthesis of Polymer Compound HTL-5

The polymer compound HTL-5 was synthesized according to the method disclosed in JP 2012-36381 A using the compound M7 and the compound M8. The polymer compound HTL-5 had Mn of 8.1 × 10⁴ and Mw of 3.4 × 10⁵.

The polymer compound HTL-5 is a copolymer constituted of a constitutional unit derived from the compound M7 and a constitutional unit derived from the compound M8 at a molar ratio of 50:50, according to the theoretical values calculated from the amounts of the charging raw materials.

### <Example D1> Fabrication and Evaluation of Light Emitting Device D1

### (Formation of Anode and Hole Injection Layer)

An ITO film with a thickness of 45 nm was attached to a glass substrate by a sputtering method to form an anode. On the anode, a film was formed with a thickness of 35 nm by a spin coating method using a polythiophene-sulfonic acid-based hole injection agent AQ-1200 (manufactured by Plextronics Inc.), and then heated on a hot plate at 170°C in an air atmosphere for 15 minutes to form a hole injection layer.

### (Formation of Second Organic Layer)

The polymer compound HTL-1 was dissolved in xylene at the concentration of 0.6% by weight. Using the resulting xylene solution, a film was formed on the hole injection layer with a thickness of 20 nm by a spin coating method, and then heated on a hot plate at 180°C in a nitrogen gas atmosphere for 60 minutes to form a second organic layer. As a result of heating, the polymer compound HTL-1 was converted into a crosslinked body.

### (Formation of First Organic Layer)

The compound H1 and the phosphorescent compound G2 (compound H1/phosphorescent compound G2 = 70% by weight/30% by weight) were dissolved in xylene at the concentration of 3.3% by weight. Using the resulting xylene solution, a film was formed with a thickness of 80 nm on the second organic layer by a spin coating method, and then heated in a nitrogen gas atmosphere at 130°C for 10 minutes to form a first organic layer.

### (Formation of Cathode)

The substrate including the first organic layer formed thereon was placed in a vapor deposition machine and the pressure in the machine was reduced to 1.0 × 10⁻⁴ Pa or less, and then sodium fluoride was vapor-deposited with a thickness of about 4 nm on the first organic layer, as an anode, and aluminum was vapor-deposited with a thickness of about 80 nm on the sodium fluoride layer. After the vapor deposition, sealing was performed using a glass substrate to fabricate a light emitting device CD1.

### (Evaluation of Light Emitting Device)

When voltage was applied to the light emitting device D1, EL emission was observed. The chromaticity coordinate (x, y) at 1,000 cd/m² was (0.32, 0.63). After setting the current value so that an initial luminance became 4,000 cd/m², the device was driven at constant current and the required time until the emission luminance reached 80% of the initial luminance was measured and found to be 75.9 hours.

### <Example D2> Fabrication and Evaluation of Light Emitting Device D2

In the same manner as in Example D1, except that "the compound H1 and the phosphorescent compound G4 (compound H1/phosphorescent compound G4 = 70% by weight/30% by weight)" were used in place of "the compound H1 and the phosphorescent compound G2 (compound H1/phosphorescent compound G2 = 70% by weight/30% by weight)" in (Formation of First Organic Layer) of Example D1, a light emitting device D2 was fabricated.

### (Evaluation of Light Emitting Device)

When voltage was applied to the light emitting device D2, EL emission was observed. The chromaticity coordinate (x, y) at 1,000 cd/m² was (0.30, 0.62). After setting the current value so that an initial luminance became 4,000 cd/m², the device was driven at constant current and the required time until the emission luminance reached 80% of the initial luminance was measured and found to be 12.1 hours.

### <Example D3> Fabrication and Evaluation of Light Emitting Device D3

In the same manner as in Example D1, except that "the compound H1 and the phosphorescent compound G5 (compound H1/phosphorescent compound G5 = 70% by weight/30% by weight)" were used in place of "the compound H1 and the phosphorescent compound G2 (compound H1/phosphorescent compound G2 = 70% by weight/30% by weight)" in (Formation of First Organic Layer) of Example D1, a light emitting device D3 was fabricated.

### (Evaluation of Light Emitting Device)

When voltage was applied to the light emitting device D3, EL emission was observed. The chromaticity coordinate (x, y) at 1,000 cd/m² was (0.32, 0.62). After setting the current value so that an initial luminance became 4,000 cd/m², the device was driven at constant current and the required time until the emission luminance reached 80% of the initial luminance was measured and found to be 88.3 hours.

### <Example D4> Fabrication and Evaluation of Light Emitting Device D4

In the same manner as in Example D1, except that "the compound H1 and the phosphorescent compound G3 (compound H1/phosphorescent compound G3 = 70% by weight/30% by weight)" were used in place of "the compound H1 and the phosphorescent compound G2 (compound H1/phosphorescent compound G2 = 70% by weight/30% by weight)" in (Formation of First Organic Layer) of Example D1, a light emitting device D4 was fabricated.

### (Evaluation of Light Emitting Device)

When voltage was applied to the light emitting device D4, EL emission was observed. The chromaticity coordinate (x, y) at 1,000 cd/m² was (0.32, 0.63). After setting the current value so that an initial luminance became 4,000 cd/m², the device was driven at constant current and the required time until the emission luminance reached 80% of the initial luminance was measured and found to be 63.9 hours.

### <Example D5> Fabrication and Evaluation of Light Emitting Device D5

In the same manner as in Example D1, except that "the compound H5 and the phosphorescent compound G3 (compound H5/phosphorescent compound G3 = 70% by weight/30% by weight) were dissolved in chlorobenzene at the concentration of 2.2% by weight" in place of "dissolving the compound H1 and the phosphorescent compound G2 (compound H1/phosphorescent compound G2 = 70% by weight/30% by weight) in xylene at the concentration of 3.3% by weight" in (Formation of First Organic Layer) of Example D1, a light emitting device D5 was fabricated.

### (Evaluation of Light Emitting Device)

When voltage was applied to the light emitting device D5, EL emission was observed. The chromaticity coordinate (x, y) at 1,000 cd/m² was (0.31, 0.64). After setting the current value so that an initial luminance became 4,000 cd/m², the device was driven at constant current and the required time until the emission luminance reached 80% of the initial luminance was measured and found to be 44.9 hours.

### <Example D6> Fabrication and Evaluation of Light Emitting Device D6

In the same manner as in Example D1, except that "the compound H5 and the phosphorescent compound G2 (compound H5/phosphorescent compound G2 = 70% by weight/30% by weight) were dissolved in chlorobenzene at the concentration of 2.2% by weight" in place of "dissolving the compound H1 and the phosphorescent compound G2 (compound H1/phosphorescent compound G2 = 70% by weight/30% by weight) in xylene at the concentration of 3.3% by weight" in (Formation of First Organic Layer) of Example D1, a light emitting device D6 was fabricated.

### (Evaluation of Light Emitting Device)

When voltage was applied to the light emitting device D6, EL emission was observed. The chromaticity coordinate (x, y) at 1,000 cd/m² was (0.32, 0.63). After setting the current value so that an initial luminance became 4,000 cd/m², the device was driven at constant current and the required time until the emission luminance reached 80% of the initial luminance was measured and found to be 63.9 hours.

### <Example D7> Fabrication and Evaluation of Light Emitting Device D7

In the same manner as in Example D1, except that "polymer compound HTL-2" was used in place of "polymer compound HTL-1" in (Formation of Second Organic Layer) of Example D1, and that "the compound H1 and the phosphorescent compound G2 (compound H1/phosphorescent compound G2 = 70% by weight/30% by weight) were dissolved in chlorobenzene at the concentration of 2.2% by weight" in place of "dissolving the compound H1 and the phosphorescent compound G2 (compound H1/phosphorescent compound G2 = 70% by weight/30% by weight) in xylene at the concentration of 3.3% by weight" in (Formation of First Organic Layer) of Example D1, a light emitting device D7 was fabricated.

### (Evaluation of Light Emitting Device)

When voltage was applied to the light emitting device D7, EL emission was observed. The chromaticity coordinate (x, y) at 1,000 cd/m² was (0.33, 0.63). After setting the current value so that an initial luminance became 4,000 cd/m², the device was driven at constant current and the required time until the emission luminance reached 80% of the initial luminance was measured and found to be 118.1 hours.

### <Comparative Example CD1> Fabrication and Evaluation of Light Emitting Device CD1

In the same manner as in Example D1, except that "the compound H2 and the phosphorescent compound G1 (compound H2/phosphorescent compound G1 = 70% by weight/30% by weight) were dissolved in chlorobenzene at the concentration of 2% by weight" in place of "dissolving the compound H1 and the phosphorescent compound G2 (compound H1/phosphorescent compound G2 = 70% by weight/30% by weight) in xylene at the concentration of 3.3% by weight" in (Formation of First Organic Layer) of Example D1, and that "the polymer compound HTL-1 was dissolved in xylene at the concentration of 0.7% by weight" in place of "dissolving the polymer compound HTL-1 in xylene at the concentration of 0.6% by weight" in (Formation of Second Organic Layer) of Example D1, a light emitting device CD1 was fabricated.

### (Evaluation of Light Emitting Device)

When voltage was applied to the light emitting device CD1, EL emission was observed. The chromaticity coordinate (x, y) at 1,000 cd/m² was (0.32, 0.63). After setting the current value so that an initial luminance became 4,000 cd/m², the device was driven at constant current and the required time until the emission luminance reached 80% of the initial luminance was measured and found to be 0.3 hour.

### <Comparative Example CD2> Fabrication and Evaluation of Light Emitting Device CD2

In the same manner as in Example D1, except that "the compound H6 and the phosphorescent compound G4 (compound H6/phosphorescent compound G4 = 70% by weight/30% by weight)" were used in place of "the compound H1 and the phosphorescent compound G2 (compound H1/phosphorescent compound G2 = 70% by weight/30% by weight)" in (Formation of First Organic Layer) of Example D1, a light emitting device CD2 was fabricated.

### (Evaluation of Light Emitting Device)

When voltage was applied to the light emitting device CD2, EL emission was observed. The chromaticity coordinate (x, y) at 1,000 cd/m² was (0.29, 0.63). After setting the current value so that an initial luminance became 4,000 cd/m², the device was driven at constant current and the required time until the emission luminance reached 80% of the initial luminance was measured and found to be 1.4 hours.

### <Comparative Example CD3> Fabrication and Evaluation of Light Emitting Device CD3

In the same manner as in Example D1, except that "the compound H2 and the phosphorescent compound G3 (compound H2/phosphorescent compound G3 = 70% by weight/30% by weight) were dissolved in chlorobenzene at the concentration of 2% by weight" in place of "dissolving the compound H1 and the phosphorescent compound G2 (compound H1/phosphorescent compound G2 = 70% by weight/30% by weight) in xylene at the concentration of 3.3% by weight" in (Formation of First Organic Layer) of Example D1, a light emitting device CD3 was fabricated.

### (Evaluation of Light Emitting Device)

When voltage was applied to the light emitting device CD3, EL emission was observed. The chromaticity coordinate (x, y) at 1,000 cd/m² was (0.31, 0.64). After setting the current value so that an initial luminance became 4,000 cd/m², the device was driven at constant current and the required time until the emission luminance reached 80% of the initial luminance was measured and found to be 4.6 hours.

### <Comparative Example CD4> Fabrication and Evaluation of Light Emitting Device CD4

In the same manner as in Example D1, except that "the compound H2 and the phosphorescent compound G4 (compound H2/phosphorescent compound G4 = 70% by weight/30% by weight) were dissolved in chlorobenzene at the concentration of 2% by weight" in place of "dissolving the compound H1 and the phosphorescent compound G2 (compound H1/phosphorescent compound G2 = 70% by weight/30% by weight) in xylene at the concentration of 3.3% by weight" in (Formation of First Organic Layer) of Example D1, a light emitting device CD4 was fabricated.

### (Evaluation of Light Emitting Device)

When voltage was applied to the light emitting device CD4, EL emission was observed. The chromaticity coordinate (x, y) at 1,000 cd/m² was (0.29, 0.64). After setting the current value so that an initial luminance became 4,000 cd/m², the device was driven at constant current and the required time until the emission luminance reached 80% of the initial luminance was measured and found to be 2.5 hours.

The results obtained in Examples D1 to D7 and Comparative Examples CD1 to CD4 are shown in Table 2.

**[Table 2]**

| | Light emitting device | Second organic layer | First organic layer | | Luminance life (hours) |
|---|---|---|---|---|---|
| | | Material | Material | Material ratio (% by weight) | |
| Example D1 | D1 | Crosslinked body of HTL-1 | H1/G2 | 70/30 | 75.9 |
| Example D2 | D2 | Crosslinked body of HTL-1 | H1/G4 | 70/30 | 12.1 |
| Example D3 | D3 | Crosslinked body of HTL-1 | H1/G5 | 70/30 | 88.3 |
| Example D4 | D4 | Crosslinked body of HTL-1 | H1/G3 | 70/30 | 63.9 |
| Example D5 | D5 | Crosslinked body of HTL-1 | H5/G3 | 70/30 | 44.9 |
| Example D6 | D6 | Crosslinked body of HTL-1 | H5/G2 | 70/30 | 63.9 |
| Example D7 | D7 | Crosslinked body of HTL-2 | H1/G2 | 70/30 | 118.1 |
| Comparative Example CD1 | CD1 | Crosslinked body of HTL-1 | H2/G1 | 70/30 | 0.3 |
| Comparative Example CD2 | CD2 | Crosslinked body of HTL-1 | H6/G4 | 70/30 | 1.4 |
| Comparative Example CD3 | CD3 | Crosslinked body of HTL-1 | H2/G3 | 70/30 | 4.6 |
| Comparative Example CD4 | CD4 | Crosslinked body of HTL-1 | H2/G4 | 70/30 | 2.5 |

### <Example D8> Fabrication and Evaluation of Light Emitting Device D8

In the same manner as in Example D1, except that "polymer compound HTL-4" was used in place of "polymer compound HTL-1" in (Formation of Second Organic Layer) of Example D1, and that "the compound H1 and the phosphorescent compound G2 (compound H1/phosphorescent compound G2 = 70% by weight/30% by weight) were dissolved in chlorobenzene at the concentration of 2.2% by weight" in place of "dissolving the compound H1 and the phosphorescent compound G2 (compound H1/phosphorescent compound G2 = 70% by weight/30% by weight) in xylene at the concentration of 3.3% by weight" in (Formation of First Organic Layer) of Example D1, a light emitting device D8 was fabricated.

### (Evaluation of Light Emitting Device)

When voltage was applied to the light emitting device D8, EL emission was observed. The chromaticity coordinate (x, y) at 1,000 cd/m² was (0.33, 0.63). After setting the current value so that an initial luminance became 4,000 cd/m², the device was driven at constant current and the required time until the emission luminance reached 75% of the initial luminance was measured and found to be 78.4 hours.

### <Comparative Example CD5> Fabrication and Evaluation of Light Emitting Device CD5

In the same manner as in Example D1, except that "polymer compound HTL-5" was used in place of "polymer compound HTL-1" in (Formation of Second Organic Layer) of Example D1, and that "the compound H1 and the phosphorescent compound G2 (compound H1/phosphorescent compound G2 = 70% by weight/30% by weight) were dissolved in chlorobenzene at the concentration of 2.2% by weight" in place of "dissolving the compound H1 and the phosphorescent compound G2 (compound H1/phosphorescent compound G2 = 70% by weight/30% by weight) in xylene at the concentration of 3.3% by weight" in (Formation of First Organic Layer) of Example D1, a light emitting device CD5 was fabricated.

### (Evaluation of Light Emitting Device)

When voltage was applied to the light emitting device CD5, EL emission was observed. The chromaticity coordinate (x, y) at 1,000 cd/m² was (0.33, 0.63). After setting the current value so that an initial luminance became 4,000 cd/m², the device was driven at constant current and the required time until the emission luminance reached 75% of the initial luminance was measured and found to be 52.5 hours.

The results obtained in Example D8 and Comparative Example CD5 are shown in Table 3.

**[Table 3]**

| | Light emitting device | Second organic layer | First organic layer | | Luminanc e life (hours) |
|---|---|---|---|---|---|
| | | Material | Materia l | Material ratio (% by weight) | |
| Example D8 | D8 | Crosslink ed body | H1/G2 | 70/30 | 78.4 |
| | | of HTL-4 | | | |
| Comparati ve Example CD5 | CD5 | HTL-5 | H1/G2 | 70/30 | 52.5 |

### <Example D9> Fabrication and Evaluation of Light Emitting Device D9

### (Formation of Anode and Hole Injection Layer)

An ITO film with a thickness of 45 nm was attached to a glass substrate by a sputtering method to form an anode. On the anode, a film was formed with a thickness of 65 nm by a spin coating method using a polythiophene-sulfonic acid-based hole injection agent AQ-1200 (manufactured by Plextronics Inc.), and then heated on a hot plate at 170°C in an air atmosphere for 15 minutes to form a hole injection layer.

### (Formation of Second Organic Layer)

The polymer compound HTL-1 was dissolved in xylene at the concentration of 0.7% by weight. Using the resulting xylene solution, a film was formed on the hole injection layer with a thickness of 20 nm by a spin coating method, and then heated on a hot plate at 180°C in a nitrogen gas atmosphere for 60 minutes to form a second organic layer. As a result of heating, the polymer compound HTL-1 was converted into a crosslinked body.

### (Formation of First Organic Layer)

The compound H1 and the phosphorescent compound R1 (compound H1/phosphorescent compound R1 = 90% by weight/10% by weight) were dissolved in chlorobenzene at the concentration of 2.5% by weight. Using the resulting chlorobenzene solution, a film was formed with a thickness of 80 nm on the second organic layer by a spin coating method, and then heated in a nitrogen gas atmosphere at 130°C for 10 minutes to form a first organic layer.

### (Formation of Cathode)

The substrate including the first organic layer formed thereon was placed in a vapor deposition machine and the pressure in the machine was reduced to 1.0 × 10⁻⁴ Pa or less, and then sodium fluoride was vapor-deposited with a thickness of about 4 nm on the first organic layer, as an anode, and aluminum was vapor-deposited with a thickness of about 80 nm on the sodium fluoride layer. After the vapor deposition, sealing was performed using a glass substrate to fabricate a light emitting device D9.

### (Evaluation of Light Emitting Device)

When voltage was applied to the light emitting device D9, EL emission was observed. The chromaticity coordinate (x, y) at 1,000 cd/m² was (0.62, 0.33). After setting the current value so that an initial luminance became 4,000 cd/m², the device was driven at constant current and the required time until the emission luminance reached 50% of the initial luminance was measured and found to be 15.4 hours.

### <Example D10> Fabrication and Evaluation of Light Emitting Device D10

In the same manner as in Example D9, except that "the compound H1 and the phosphorescent compound R2 (compound H1/phosphorescent compound R2 = 90% by weight/10% by weight)" were used in place of "the compound H1 and the phosphorescent compound R1 (compound H1/phosphorescent compound R1 = 90% by weight/10% by weight)" in (Formation of First Organic Layer) of Example D9, a light emitting device D10 was fabricated.

### (Evaluation of Light Emitting Device)

When voltage was applied to the light emitting device D10, EL emission was observed. The chromaticity coordinate (x, y) at 1,000 cd/m² was (0.59, 0.40). After setting the current value so that an initial luminance became 4,000 cd/m², the device was driven at constant current and the required time until the emission luminance reached 50% of the initial luminance was measured and found to be 75.4 hours.

### <Example D11> Fabrication and Evaluation of Light Emitting Device D11

In the same manner as in Example D9, except that "the compound H1 and the phosphorescent compound R3 (compound H1/phosphorescent compound R3 = 90% by weight/10% by weight)" were used in place of "the compound H1 and the phosphorescent compound R1 (compound H1/phosphorescent compound R1 = 90% by weight/10% by weight)" in (Formation of First Organic Layer) of Example D9, a light emitting device D11 was fabricated.

### (Evaluation of Light Emitting Device)

When voltage was applied to the light emitting device D11, EL emission was observed. The chromaticity coordinate (x, y) at 1,000 cd/m² was (0.62, 0.36). After setting the current value so that an initial luminance became 4,000 cd/m², the device was driven at constant current and the required time until the emission luminance reached 50% of the initial luminance was measured and found to be 34.3 hours.

### <Example D12> Fabrication and Evaluation of Light Emitting Device D12

In the same manner as in Example D9, except that "the compound H5 and the phosphorescent compound R1 (compound H5/phosphorescent compound R1 = 90% by weight/10% by weight) were dissolved in chlorobenzene at the concentration of 2.2% by weight" in place of "dissolving the compound H1 and the phosphorescent compound R1 (compound H1/phosphorescent compound R1 = 90% by weight/10% by weight) in chlorobenzene at the concentration of 2.5% by weight" in (Formation of First Organic Layer) of Example D9, a light emitting device D12 was fabricated.

### (Evaluation of Light Emitting Device)

When voltage was applied to the light emitting device D12, EL emission was observed. The chromaticity coordinate (x, y) at 1,000 cd/m² was (0.68, 0.32). After setting the current value so that an initial luminance became 4,000 cd/m², the device was driven at constant current and the required time until the emission luminance reached 50% of the initial luminance was measured and found to be 20.0 hours.

### <Example D13> Fabrication and Evaluation of Light Emitting Device D13

In the same manner as in Example D9, except that "polymer compound HTL-2" was used in place of "polymer compound HTL-1" in (Formation of Second Organic Layer) of Example D9, and that "the compound H1 and the phosphorescent compound R1 (compound H1/phosphorescent compound R1 = 90% by weight/10% by weight) were dissolved in toluene at the concentration of 2.2% by weight" in place of "dissolving the compound H1 and the phosphorescent compound R1 (compound H1/phosphorescent compound R1 = 90% by weight/10% by weight) in chlorobenzene at the concentration of 2.5% by weight" in (Formation of First Organic Layer) of Example D9, a light emitting device D13 was fabricated.

### (Evaluation of Light Emitting Device)

When voltage was applied to the light emitting device D13, EL emission was observed. The chromaticity coordinate (x, y) at 1,000 cd/m² was (0.67, 0.33). After setting the current value so that an initial luminance became 4,000 cd/m², the device was driven at constant current and the required time until the emission luminance reached 50% of the initial luminance was measured and found to be 12.9 hours.

### <Comparative Example CD6> Fabrication and Evaluation of Light Emitting Device CD6

In the same manner as in Example D9, except that "the compound H3 and the phosphorescent compound R1 (compound H3/phosphorescent compound R1 = 90% by weight/10% by weight)" were used in place of "the compound H1 and the phosphorescent compound R1 (compound H1/phosphorescent compound R1 = 90% by weight/10% by weight)" in (Formation of First Organic Layer) of Example D9, a light emitting device CD6 was fabricated.

### (Evaluation of Light Emitting Device)

When voltage was applied to the light emitting device CD6, EL emission was observed. The chromaticity coordinate (x, y) at 1,000 cd/m² was (0.67, 0.33). After setting the current value so that an initial luminance became 4,000 cd/m², the device was driven at constant current and the required time until the emission luminance reached 50% of the initial luminance was measured and found to be 10.4 hours.

### <Comparative Example CD7> Fabrication and Evaluation of Light Emitting Device CD7

In the same manner as in Example D9, except that "the compound H4 and the phosphorescent compound R1 (compound H4/phosphorescent compound R1 = 90% by weight/10% by weight)" were used in place of "the compound H1 and the phosphorescent compound R1 (compound H1/phosphorescent compound R1 = 90% by weight/10% by weight)" in (Formation of First Organic Layer) of Example D9, a light emitting device CD7 was fabricated.

### (Evaluation of Light Emitting Device)

When voltage was applied to the light emitting device CD7, EL emission was observed. The chromaticity coordinate (x, y) at 1,000 cd/m² was (0.66, 0.34). After setting the current value so that an initial luminance became 4,000 cd/m², the device was driven at constant current and the required time until the emission luminance reached 50% of the initial luminance was measured and found to be 2.0 hours.

### <Comparative Example CD8> Fabrication and Evaluation of Light Emitting Device CD8

In the same manner as in Example D9, except that "polymer compound HTL-3" was used in place of "polymer compound HTL-1" in (Formation of Second Organic Layer) of Example D9, and that "the compound H1 and the phosphorescent compound R1 (compound H1/phosphorescent compound R1 = 90% by weight/10% by weight) were dissolved in toluene at the concentration of 2.2% by weight" in place of "dissolving the compound H1 and the phosphorescent compound R1 (compound H1/phosphorescent compound R1 = 90% by weight/10% by weight) in chlorobenzene at the concentration of 2.5% by weight" in (Formation of First Organic Layer) of Example D9, a light emitting device CD8 was fabricated.

### (Evaluation of Light Emitting Device)

When voltage was applied to the light emitting device CD8, EL emission was observed. The chromaticity coordinate (x, y) at 1,000 cd/m² was (0.67, 0.32). After setting the current value so that an initial luminance became 4,000 cd/m², the device was driven at constant current and the required time until the emission luminance reached 50% of the initial luminance was measured and found to be 4.9 hours.

The results obtained in Examples D9 to D13 and Comparative Examples CD6 to CD8 are shown in Table 4.

**[Table 4]**

| | Light emitting device | Second organic layer | First organic layer | | Luminanc e life (hours) |
|---|---|---|---|---|---|
| | | Material | Material | Material ratio (% by weight) | |
| Example D9 | D9 | Crosslinked body of HTL-1 | H1/R1 | 90/10 | 15.4 |
| Example D10 | D10 | Crosslinked body of HTL-1 | H1/R2 | 90/10 | 75.4 |
| Example D11 | D11 | Crosslinked body of HTL-1 | H1/R3 | 90/10 | 34.3 |
| Example D12 | D12 | Crosslinked body of HTL-1 | H5/R1 | 90/10 | 20.0 |
| Example D13 | D13 | Crosslinked body of HTL-2 | H1/R1 | 90/10 | 12.9 |
| Comparative Example CD6 | CD6 | Crosslinked body of HTL-1 | H3/R1 | 90/10 | 10.4 |
| Comparative Example CD7 | CD7 | Crosslinked body of HTL-1 | H4/R1 | 90/10 | 2.0 |
| Comparative Example CD8 | CD8 | HTL-3 | H1/R1 | 90/10 | 4.9 |

### Industrial Applicability

An object of the embodiment of the present invention is to provide a light emitting device excellent in luminance life.

## Claims

1. A light emitting device comprising an anode, a cathode, a first organic layer disposed between the anode and the cathode, and a second organic layer disposed between the anode and the cathode, wherein
the first organic layer is a layer comprising a phosphorescent compound represented by formula (1) and a compound represented by formula (H), and
the second organic layer is a layer comprising a crosslinked body of a crosslinkable material: 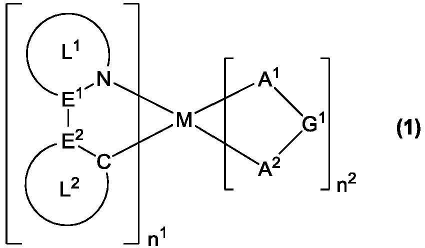 wherein
M represents a ruthenium atom, a rhodium atom, a palladium atom, an iridium atom or a platinum atom,
n¹ represents an integer of 1 or more, n² represents an integer of 0 or more, n¹+n² is 2 or 3, n¹+n² is 3 when M is a ruthenium atom, a rhodium atom or an iridium atom, and n¹+n² is 2 when M is a palladium atom or a platinum atom,
E¹ and E² each independently represent a carbon atom or a nitrogen atom, and at least one of E¹ and E² is a carbon atom, and when a plurality of E¹ and E² are present, they may be the same or different at each occurrence,
ring L¹ represents an aromatic heterocyclic ring, and the ring optionally has a substituent, and when a plurality of the substituents are present, they may be the same or different and may be combined together to form a ring together with the atoms to which they are attached, and when a plurality of the rings L¹ are present, they may be the same or different,
ring L² represents an aromatic hydrocarbon ring or an aromatic heterocyclic ring, these rings each optionally have a substituent, and when a plurality of the substituents are present, they may be the same or different and may be combined together to form a ring together with the atoms to which they are attached, and when a plurality of the rings L² are present, they may be the same or different,
the substituent which the ring L¹ optionally has and the substituent which the ring L² optionally has may be combined together to form a ring together with the atoms to which they are attached, and
A¹-G¹-A² represents an anionic bidentate ligand, A¹ and A² each independently represent a carbon atom, an oxygen atom or a nitrogen atom, and these atoms each may be an atom constituting a ring, G¹ represents a single bond or an atomic group constituting a bidentate ligand together with A¹ and A², and when a plurality of A¹-G¹-A² are present, they may be the same or different: 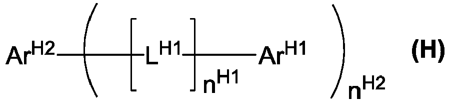
wherein
n^{H1} represents an integer of 0 or more and 5 or less, and when a plurality of n^{H1} are present, they may be the same or different,
n^{H2} represents an integer of 1 or more and 10 or less,
Ar^{H1} represents a group represented by formula (H1-1), and when a plurality of Ar^{H1} are present, they may be the same or different,
L^{H1} represents an alkylene group, a cycloalkylene group, an arylene group, a divalent heterocyclic group, a group represented by -NR^{H1'}-, an oxygen atom or a sulfur atom, and these groups each optionally have a substituent, R^{H1'} represents a hydrogen atom, an alkyl group, a cycloalkyl group, an aryl group or a monovalent heterocyclic group, and these groups each optionally have a substituent, and when a plurality of L^{H1} are present, they may be the same or different, and
Ar^{H2} represents an aromatic hydrocarbon group or an aromatic heterocyclic group, and these groups each optionally have a substituent: 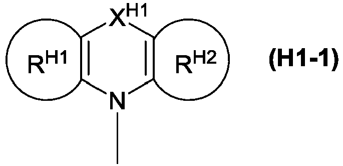
wherein
ring R^{H1} and ring R^{H2} each independently represent a monocyclic or fused-ring aromatic hydrocarbon ring, or a monocyclic or fused-ring aromatic heterocyclic ring, and these rings each optionally have a substituent, and when a plurality of the substituents are present, they may be the same or different and may be combined together to form a ring together with the atoms to which they are attached,
at least one of the ring R^{H1} and the ring R^{H2} represents a fused-ring aromatic hydrocarbon ring or a fused-ring aromatic heterocyclic ring, and these rings each optionally have a substituent,
X^{H1} represents a single bond, an oxygen atom, a sulfur atom, a group represented by -N(R^{XH1})- or a group represented by -C(R^{XH1'})₂-, R^{XH1} and R^{XH1'} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and these groups each optionally have a substituent, and the plurality of R^{XH1'} may be the same or different and may be combined together to form a ring together with the carbon atoms to which they are attached, and
R^{XH1} and the substituent which the ring R^{H1} optionally has, R^{XH1} and the substituent which the ring R^{H2} optionally has, R^{XH1'} and the substituent which the ring R^{H1} optionally has, and R^{XH1'} and the substituent which the ring R^{H2} optionally has each may be combined together to form a ring together with the atoms to which they are attached.

2. The light emitting device according to claim 1, wherein the crosslinkable material is a low molecular weight compound having at least one crosslinkable group selected from Group A of crosslinkable group, or a polymer compound comprising a crosslinkable constitutional unit having at least one crosslinkable group selected from Group A of crosslinkable group:
(Group A of Crosslinkable Group) 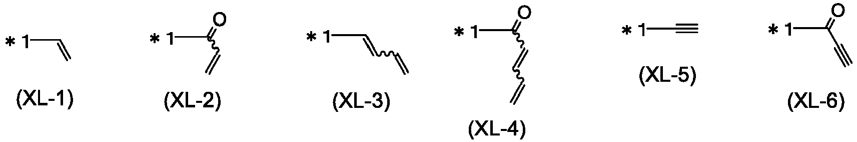 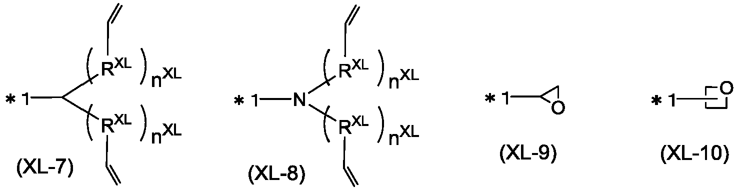 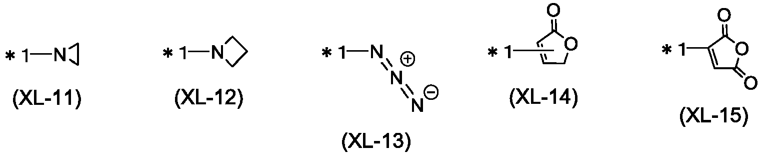 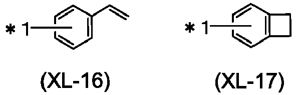 wherein R^{XL} represents a methylene group, an oxygen atom or a sulfur atom, n^{XL} represents an integer of 0 to 5, and when a plurality of R^{XL} are present, they may be the same or different, and when a plurality of n^{XL} are present, they may be the same or different, *1 represents a binding site, and these crosslinkable groups each optionally have a substituent.

3. The light emitting device according to claim 2, wherein the crosslinkable material is a polymer compound comprising a crosslinkable constitutional unit having at least one crosslinkable group selected from Group A of crosslinkable group.

4. The light emitting device according to claim 3,
wherein the crosslinkable constitutional unit is a constitutional unit represented by formula (2) or a constitutional unit represented by formula (2'): 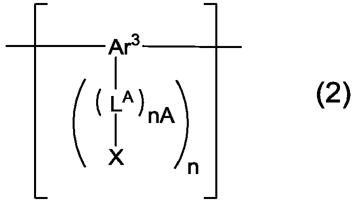 wherein
nA represents an integer of 0 to 5, n represents 1 or 2, and when a plurality of nA are present, they may be the same or different,
Ar³ represents an aromatic hydrocarbon group or a heterocyclic group, and these groups each optionally have a substituent,
L^{A} represents an alkylene group, a cycloalkylene group, an arylene group, a divalent heterocyclic group, a group represented by -NR'-, an oxygen atom or a sulfur atom, and these groups each optionally have a substituent, R' represents a hydrogen atom, an alkyl group, a cycloalkyl group, an aryl group or a monovalent heterocyclic group, and these groups each optionally have a substituent, and when a plurality of L^{A} are present, they may be the same or different, and
X represents a crosslinkable group selected from Group A of crosslinkable group, and when a plurality of X are present, they may be the same or different: 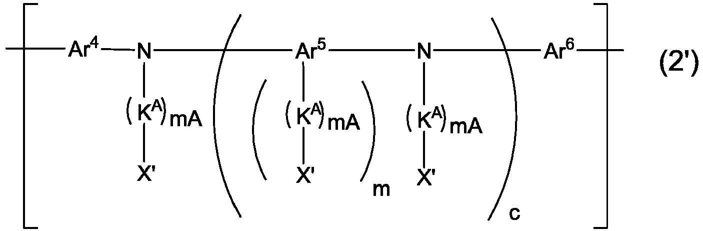
wherein
mA represents an integer of 0 to 5, m represents an integer of 1 to 4, c represents an integer of 0 or 1, and when a plurality of mA are present, they may be the same or different,
Ar⁵ represents an aromatic hydrocarbon group, a heterocyclic group, or a group in which at least one aromatic hydrocarbon ring and at least one heterocyclic ring are bonded directly to each other, and these groups each optionally have a substituent,
Ar⁴ and Ar⁶ each independently represent an arylene group or a divalent heterocyclic group, and these groups each optionally have a substituent,
Ar⁴, Ar⁵ and Ar⁶ each may be bonded directly or via an oxygen atom or a sulfur atom to a group other than these groups bonding to the nitrogen atom to which these groups are attached, thereby forming a ring,
K^{A} represents an alkylene group, a cycloalkylene group, an arylene group, a divalent heterocyclic group, a group represented by -NR'-, an oxygen atom or a sulfur atom, and these groups each optionally have a substituent, R' represents a hydrogen atom, an alkyl group, a cycloalkyl group, an aryl group or a monovalent heterocyclic group, and these groups each optionally have a substituent, and when a plurality of K^{A} are present, they may be the same or different, and
X' represents a crosslinkable group selected from Group A of crosslinkable group, a hydrogen atom, an alkyl group, a cycloalkyl group, an aryl group or a monovalent heterocyclic group, and these groups each optionally have a substituent, and when a plurality of X' are present, they may be the same or different, and at least one X' is a crosslinkable group selected from Group A of crosslinkable group.

5. The light emitting device according to any one of claims 2 to 4, wherein the crosslinkable group which the crosslinkable material has is a group represented by formula (XL-2), formula (XL-3), formula (XL-4), formula (XL-5), formula (XL-6), formula (XL-7), formula (XL-8), formula (XL-9), formula (XL-10), formula (XL-11), formula (XL-12), formula (XL-13), formula (XL-14), formula (XL-15) or formula (XL-17).

6. The light emitting device according to any one of claims 1 to 5, wherein the group represented by formula (H1-1) is a group represented by formula (H1-1B), a group represented by formula (H1-1C) or a group represented by formula (H1-1D): 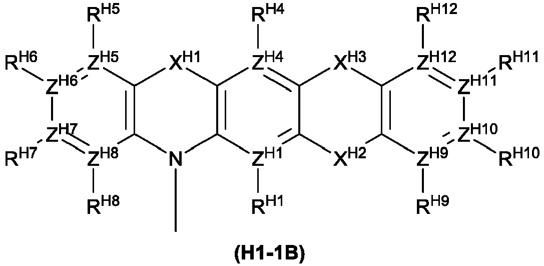 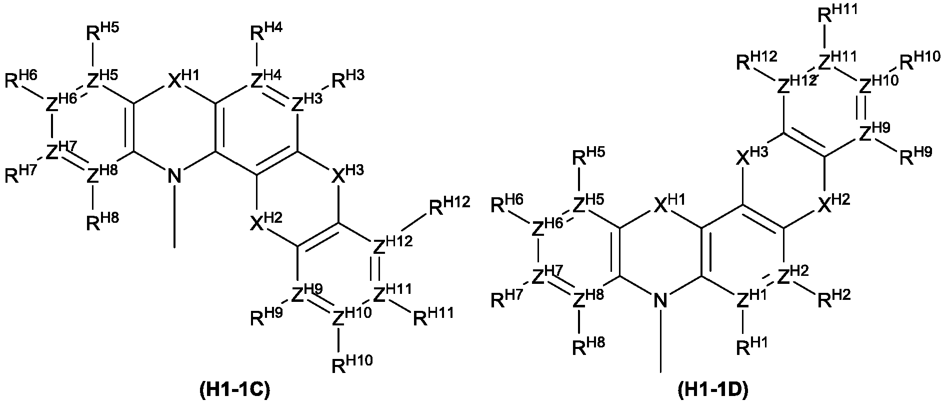 wherein
X^{H1} represents the same meaning as defined above,
X^{H2} and X^{H3} each independently represent a single bond, an oxygen atom, a sulfur atom, a group represented by - N(R^{XH2})- or a group represented by -C(R^{XH2'})₂-, R^{XH2} and R^{XH2'} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and these groups each optionally have a substituent, and the plurality of R^{XH2'} may be the same or different and may be combined together to form a ring together with the carbon atoms to which they are attached,
Z^{H1}, Z^{H2}, Z^{H3}, Z^{H4}, Z^{H5}, Z^{H6}, Z^{H7}, Z^{H8}, Z^{H9}, Z^{H10}, Z^{H11} and Z^{H12} each independently represent a carbon atom or a nitrogen atom,
R^{H1}, R^{H2}, R^{H3}, R^{H4}, R^{H5}, R^{H6}, R^{H7}, R^{H8}, R^{H9}, R^{H10}, R^{H11} and R^{H12} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and these groups each optionally have a substituent,
R^{H1} is not present when Z^{H1} is a nitrogen atom, R^{H2} is not present when Z^{H2} is a nitrogen atom, R^{H3} is not present when Z^{H3} is a nitrogen atom, R^{H4} is not present when Z^{H4} is a nitrogen atom, R^{H5} is not present when Z^{H5} is a nitrogen atom, R^{H6} is not present when Z^{H6} is a nitrogen atom, R^{H7} is not present when Z^{H7} is a nitrogen atom, R^{H8} is not present when Z^{H8} is a nitrogen atom, R^{H9} is not present when Z^{H9} is a nitrogen atom, R^{H10} is not present when Z^{H10} is a nitrogen atom, R^{H11} is not present when Z^{H11} is a nitrogen atom, and R^{H12} is not present when Z^{H12} is a nitrogen atom, and
R^{H1} and R^{H2}, R^{H3} and R^{H4}, R^{H5} and R^{H6}, R^{H6} and R^{H7}, R^{H7} and R^{H8}, R^{H9} and R^{H10}, R^{H10} and R^{H11}, and R^{H11} and R^{H12} each may be combined together to form a ring together with the carbon atoms to which they are attached.

7. The light emitting device according to any one of claims 1 to 6, wherein the phosphorescent compound represented by formula (1) is a phosphorescent compound represented by formula (1-B): 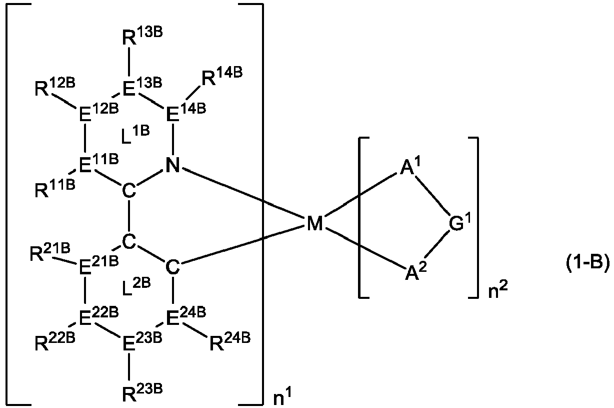 wherein
M, n¹, n² and A¹-G¹-A² represent the same meaning as defined above,
E^{11B}, E^{12B}, E^{13B}, E^{14B}, E^{21B}, E^{22B}, E^{23B} and E^{24B} each independently represent a nitrogen atom or a carbon atom, and when a plurality of E^{11B}, E^{12B}, E^{13B}, E^{14B}, E^{21B}, E^{22B}, E^{23B} and E^{24B} are present, they may be the same or different at each occurrence, R^{11B} is not present when E^{11B} is a nitrogen atom, R^{12B} is not present when E^{12B} is a nitrogen atom, R^{13B} is not present when E^{13B} is a nitrogen atom, R^{14B} is not present when E^{14B} is a nitrogen atom, R^{21B} is not present when E^{21B} is a nitrogen atom, R^{22B} is not present when E^{22B} is a nitrogen atom, R^{23B} is not present when E^{23B} is a nitrogen atom, and R^{24B} is not present when E^{24B} is a nitrogen atom,
R^{11B}, R^{12B}, R^{13B}, R^{14B}, R^{21B}, R^{22B}, R^{23B} and R^{24B} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and these groups each optionally have a substituent, and when a plurality of R^{11B}, R^{12B}, R^{13B}, R^{14B}, R^{21B}, R^{22B}, R^{23B} and R^{24B} are present, they may be the same or different at each occurrence, and R^{11B} and R^{12B}, R^{12B} and R^{13B}, R^{13B} and R^{14B} , R^{11B} and R^{21B}, R^{21B} and R^{22B}, R^{22B} and R^{23B}, and R^{23B} and R^{24B} each may be combined together to form a ring together with the atoms to which they are attached,
ring L^{1B} represents a pyridine ring or a pyrimidine ring constituted of a nitrogen atom, a carbon atom, E^{11B}, E^{12B}, E^{13B} and E^{14B}, and
ring L^{2B} represents a benzene ring, a pyridine ring or a pyrimidine ring constituted of two carbon atoms, E^{21B}, E^{22B}, E²3B and E^{24B}.

8. The light emitting device according to claim 7, wherein the phosphorescent compound represented by formula (1-B) is a phosphorescent compound represented by formula (1-B1), a phosphorescent compound represented by formula (1-B2), a phosphorescent compound represented by formula (1-B3), a phosphorescent compound represented by formula (1-B4) or a phosphorescent compound represented by formula (1-B5): 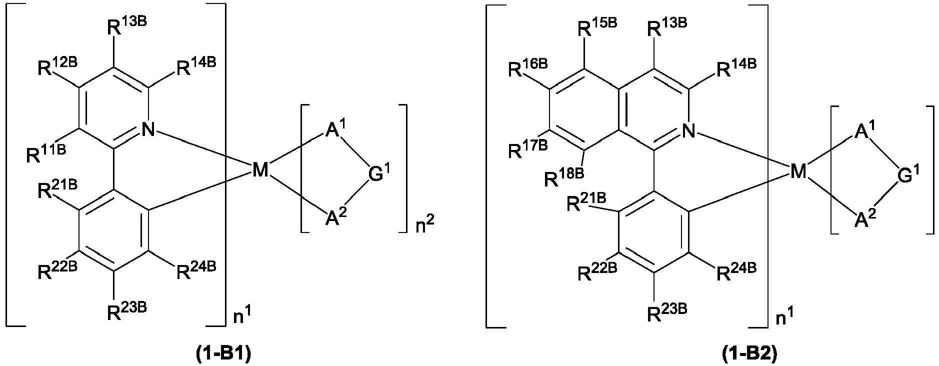 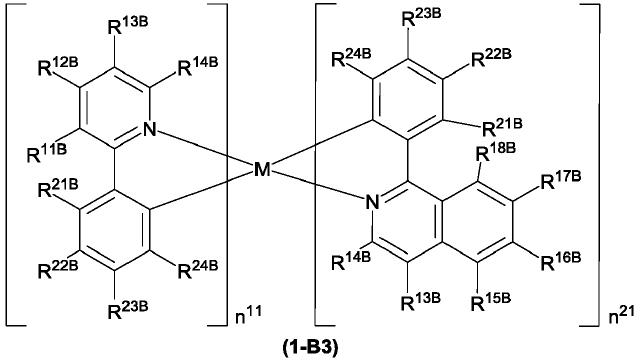 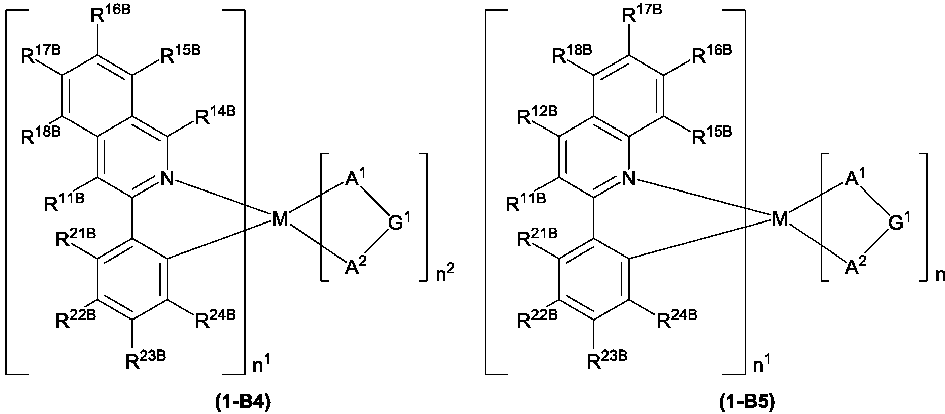 wherein
M, n¹, n², A¹-G¹-A², R^{11B}, R^{12B}, R^{13B}, R^{14B}, R^{21B}, R^{22B}, R^{23B} and R^{24B} represent the same meaning as defined above,
n¹¹ and n¹² each independently represent an integer of 1 or more, n¹¹+n¹² is 2 or 3, n¹¹+n¹² is 3 when M is a ruthenium atom, a rhodium atom or an iridium atom, and n¹¹+n¹² is 2 when M is a palladium atom or a platinum atom, and
R^{15B}, R^{16B}, R^{17B} and R^{18B} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and these groups each optionally have a substituent, and when a plurality of R^{15B}, R^{16B}, R^{17B} and R^{18B} are present, they may be the same or different at each occurrence, and R^{13B} and R^{15B}, R^{15B} and R^{16B}, R^{16B} and R^{17B}, R^{17B} and R^{18B}, and R^{18B} and R^{21B} each may be combined together to form a ring together with the atoms to which they are attached.

9. The light emitting device according to any one of claims 1 to 6, wherein the phosphorescent compound represented by formula (1) is a phosphorescent compound represented by formula (1-A): 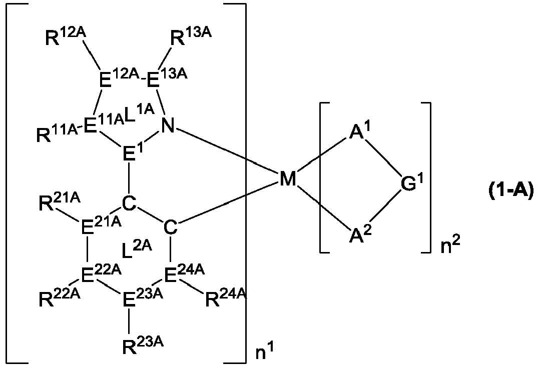 wherein
M, n¹, n², E¹ and A¹-G¹-A² represent the same meaning as defined above,
E^{11A}, E^{12A}, E^{13A}, E^{21A}, E^{22A}, E^{23A} and E^{24A} each independently represent a nitrogen atom or a carbon atom, and when a plurality of E^{11A}, E^{12A}, E^{13A}, E^{21A}, E^{22A}, E^{23A} and E^{24A} are present, they may be the same or different at each occurrence, R^{11A} may be either present or not present when E^{11A} is a nitrogen atom, R^{12A} may be either present or not present when E^{12A} is a nitrogen atom, R^{13A} may be either present or not present when E^{13A} is a nitrogen atom, R^{21A} is not present when E^{21A} is a nitrogen atom, R^{22A} is not present when E^{22A} is a nitrogen atom, R^{23A} is not present when E^{23A} is a nitrogen atom, and R^{24A} is not present when E^{24A} is a nitrogen atom,
R^{11A}, R^{12A}, R^{13A}, R^{21A}, R^{22A}, R^{23A} and R^{24A} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and these groups each optionally have a substituent, and when a plurality of R^{11A}, R^{12A}, R^{13A}, R^{21A}, R^{22A}, R^{23A} and R^{24A} are present, they may be the same or different at each occurrence, and R^{11A} and R^{12A}, R^{12A} and R^{13A}, R^{11A} and R^{21A}, R^{21A} and R^{22A}, R^{22A} and R^{23A}, and R^{23A} and R^{24A} each may be combined together to form a ring together with the atoms to which they are attached,
ring L^{1A} represents a triazole ring or a diazole ring constituted of a nitrogen atom, E¹, E^{11A}, E^{12A} and E^{13A}, and
ring L^{2A} represents a benzene ring, a pyridine ring or a pyrimidine ring constituted of two carbon atoms, E^{21A}, E^{22A}, E^{23A} and E^{24A}.

10. The light emitting device according to any one of claims 1 to 9, wherein the first organic layer and the second organic layer are adjacent to each other.

11. The light emitting device according to any one of claims 1 to 10, wherein the second organic layer is a layer disposed between the anode and the first organic layer.

12. The light emitting device according to any one of claims 1 to 6, wherein the substituent which the ring L¹ optionally has is an aryl group or a monovalent heterocyclic group, and these groups each optionally further have a substituent, and the substituent which the ring L² optionally has is an aryl group or a monovalent heterocyclic group, and these groups each optionally further have a substituent.

13. The light emitting device according to claim 7 or 8, wherein the R^{11B}, the R^{12B}, the R^{13B}, the R^{14B}, the R^{21B}, the R^{22B}, the R^{23B} and the R^{24B} each independently represent a hydrogen atom or a group represented by formula (D-A), a group represented by formula (D-B) or a group represented by 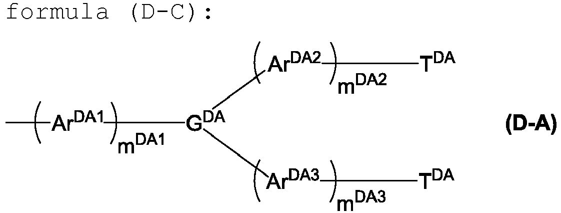 wherein
m^{DA1}, m^{DA2} and m^{DA3} each independently represent an integer of 0 or more,
G^{DA} represents a nitrogen atom, an aromatic hydrocarbon group or a heterocyclic group, and these groups each optionally have a substituent,
Ar^{DA1}, Ar^{DA2} and Ar^{DA3} each independently represent an arylene group or a divalent heterocyclic group, and these groups each optionally have a substituent, and when a plurality of Ar^{DA1}, Ar^{DA2} and Ar^{DA3} are present, they may be the same or different at each occurrence, and
T^{DA} represents an aryl group or a monovalent heterocyclic group, and these groups each optionally have a substituent, and the plurality of T^{DA} may be the same or different: 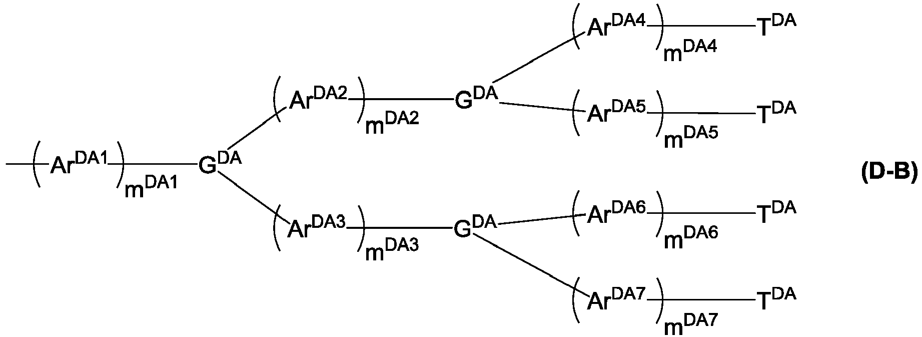
wherein
m^{DA1}, m^{DA2}, m^{DA3}, m^{DA4}, m^{DA5}, m^{DA6} and m^{DA7} each independently represent an integer of 0 or more,
G^{DA} represents a nitrogen atom, an aromatic hydrocarbon group or a heterocyclic group, and these groups each optionally have a substituent, and the plurality of G^{DA} may be the same or different,
Ar^{DA1}, Ar^{DA2}, Ar^{DA3}, Ar^{DA4}, Ar^{DA5}, Ar^{DA6} and Ar^{DA7} each independently represent an arylene group or a divalent heterocyclic group, and these groups each optionally have a substituent, and when a plurality of Ar^{DA1}, Ar^{DA2}, Ar^{DA3}, Ar^{DA4}, Ar^{DA5}, Ar^{DA6} and Ar^{DA7} are present, they may be the same or different at each occurrence, and
T^{DA} represents an aryl group or a monovalent heterocyclic group, and these groups each optionally have a substituent, and the plurality of T^{DA} may be the same or different: 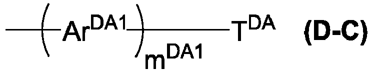
wherein
m^{DA1} represents an integer of 0 or more,
Ar^{DA1} represents an arylene group or a divalent heterocyclic group, and these groups each optionally have a substituent, and when a plurality of Ar^{DA1} are present, they may be the same or different, and
T^{DA} represents an aryl group or a monovalent heterocyclic group, and these groups each optionally have a substituent.

14. The light emitting device according to any one of claims 2 to 13, wherein the crosslinkable group selected from Group A of crosslinkable group is a group represented by formula (XL-17).

## Patentansprüche

1. Lichtemittierende Vorrichtung, umfassend eine Anode, eine Kathode, eine erste organische Schicht, die zwischen der Anode und der Kathode angeordnet ist, und eine zweite organische Schicht, die zwischen der Anode und der Kathode angeordnet ist, wobei
die erste organische Schicht eine Schicht ist, die eine phosphoreszierende Verbindung, dargestellt durch die Formel (1), und eine Verbindung, dargestellt durch die Formel (H), umfasst, und
die zweite organische Schicht eine Schicht ist, die einen vernetzten Körper aus einem vernetzbaren Material umfasst: 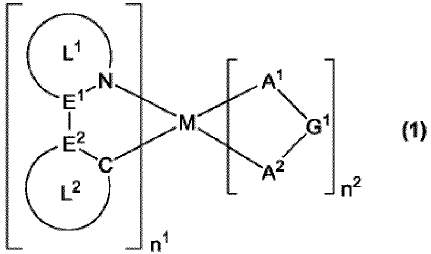 wobei
M ein Rutheniumatom, ein Rhodiumatom, ein Palladiumatom, ein Iridiumatom oder ein Platinatom darstellt
n¹ eine ganze Zahl von 1 oder mehr darstellt, n² eine ganze Zahl von 0 oder mehr darstellt, n¹ +n² 2 oder 3 ist, n¹ +n² 3 ist, wenn M ein Rutheniumatom, ein Rhodiumatom oder ein Iridiumatom ist, und n¹ +n² 2 ist, wenn M ein Palladiumatom oder ein Platinatom ist,
E¹ und E² jeweils unabhängig voneinander ein Kohlenstoffatom oder ein Stickstoffatom darstellen und mindestens eines von E¹ und E² ein Kohlenstoffatom ist und wenn eine Vielzahl von E¹ und E² vorhanden ist, diese bei jedem Auftreten gleich oder verschieden sein können,
der Ring L¹ einen aromatischen heterocyclischen Ring darstellt und der Ring optional einen Substituenten aufweist und wenn eine Vielzahl der Substituenten vorhanden ist, diese gleich oder verschieden sein können und miteinander kombiniert werden können, um zusammen mit den Atomen, an die sie gebunden sind, einen Ring zu bilden, und wenn eine Vielzahl der Ringe L¹ vorhanden ist, diese gleich oder verschieden sein können,
der Ring L² einen aromatischen Kohlenwasserstoffring oder einen aromatischen heterocyclischen Ring darstellt, diese Ringe jeweils optional einen Substituenten aufweisen und wenn eine Vielzahl der Substituenten vorhanden ist, diese gleich oder verschieden sein können und zusammen kombiniert werden können, um zusammen mit den Atomen, an die sie gebunden sind, einen Ring zu bilden, und wenn eine Vielzahl der Ringe L² vorhanden ist, diese gleich oder verschieden sein können,
der Substituent, den der Ring L¹ optional aufweist, und der Substituent, den der Ring L² optional aufweist, miteinander kombiniert werden können, um zusammen mit den Atomen, an die sie gebunden sind, einen Ring zu bilden, und
A¹ -G¹ -A² einen anionischen zweizähnigen Liganden darstellt, A¹ und A² jeweils unabhängig voneinander ein Kohlenstoffatom, ein Sauerstoffatom oder ein Stickstoffatom darstellen und diese Atome jeweils ein Atom sein können, das einen Ring bildet, G¹ eine Einfachbindung oder eine Atomgruppe darstellt, die zusammen mit A¹ und
A² einen zweizähnigen Liganden bildet, und wenn eine Vielzahl von A¹ -G¹ -A² vorhanden ist, diese gleich oder verschieden sein können: 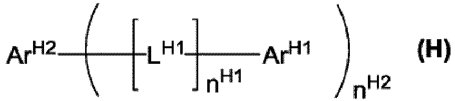
wobei
n^{H1} eine ganze Zahl von 0 oder mehr und 5 oder weniger darstellt und wenn eine Vielzahl von n^{H1} vorhanden ist, diese gleich oder verschieden sein können,
n^{H2} eine ganze Zahl von 1 oder mehr und 10 oder weniger darstellt,
Ar^{H1} eine durch die Formel (H1-1) dargestellte Gruppe darstellt und wenn eine Vielzahl von Ar^{H1} vorhanden ist, diese gleich oder verschieden sein können,
L^{H1} eine Alkylengruppe, eine Cycloalkylengruppe, eine Arylengruppe, eine zweiwertige heterocyclische Gruppe, eine durch -NR^{H1}' - dargestellte Gruppe, ein Sauerstoffatom oder ein Schwefelatom darstellt und diese Gruppen jeweils optional einen Substituenten aufweisen, R^{H1}' ein Wasserstoffatom, eine Alkylgruppe, eine Cycloalkylgruppe, eine Arylgruppe oder eine einwertige heterocyclische Gruppe darstellt und diese Gruppen jeweils optional einen Substituenten aufweisen und wenn eine Vielzahl von L^{H1} vorhanden ist, diese gleich oder verschieden sein können, und
Ar^{H2} eine aromatische Kohlenwasserstoffgruppe oder eine aromatische heterocyclische Gruppe darstellt und diese Gruppen jeweils optional einen Substituenten aufweisen: 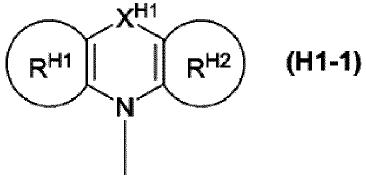
wobei
der Ring R^{H1} und der Ring R^{H2} jeweils unabhängig voneinander einen monocyclischen oder mit einem kondensierten Ring versehenen aromatischen Kohlenwasserstoffring oder einen monocyclischen oder mit einem kondensierten Ring versehenen aromatischen heterocyclischen Ring darstellen und diese Ringe jeweils optional einen Substituenten aufweisen und wenn eine Vielzahl der Substituenten vorhanden ist, diese gleich oder verschieden sein können und miteinander kombiniert werden können, um zusammen mit den Atomen, an die sie gebunden sind, einen Ring zu bilden,
mindestens einer von dem Ring R^{H1} und dem Ring R^{H2} einen mit einem kondensierten Ring versehenen aromatischen Kohlenwasserstoffring oder einen mit einem kondensierten Ring versehenen aromatischen heterocyclischen Ring darstellt und diese Ringe jeweils optional einen Substituenten aufweisen,
X^{H1} eine Einfachbindung, ein Sauerstoffatom, ein Schwefelatom, eine durch -N(R^{XH1})-dargestellte Gruppe oder eine durch -C(R^{XH1'})₂- dargestellte Gruppe darstellt, R^{XH1} und R^{XH1'} jeweils unabhängig voneinander ein Wasserstoffatom, eine Alkylgruppe, eine Cycloalkylgruppe, eine Alkoxygruppe, eine Cycloalkoxygruppe, eine Arylgruppe, eine Aryloxygruppe, eine einwertige heterocyclische Gruppe, eine substituierte Aminogruppe oder ein Halogenatom darstellen und diese Gruppen jeweils optional einen Substituenten aufweisen und die Vielzahl von R^{XH1'} gleich oder verschieden sein können und miteinander kombiniert werden können, um zusammen mit den Kohlenstoffatomen, an die sie gebunden sind, einen Ring zu bilden, und
R^{XH1} und der Substituent, den der Ring R^{H1} optional aufweist, R^{XH1} und der Substituent, den der Ring R^{H2} optional aufweist, R^{XH1'} und der Substituent, den der Ring R^{H1} optional aufweist, und R^{XH1'} und der Substituent, den der Ring R^{H2} optional aufweist, jeweils miteinander kombiniert werden können, um zusammen mit den Atomen, an die sie gebunden sind, einen Ring zu bilden.

2. Lichtemittierende Vorrichtung nach Anspruch 1, wobei das vernetzbare Material eine niedermolekulare Verbindung mit mindestens einer vernetzbaren Gruppe, ausgewählt aus der Gruppe A der vernetzbaren Gruppe, oder eine Polymerverbindung ist, die eine vernetzbare Struktureinheit mit mindestens einer vernetzbaren Gruppe, ausgewählt aus der Gruppe A der vernetzbaren Gruppe, umfasst:
(Gruppe A der vernetzbaren Gruppe) 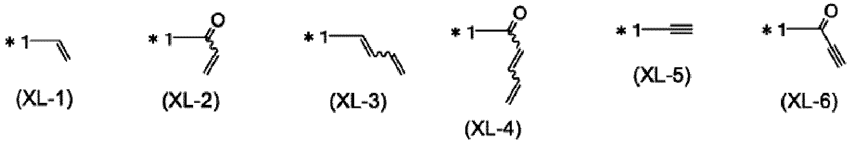 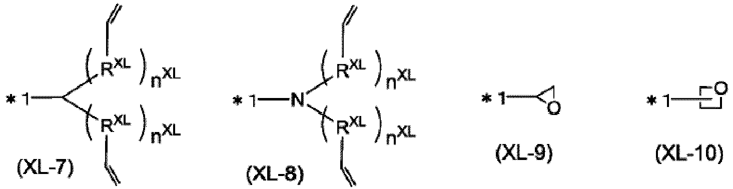 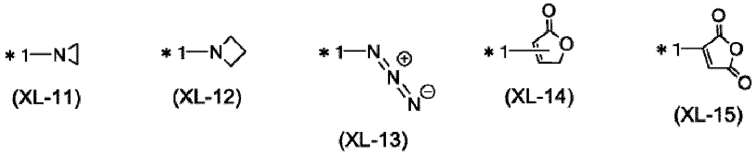 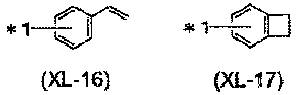 wobei R^{XL} eine Methylengruppe, ein Sauerstoffatom oder ein Schwefelatom darstellt, n^{XL} eine ganze Zahl von 0 bis 5 darstellt und wenn eine Vielzahl von R^{XL} vorhanden ist, diese gleich oder verschieden sein können, und wenn eine Vielzahl von n^{XL} vorhanden ist, diese gleich oder verschieden sein können, *1 eine Bindungsstelle darstellt und diese vernetzbaren Gruppen jeweils optional einen Substituenten aufweisen.

3. Lichtemittierende Vorrichtung nach Anspruch 2, wobei das vernetzbare Material eine Polymerverbindung ist, die eine vernetzbare Struktureinheit mit mindestens einer vernetzbaren Gruppe, ausgewählt aus der Gruppe A der vernetzbaren Gruppen, aufweist.

4. Lichtemittierende Vorrichtung nach Anspruch 3,
wobei die vernetzbare Struktureinheit eine Struktureinheit ist, die durch die Formel (2) dargestellt wird, oder eine Struktureinheit ist, die durch die Formel (2') dargestellt wird: 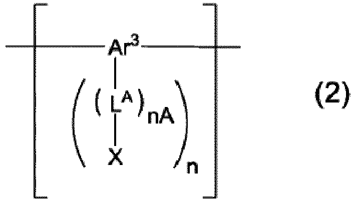 wobei
nA eine ganze Zahl von 0 bis 5 darstellt, n 1 oder 2 darstellt und wenn eine Vielzahl von nA vorhanden ist, diese gleich oder verschieden sein können,
Ar³ eine aromatische Kohlenwasserstoffgruppe oder eine heterocyclische Gruppe darstellt und diese Gruppen jeweils optional einen Substituenten aufweisen,
L^{A} eine Alkylengruppe, eine Cycloalkylengruppe, eine Arylengruppe, eine zweiwertige heterocyclische Gruppe, eine durch -NR'- dargestellte Gruppe, ein Sauerstoffatom oder ein Schwefelatom darstellt und diese Gruppen jeweils optional einen Substituenten aufweisen, R' ein Wasserstoffatom, eine Alkylgruppe, eine Cycloalkylgruppe, eine Arylgruppe oder eine einwertige heterocyclische Gruppe darstellt und diese Gruppen jeweils optional einen Substituenten aufweisen und wenn eine Vielzahl von L^{A} vorhanden ist, diese gleich oder verschieden sein können, und
X eine vernetzbare Gruppe darstellt, die aus der Gruppe A der vernetzbaren Gruppe ausgewählt ist, und wenn eine Vielzahl von X vorhanden ist, diese gleich oder verschieden sein können: 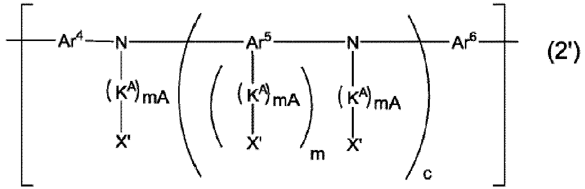
wobei
mA eine ganze Zahl von 0 bis 5 darstellt, m eine ganze Zahl von 1 bis 4 darstellt, c eine ganze Zahl von 0 oder 1 darstellt und wenn eine Vielzahl von mA vorhanden ist, diese gleich oder verschieden sein können,
Ar⁵ eine aromatische Kohlenwasserstoffgruppe, eine heterocyclische Gruppe oder eine Gruppe darstellt, in der mindestens ein aromatischer Kohlenwasserstoffring und mindestens ein heterocyclischer Ring direkt aneinander gebunden sind, und diese Gruppen jeweils optional einen Substituenten aufweisen,
Ar⁴ und Ar⁶ jeweils unabhängig voneinander eine Arylengruppe oder eine zweiwertige heterocyclische Gruppe darstellen und diese Gruppen jeweils optional einen Substituenten aufweisen,
Ar⁴ , Ar⁵ und Ar⁶ jeweils direkt oder über ein Sauerstoffatom oder ein Schwefelatom an eine andere Gruppe als diese Gruppen gebunden sein können, die an das Stickstoffatom binden, an das diese Gruppen gebunden sind, wodurch ein Ring gebildet wird,
K^{A} eine Alkylengruppe, eine Cycloalkylengruppe, eine Arylengruppe, eine zweiwertige heterocyclische Gruppe, eine durch -NR'- dargestellte Gruppe, ein Sauerstoffatom oder ein Schwefelatom darstellt und diese Gruppen jeweils optional einen Substituenten aufweisen, R' ein Wasserstoffatom, eine Alkylgruppe, eine Cycloalkylgruppe, eine Arylgruppe oder eine einwertige heterocyclische Gruppe darstellt und diese Gruppen jeweils optional einen Substituenten aufweisen und wenn eine Vielzahl von K^{A} vorhanden ist, diese gleich oder verschieden sein können, und
X' eine vernetzbare Gruppe darstellt, ausgewählt aus der Gruppe A der vernetzbaren Gruppe, einem Wasserstoffatom, einer Alkylgruppe, einer Cycloalkylgruppe, einer Arylgruppe oder einer einwertigen heterocyclischen Gruppe, und diese Gruppen jeweils optional einen Substituenten aufweisen und wenn eine Vielzahl von X' vorhanden ist, diese gleich oder verschieden sein können und mindestens ein X' eine vernetzbare Gruppe ist, die aus der Gruppe A der vernetzbaren Gruppe ausgewählt ist.

5. Lichtemittierende Vorrichtung nach einem der Ansprüche 2 bis 4, wobei die vernetzbare Gruppe, die das vernetzbare Material aufweist, eine Gruppe ist, die dargestellt wird durch die Formel (XL-2), Formel (XL-3), Formel (XL-4), Formel (XL-5), Formel (XL-6), Formel (XL-7), Formel (XL-8), Formel (XL-9), Formel (XL-10), Formel (XL-11), Formel (XL-12), Formel (XL-13), Formel (XL-14), Formel (XL-15) oder Formel (XL-17).

6. Lichtemittierende Vorrichtung nach einem der Ansprüche 1 bis 5, wobei die Gruppe, die durch die Formel (H1-1) dargestellt wird, eine durch die Formel (H1-1B) dargestellte Gruppe, eine durch die Formel (H1-1C) dargestellte Gruppe oder eine durch die Formel (H1-1D) dargestellte Gruppe ist: 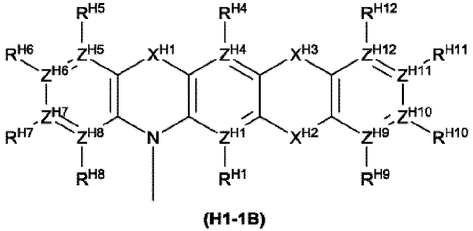 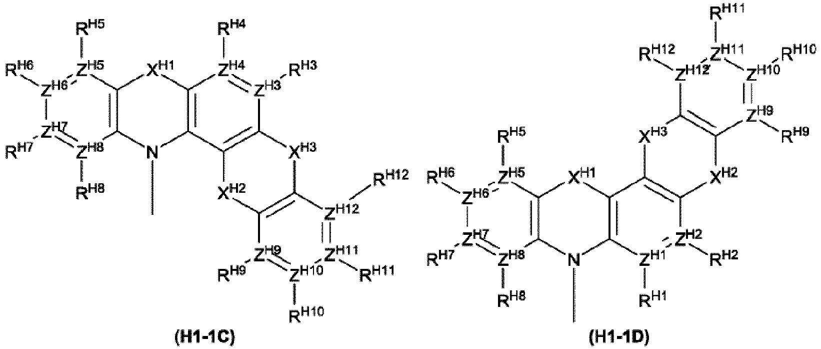 wobei
X^{H1} die gleiche Bedeutung wie oben definiert darstellt,
X^{H2} und X^{H3} jeweils unabhängig voneinander eine Einfachbindung, ein Sauerstoffatom, ein Schwefelatom, eine durch -N(R^{XH2})- dargestellte Gruppe oder eine durch -C(R^{XH2'})₂- dargestellte Gruppe darstellen, R^{XH2} und R^{XH2'} jeweils unabhängig voneinander ein Wasserstoffatom, eine Alkylgruppe, eine Cycloalkylgruppe, eine Alkoxygruppe, eine Cycloalkoxygruppe, eine Arylgruppe, eine Aryloxygruppe, eine einwertige heterocyclische Gruppe, eine substituierte Aminogruppe oder ein Halogenatom darstellen und diese Gruppen jeweils optional einen Substituenten aufweisen und die Vielzahl von R^{XH2'} gleich oder verschieden sein können und zusammen mit den Kohlenstoffatomen, an die sie gebunden sind, kombiniert werden können, um einen Ring zu bilden,
ZH¹, ZH², ZH³, ZH⁴, ZH⁵, ZH⁶, ZH⁷, ZH⁸, ZH⁹, ZH¹⁰, ZH¹¹ und ZH¹² jeweils unabhängig voneinander ein Kohlenstoffatom oder ein Stickstoffatom darstellen,
RH¹, RH², RH³, RH⁴, RH⁵, RH⁶, RH⁷, RH⁸, RH⁹, RH¹⁰, RH¹¹ und RH¹² jeweils unabhängig voneinander ein Wasserstoffatom, eine Alkylgruppe, eine Cycloalkylgruppe, eine Alkoxygruppe, eine Cycloalkoxygruppe, eine Arylgruppe, eine Aryloxygruppe, eine einwertige heterocyclische Gruppe, eine substituierte Aminogruppe oder ein Halogenatom darstellen und diese Gruppen jeweils optional einen Substituenten aufweisen,
R^{H1} nicht vorhanden ist, wenn Z^{H1} ein Stickstoffatom ist, R^{H2} nicht vorhanden ist, wenn Z^{H2} ein Stickstoffatom ist, R^{H3} nicht vorhanden ist, wenn Z^{H3} ein Stickstoffatom ist, R^{H4} nicht vorhanden ist, wenn Z^{H4} ein Stickstoffatom ist, R^{H5} nicht vorhanden ist, wenn Z^{H5} ein Stickstoffatom ist, R^{H6} nicht vorhanden ist, wenn Z^{H6} ein Stickstoffatom ist, R^{H7} nicht vorhanden ist, wenn Z^{H7} ein Stickstoffatom ist, R^{H8} nicht vorhanden ist, wenn Z^{H8} ein Stickstoffatom ist, R^{H9} nicht vorhanden ist, wenn Z^{H9} ein Stickstoffatom ist, R^{H10} nicht vorhanden ist, wenn Z^{H10} ein Stickstoffatom ist, RH¹¹ nicht vorhanden ist, wenn Z^{H11} ein Stickstoffatom ist, und R^{H12} nicht vorhanden ist, wenn Z^{H12} ein Stickstoffatom ist, und
R^{H1} und R^{H2}, R^{H3} und R^{H4}, R^{H5} und R^{H6}, R^{H6} und R^{H7}, R^{H7} und R^{H8}, R^{H9} und R^{H10}, R^{H10} und R^{H11}, und R^{H11} und RH¹² jeweils miteinander kombiniert werden können, um zusammen mit den Kohlenstoffatomen, an die sie gebunden sind, einen Ring zu bilden.

7. Lichtemittierende Vorrichtung nach einem der Ansprüche 1 bis 6, wobei die durch die Formel (1) dargestellte phosphoreszierende Verbindung eine durch die Formel (1-B) dargestellte phosphoreszierende Verbindung ist: 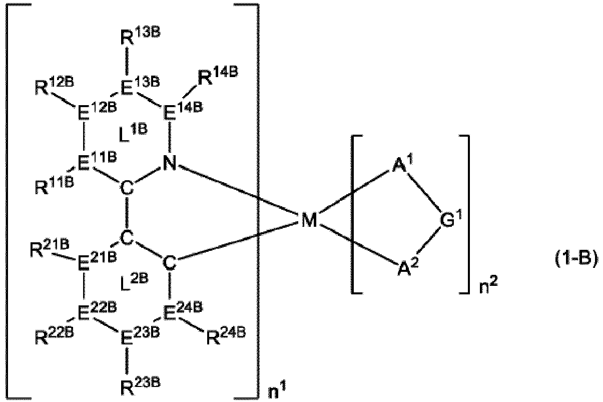 wobei
M, n¹, n² und A¹ -G¹ -A² die gleiche Bedeutung wie oben definiert darstellen,
E^{11B}, E^{12B}, E^{13B}, E^{14B}, E^{21E}, E^{22B}, E^{23B} und E^{24B} jeweils unabhängig voneinander ein Stickstoffatom oder ein Kohlenstoffatom darstellen und wenn eine Vielzahl von E^{11B}, E^{12B}, E^{13B}, E^{14B}, E^{21B}, E^{22B}, E^{23B} und E^{24B} vorhanden ist, diese bei jedem Auftreten gleich oder verschieden sein können, R^{11B} nicht vorhanden ist, wenn E^{11B} ein Stickstoffatom ist, R^{12B} nicht vorhanden ist, wenn E^{12B} ein Stickstoffatom ist, R^{13B} nicht vorhanden ist, wenn E^{13B} ein Stickstoffatom ist, R^{14B} nicht vorhanden ist, wenn E^{14B} ein Stickstoffatom ist, R^{21B} nicht vorhanden ist, wenn E^{21B} ein Stickstoffatom ist, R^{22B} nicht vorhanden ist, wenn E^{22B} ein Stickstoffatom ist, R^{23B} nicht vorhanden ist, wenn E^{23B} ein Stickstoffatom ist und R^{24B} nicht vorhanden ist, wenn E^{24B} ein Stickstoffatom ist,
R^{11B}, R^{12B}, R^{13B}, R^{14B}, R^{21B}, R^{22B} , R^{23B} und R^{24B} jeweils unabhängig voneinander ein Wasserstoffatom, eine Alkylgruppe, eine Cycloalkylgruppe, eine Alkoxygruppe, eine Cycloalkoxygruppe, eine Arylgruppe, eine Aryloxygruppe, eine einwertige heterocyclische Gruppe, eine substituierte Aminogruppe oder ein Halogenatom darstellen und diese Gruppen jeweils optional einen Substituenten aufweisen und wenn eine Vielzahl von R^{11B}, R^{12B}, R^{13B}, R^{14B}, R^{21B}, R^{22B} , R^{23B} und R^{24B} vorhanden ist, diese bei jedem Auftreten gleich oder verschieden sein können und R^{11B} und R^{12B}, R^{12B} und R^{13B} , R^{13B} und R^{14B}, R^{11B} und R^{21B}, R^{21B} und R^{22B}, R^{22B} und R^{23B}, und R^{23B} und R^{24B} jeweils miteinander kombiniert werden können, um zusammen mit den Atomen, an die sie gebunden sind, einen Ring zu bilden,
der Ring L^{1B} einen Pyridinring oder einen Pyrimidinring darstellt, bestehend aus einem Stickstoffatom, einem Kohlenstoffatom, E^{11B}, E^{12B}, E^{13B} und E^{14B}, und
der Ring L^{2B} einen Benzolring, einen Pyridinring oder einen Pyrimidinring darstellt, bestehend aus zwei Kohlenstoffatomen, E^{21B}, E^{22B}, E^{23B} und E^{24B}.

8. Lichtemittierende Vorrichtung nach Anspruch 7, wobei die phosphoreszierende Verbindung, die durch die Formel (1-B) dargestellt wird, eine durch die Formel (1-B1) dargestellte phosphoreszierende Verbindung, eine durch die Formel (1-B2) dargestellte phosphoreszierende Verbindung, eine durch die Formel (1-B3) dargestellte phosphoreszierende Verbindung, eine durch die Formel (1-B4) dargestellte phosphoreszierende Verbindung oder eine durch die Formel (1-B5) dargestellte phosphoreszierende Verbindung ist: 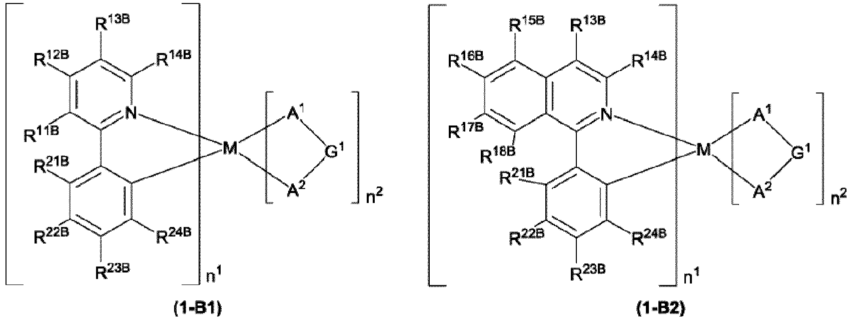 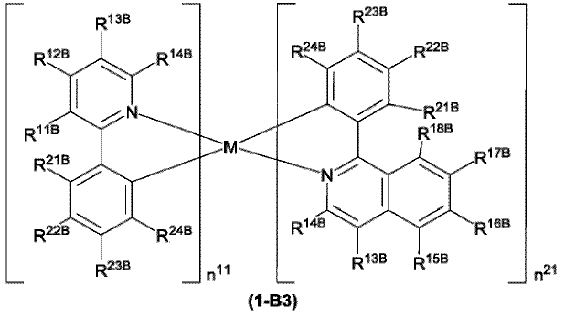 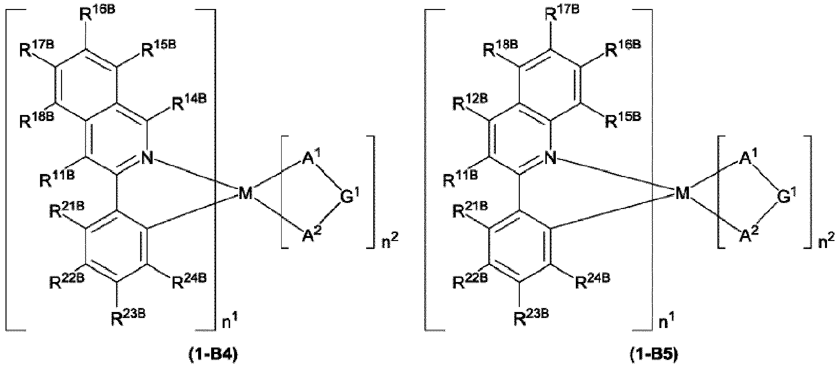 wobei
M, n¹, n², A¹ -G¹ -A², R^{11B}, R^{12B}, R^{13B}, R^{14B}, R^{21B}, R^{22B}, R^{23B} und R^{24B} die gleiche Bedeutung wie oben definiert darstellen,
n¹¹ und n¹² jeweils unabhängig voneinander eine ganze Zahl von 1 oder mehr darstellen, n¹¹ +n¹² 2 oder 3 ist, n¹¹ + n¹² 3 ist, wenn M ein Rutheniumatom, ein Rhodiumatom oder ein Iridiumatom ist, und n¹¹ + n¹² 2 ist, wenn M ein Palladiumatom oder ein Platinatom ist, und
R^{15B}, R^{16B}, R^{17B} und R^{18B} jeweils unabhängig voneinander ein Wasserstoffatom, eine Alkylgruppe, eine Cycloalkylgruppe, eine Alkoxygruppe, eine Cycloalkoxygruppe, eine Arylgruppe, eine Aryloxygruppe, eine einwertige heterocyclische Gruppe, eine substituierte Aminogruppe oder ein Halogenatom darstellen und diese Gruppen jeweils optional einen Substituenten aufweisen und wenn eine Vielzahl von R^{15B}, R^{16B}, R^{17B} und R^{18B} vorhanden ist, diese bei jedem Auftreten gleich oder verschieden sein können und R^{13B} und R^{15B}, R^{15B} und R^{16B}, R^{16B} und R^{17B}, R^{17B} und R^{18B}, und R^{18B} und R^{21B} jeweils miteinander kombiniert werden können, um zusammen mit den Atomen, an die sie gebunden sind, einen Ring zu bilden.

9. Lichtemittierende Vorrichtung nach einem der Ansprüche 1 bis 6, wobei die durch die Formel (1) dargestellte phosphoreszierende Verbindung eine durch die Formel (1-A) dargestellte phosphoreszierende Verbindung ist: 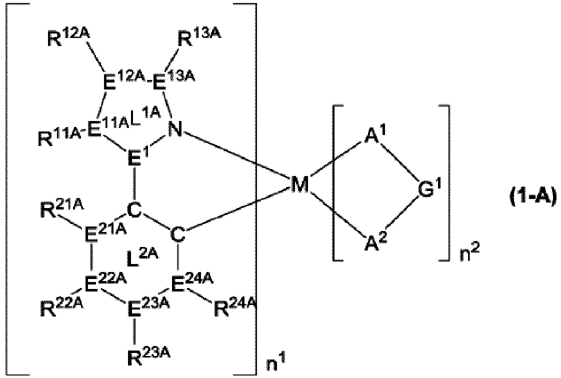 wobei
M, n¹, n², E¹ und A¹ -G¹ -A² die gleiche Bedeutung wie oben definiert darstellen,
E^{11A}, E^{12A}, E^{13A}, E^{21A}, E^{22A}, E^{23A} und E^{21A} jeweils unabhängig voneinander ein Stickstoffatom oder ein Kohlenstoffatom darstellen und wenn eine Vielzahl von E^{11A}, E^{12A}, E^{13A}, E^{21A}, E^{22A}, E^{23A} und E^{24A} vorhanden ist, diese bei jedem Auftreten gleich oder verschieden sein können, R^{11A} entweder vorhanden oder nicht vorhanden sein kann, wenn E^{11A} ein Stickstoffatom ist, R^{12A} entweder vorhanden oder nicht vorhanden sein kann, wenn E^{12A} ein Stickstoffatom ist, R^{13A} entweder vorhanden oder nicht vorhanden sein kann, wenn E^{13A} ein Stickstoffatom ist, R^{21A} nicht vorhanden ist, wenn E^{21A} ein Stickstoffatom ist, R^{22A} nicht vorhanden ist, wenn E^{22A} ein Stickstoffatom ist, R^{23A} nicht vorhanden ist, wenn E^{23A} ein Stickstoffatom ist und R^{24A} nicht vorhanden ist, wenn E^{24A} ein Stickstoffatom ist,
R^{11A}, R^{12A}, R^{13A}, R^{21A}, R^{22A}, R^{23A} und R^{24A} jeweils unabhängig voneinander ein Wasserstoffatom, eine Alkylgruppe, eine Cycloalkylgruppe, eine Alkoxygruppe, eine Cycloalkoxygruppe, eine Arylgruppe, eine Aryloxygruppe, eine einwertige heterocyclische Gruppe, eine substituierte Aminogruppe oder ein Halogenatom darstellen und diese Gruppen jeweils optional einen Substituenten aufweisen und wenn eine Vielzahl von R^{11A}, R^{12A}, R^{13A}, R^{21A}, R^{22A}, R^{23A} und R^{24A} vorhanden ist, diese bei jedem Auftreten gleich oder verschieden sein können und R^{11A} und R^{12A}, R^{12A} und R^{13A}, R^{11A} und R^{21A}, R^{21A} und R^{22A}, R^{22A} und R^{23A}, und R^{23A} und R^{24A} jeweils miteinander kombiniert werden können, um zusammen mit den Atomen, an die sie gebunden sind, einen Ring zu bilden,
der Ring L^{1A} einen Triazolring oder einen Diazolring darstellt, der aus einem Stickstoffatom, E¹, E^{11A}, E^{12A} und E^{13A} besteht, und
der Ring L^{2A} einen Benzolring, einen Pyridinring oder einen Pyrimidinring darstellt, der aus zwei Kohlenstoffatomen, E^{21A} , E^{22A}, E^{23A} und E^{24A} besteht.

10. Lichtemittierende Vorrichtung nach einem der Ansprüche 1 bis 9, wobei die erste organische Schicht und die zweite organische Schicht aneinander angrenzen.

11. Lichtemittierende Vorrichtung nach einem der Ansprüche 1 bis 10, wobei die zweite organische Schicht eine Schicht ist, die zwischen der Anode und der ersten organischen Schicht angeordnet ist.

12. Lichtemittierende Vorrichtung nach einem der Ansprüche 1 bis 6, wobei der Substituent, den der Ring L¹ optional aufweist, eine Arylgruppe oder eine einwertige heterocyclische Gruppe ist und diese Gruppen jeweils optional ferner einen Substituenten aufweisen und der Substituent, den der Ring L² optional aufweist, eine Arylgruppe oder eine einwertige heterocyclische Gruppe ist und diese Gruppen jeweils optional ferner einen Substituenten aufweisen.

13. Lichtemittierende Vorrichtung nach Anspruch 7 oder 8, wobei das R^{11B}, das R^{12B}, das R^{13B}, das R^{14B}, das R^{21B}, das R^{22B}, das R^{23B} und das R^{24B} jeweils unabhängig voneinander ein Wasserstoffatom oder eine durch die Formel (D-A) dargestellte Gruppe, eine durch die Formel (D-B) dargestellte Gruppe oder eine durch die Formel (D-C) dargestellte Gruppe darstellen: 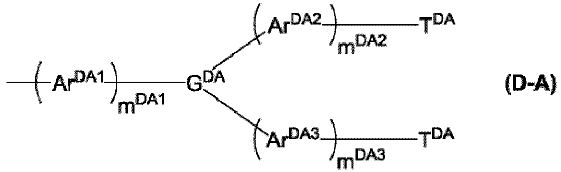 wobei
m^{DA1}, m^{DA2} und m^{DA3} jeweils unabhängig voneinander eine ganze Zahl von 0 oder mehr darstellen,
G^{DA} ein Stickstoffatom, eine aromatische Kohlenwasserstoffgruppe oder eine heterocyclische Gruppe darstellt und diese Gruppen jeweils optional einen Substituenten aufweisen,
Ar^{DA1}, Ar^{DA2} und Ar^{DA3} jeweils unabhängig voneinander eine Arylengruppe oder eine zweiwertige heterocyclische Gruppe darstellen und diese Gruppen jeweils optional einen Substituenten aufweisen und wenn eine Vielzahl von Ar^{DA1}, Ar^{DA2} und Ar^{DA3} vorhanden ist, diese bei jedem Auftreten gleich oder verschieden sein können, und
T^{DA} eine Arylgruppe oder eine einwertige heterocyclische Gruppe darstellt und diese Gruppen jeweils optional einen Substituenten aufweisen und die Vielzahl von T^{DA} gleich oder verschieden sein können: 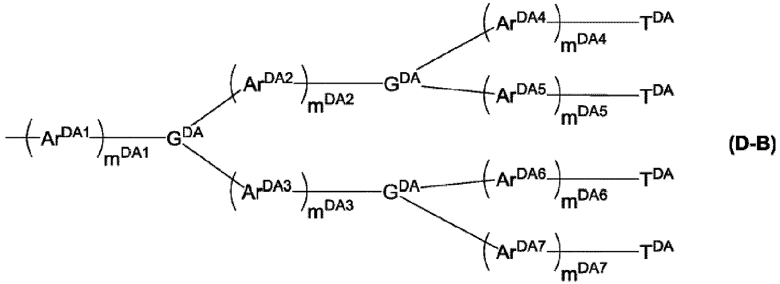
wobei
m^{DA1}, m^{DA2}, m^{DA3}, m^{DA4}, m^{DA5}, m^{DA6} und m^{DA7} jeweils unabhängig voneinander eine ganze Zahl von 0 oder mehr darstellen,
G^{DA} ein Stickstoffatom, eine aromatische Kohlenwasserstoffgruppe oder eine heterocyclische Gruppe darstellt und diese Gruppen jeweils optional einen Substituenten aufweisen und die Vielzahl von G^{DA} gleich oder verschieden sein können,
Ar^{DA1}, Ar^{DA2}, Ar^{DA3}, Ar^{DA4}, Ar^{DA5}, Ar^{DA6} und Ar^{DA7} jeweils unabhängig voneinander eine Arylengruppe oder eine zweiwertige heterocyclische Gruppe darstellen und diese Gruppen jeweils optional einen Substituenten aufweisen und wenn eine Vielzahl von Ar^{DA1}, Ar^{DA2}, Ar^{DA3}, Ar^{DA4}, Ar^{DA5}, Ar^{DA6} und Ar^{DA7} vorhanden ist, diese bei jedem Auftreten gleich oder verschieden sein können, und
T^{DA} eine Arylgruppe oder eine einwertige heterocyclische Gruppe darstellt und diese Gruppen jeweils optional einen Substituenten aufweisen und die Vielzahl von T^{DA} gleich oder verschieden sein können: 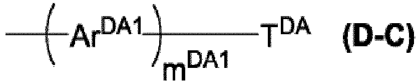
wobei
m^{DA1} eine ganze Zahl von 0 oder mehr darstellt,
Ar^{DA1} eine Arylengruppe oder eine zweiwertige heterocyclische Gruppe darstellt und diese Gruppen jeweils optional einen Substituenten aufweisen und wenn eine Vielzahl von Ar^{DA1} vorhanden ist, diese gleich oder verschieden sein können, und
T^{DA} eine Arylgruppe oder eine einwertige heterocyclische Gruppe darstellt und diese Gruppen jeweils optional einen Substituenten aufweisen.

14. Lichtemittierende Vorrichtung nach einem der Ansprüche 2 bis 13, wobei die vernetzbare Gruppe, ausgewählt aus der Gruppe A der vernetzbaren Gruppe, eine durch die Formel (XL-17) dargestellt Gruppe ist.

## Revendications

1. Dispositif luminescent, comprenant une anode, une cathode, une première couche organique disposée entre l'anode et la cathode, et une seconde couche organique disposée entre l'anode et la cathode, dans lequel
la première couche organique est une couche comprenant un composé phosphorescent représenté par la formule (1) et un composé représenté par la formule (H), et
la seconde couche organique est une couche comprenant un corps réticulé d'un matériau réticulable : 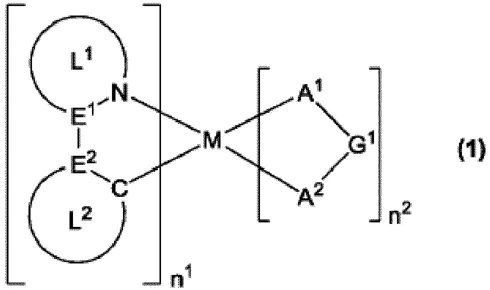 dans lequel
M représente un atome de ruthénium, un atome de rhodium, un atome de palladium, un atome d'iridium ou un atome de platine,
n¹ représente un nombre entier relatif d' 1 ou plus, n² représente un nombre entier relatif de 0 ou plus, n¹+n² est 2 ou 3, n¹+n² est 3 lorsque M est un atome de ruthénium, un atome de rhodium ou un atome d'iridium, et n¹+n² est 2 lorsque M est un atome de palladium ou un atome de platine,
E¹ et E² représentent chacun indépendamment un atome de carbone ou un atome d'azote, et au moins un de E¹ et de E² est un atome de carbone, et, lorsqu'une pluralité de E¹ et de E² sont présents, ils peuvent être les mêmes ou différents dans chaque cas,
l'anneau L¹ représente un anneau hétérocyclique aromatique, et l'anneau a optionnellement un substituant, et, lorsqu'une pluralité des substituants sont présents, ils peuvent être les mêmes ou différents et peuvent être combinés ensemble pour former un anneau conjointement avec les atomes auxquels ils sont attachés, et, lorsqu'une pluralité des anneaux L¹ sont présents, ils peuvent être les mêmes ou différents,
l'anneau L² représente un anneau hydrocarbure aromatique ou un anneau hétérocyclique aromatique, ces anneaux ont chacun optionnellement un substituant, et, lorsqu'une pluralité des substituants sont présents, ils peuvent être les mêmes ou différents et peuvent être combinés ensemble pour former un anneau conjointement avec les atomes auxquels ils sont attachés, et, lorsqu'une pluralité des anneaux L² sont présents, ils peuvent être les mêmes ou différents,
le substituant que l'anneau L¹ a optionnellement et le substituant que l'anneau L² a optionnellement peuvent être combinés ensemble pour former un anneau conjointement avec les atomes auxquels ils sont attachés, et
A¹-G¹-A² représente un ligand bidenté anionique, A¹ et A² représentent chacun indépendamment un atome de carbone, un atome d'oxygène ou un atome d'azote, et ces atomes peuvent chacun être un atome constituant un anneau, G¹ représente une seule liaison ou un groupe aromatique constituant un ligand bidenté conjointement avec A¹ et A², et, lorsqu'une pluralité de A¹-G¹-A² sont présents, ils peuvent être les mêmes ou différents : 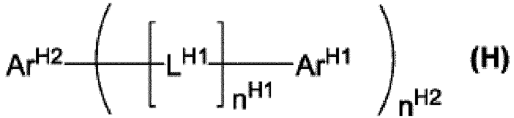
dans lequel
n^{H1} représente un nombre entier relatif de 0 ou plus et 5 ou moins, et, lorsqu'une pluralité de n^{H1} sont présents, ils peuvent être les mêmes ou différents,
n^{H2} représente un nombre entier relatif d' 1 ou plus et 10 ou moins,
Ar^{H1} représente un groupe représenté par la formule (H1-1), et, lorsqu'une pluralité de Ar^{H1} sont présents, ils peuvent être les mêmes ou différents,
L^{H1} représente un groupe alkylène, un groupe cycloalkylène, un groupe arylène, un groupe hétérocyclique divalent, un groupe représenté par -NR^{H1'}-, un atome d'oxygène ou un atome de soufre, et ces groupes ont chacun optionnellement un substituant, R^{H1'} représente un atome d'hydrogène, un groupe alkyle, un groupe cycloalkyle, un groupe aryle ou un groupe hétérocyclique monovalent, et ces groupes ont chacun optionnellement un substituant, et, lorsqu'une pluralité de L^{H1} sont présents, ils peuvent être les mêmes ou différents, et
Ar^{H2} représente un groupe hydrocarbure aromatique ou un groupe hétérocyclique aromatique, et ces groupes ont chacun optionnellement un substituant : 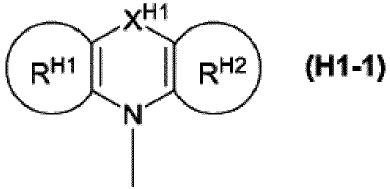
dans lequel
l'anneau R^{H1} et l'anneau R^{H2} représentent chacun indépendamment un anneau hydrocarbure aromatique monocyclique ou à anneau fusionné, ou un anneau hétérocyclique aromatique monocyclique ou à anneau fusionné, et ces anneaux ont chacun optionnellement un substituant, et, lorsqu'une pluralité des substituants sont présents, ils peuvent être les mêmes ou différents et peuvent être combinés ensemble pour former un anneau conjointement avec les atomes auxquels ils sont attachés,
au moins un de l'anneau R^{H1} et de l'anneau R^{H2} représente un anneau hydrocarbure aromatique à anneau fusionné ou un anneau hétérocyclique aromatique à anneau fusionné, et ces anneaux ont chacun optionnellement un substituant,
X^{H1} représente une seule liaison, un atome d'oxygène, un atome de soufre, un groupe représenté par -N(R^{XH1})- ou un groupe représenté par -C(R^{XH1'})₂-, R^{XH1} et R^{XH1'} représentent chacun indépendamment un atome d'hydrogène, un groupe alkyle, un groupe cycloalkyle, un groupe alkoxy, un groupe cycloalkoxy, un groupe aryle, un groupe aryloxy, un groupe hétérocyclique monovalent, un groupe amino substitué ou un atome d'halogène, et ces groupes ont chacun optionnellement un substituant, et la pluralité de R^{XH1}' peuvent être les mêmes ou différents et peuvent être combinés ensemble pour former un anneau conjointement avec les atomes de carbone auxquels ils sont attachés, et
R^{XH1} et le substituant que l'anneau R^{H1} a optionnellement, R^{XH1} et le substituant que l'anneau R^{H2} a optionnellement, R^{XH1'} et le substituant que l'anneau R^{H1} a optionnellement, et R^{XH1'} et le substituant que l'anneau R^{H2} a optionnellement peuvent chacun être combinés ensemble pour former un anneau conjointement avec les atomes auxquels ils sont attachés.

2. Dispositif luminescent selon la revendication 1,
dans lequel le matériau réticulable est un composé de bas poids moléculaire ayant au moins un groupe réticulable sélectionné parmi Groupe A de groupe réticulable, ou un composé polymère comprenant une unité constitutive réticulable ayant au moins un groupe réticulable sélectionné parmi Groupe A de groupe réticulable :
(Groupe A de Groupe réticulable) 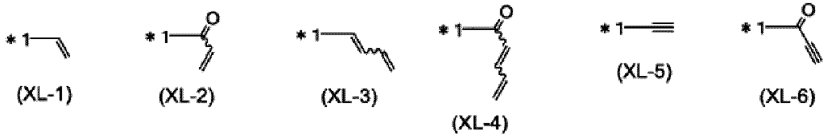 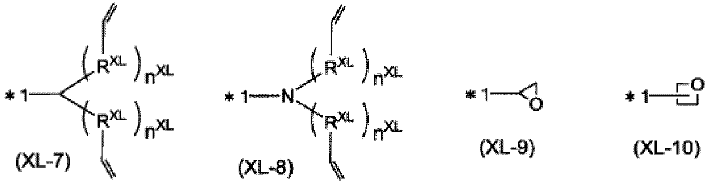 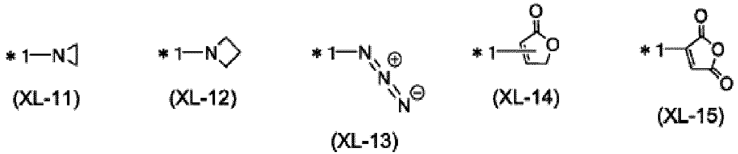 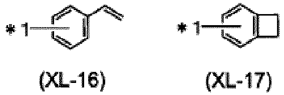 dans lequel R^{XL} représente un groupe méthylène, un atome d'oxygène ou un atome de soufre, n^{XL} représente un nombre entier relatif de 0 à 5, et, lorsqu'une pluralité de R^{XL} sont présents, ils peuvent être les mêmes ou différents, et, lorsqu'une pluralité de n^{XL} sont présents, ils peuvent être les mêmes ou différents, *1 représente un site de liaison, et ces groupes réticulables ont chacun optionnellement un substituant.

3. Dispositif luminescent selon la revendication 2,
dans lequel le matériau réticulable est un composé polymère comprenant une unité constitutive réticulable ayant au moins un groupe réticulable sélectionné parmi Groupe A de groupe réticulable.

4. Dispositif luminescent selon la revendication 3,
dans lequel l'unité constitutive réticulable est une unité constitutive représentée par la formule (2) ou une unité constitutive représentée par la formule (2') : 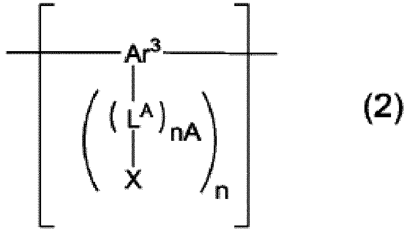 dans lequel
nA représente un nombre entier relatif de 0 à 5, n représente 1 ou 2, et, lorsqu'une pluralité de nA sont présents, ils peuvent être les mêmes ou différents,
Ar³ représente un groupe hydrocarbure aromatique ou un groupe hétérocyclique, et ces groupes ont chacun optionnellement un substituant,
L^{A} représente un groupe alkylène, un groupe cycloalkylène, un groupe arylène, un groupe hétérocyclique divalent, un groupe représenté par -NR'-, un atome d'oxygène ou un atome de soufre, et ces groupes ont chacun optionnellement un substituant, R' représente un atome d'hydrogène, un groupe alkyle, un groupe cycloalkyle, un groupe aryle ou un groupe hétérocyclique monovalent, et ces groupes ont chacun optionnellement un substituant, et, lorsqu'une pluralité de L^{A} sont présents, ils peuvent être les mêmes ou différents, et
X représente un groupe réticulable sélectionné parmi Groupe A de groupe réticulable, et, lorsqu'une pluralité de X sont présents, ils peuvent être les mêmes ou différents : 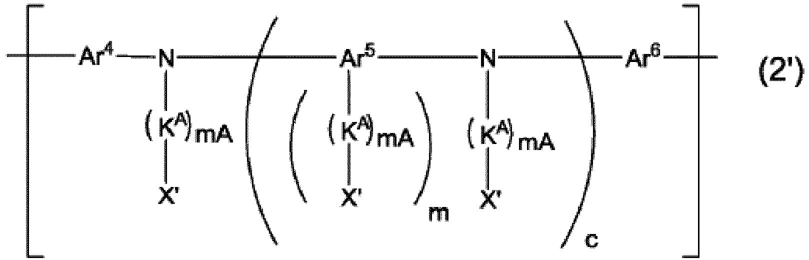
dans lequel
mA représente un nombre entier relatif de 0 à 5, m représente un nombre entier relatif d' 1 à 4, c représente un nombre entier relatif de 0 ou 1, et, lorsqu'une pluralité de mA sont présents, ils peuvent être les mêmes ou différents,
Ar⁵ représente un groupe hydrocarbure aromatique, un groupe hétérocyclique, ou un groupe dans lequel au moins un anneau hydrocarbure aromatique et au moins un anneau hétérocyclique sont liés directement l'un à l'autre, et ces groupes ont chacun optionnellement un substituant,
Ar⁴ et Ar⁶ représentent chacun indépendamment un groupe arylène ou un groupe hétérocyclique divalent, et ces groupes ont chacun optionnellement un substituant,
Ar⁴, Ar⁵ et Ar⁶ peuvent chacun être liés directement ou par l'intermédiaire d'un atome d'oxygène ou d'un atome de soufre à un groupe autre que ces groupes se liant à l'atome d'azote auquel ces groupes sont attachés, ainsi formant un anneau,
K^{A} représente un groupe alkylène, un groupe cycloalkylène, un groupe arylène, un groupe hétérocyclique divalent, un groupe représenté par -NR'-, un atome d'oxygène ou un atome de soufre, et ces groupes ont chacun optionnellement un substituant, R' représente un atome d'hydrogène, un groupe alkyle, un groupe cycloalkyle, un groupe aryle ou un groupe hétérocyclique monovalent, et ces groupes ont chacun optionnellement un substituant, et, lorsqu'une pluralité de K^{A} sont présents, ils peuvent être les mêmes ou différents, et
X' représente un groupe réticulable sélectionné parmi Groupe A de groupe réticulable, un atome d'hydrogène, un groupe alkyle, un groupe cycloalkyle, un groupe aryle ou un groupe hétérocyclique monovalent, et ces groupes ont chacun optionnellement un substituant, et, lorsqu'une pluralité de X' sont présents, ils peuvent être les mêmes ou différents, et au moins un X' est un groupe réticulable sélectionné parmi Groupe A de groupe réticulable.

5. Dispositif luminescent selon l'une quelconque des revendications 2 à 4, dans lequel le groupe réticulable que le matériau réticulable a est un groupe représenté par la formule (XL-2), la formule (XL-3), la formule (XL-4), la formule (XL-5), la formule (XL-6), la formule (XL-7), la formule (XL-8), la formule (XL-9), la formule (XL-10), la formule (XL-11), la formule (XL-12), la formule (XL-13), la formule (XL-14), la formule (XL-15) ou la formule (XL-17).

6. Dispositif luminescent selon l'une quelconque des revendications 1 à 5, dans lequel le groupe représenté par la formule (H1-1) est un groupe représenté par la formule (H1-1B), un groupe représenté par la formule (H1-1C) ou un groupe représenté par la formule (H1-1D) : 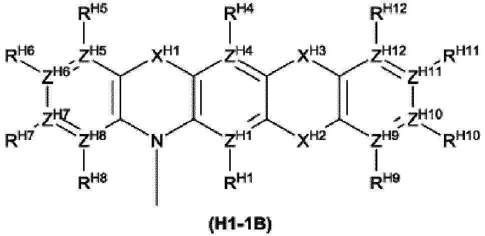 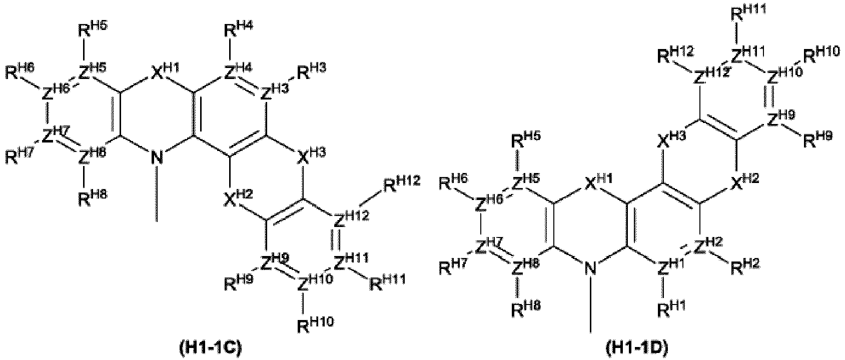 dans lequel
X^{H1} représente la même signification que celle définie ci-dessus,
X^{H2} et X^{H3} représentent chacun indépendamment une seule liaison, un atome d'oxygène, un atome de soufre, un groupe représenté par -N(R^{XH2})- ou un groupe représenté par -C(R^{XH2'})₂-, R^{XH2} et R^{XH2'} représentent chacun indépendamment un atome d'hydrogène, un groupe alkyle, un groupe cycloalkyle, un groupe alkoxy, un groupe cycloalkoxy, un groupe aryle, un groupe aryloxy, un groupe hétérocyclique monovalent, un groupe amino substitué ou un atome d'halogène, et ces groupes ont chacun optionnellement un substituant, et la pluralité de R^{XH2}' peuvent être les mêmes ou différents et peuvent être combinés ensemble pour former un anneau conjointement avec les atomes de carbone auxquels ils sont attachés,
Z^{H1}, Z^{H2}, Z^{H3}, Z^{H4}, Z^{H5}, Z^{H6}, Z^{H7}, Z^{H8}, Z^{H9}, Z^{H10}, Z^{H11} et Z^{H12} représentent chacun indépendamment un atome de carbone ou un atome d'azote,
R^{H1}, R^{H2}, R^{H3}, R^{H4}, R^{H5}, R^{H6}, R^{H7}, R^{H8}, R^{H9}, R^{H10}, R^{H11} et R^{H12} représentent chacun indépendamment un atome d'hydrogène, un groupe alkyle, un groupe cycloalkyle, un groupe alkoxy, un groupe cycloalkoxy, un groupe aryle, un groupe aryloxy, un groupe hétérocyclique monovalent, un groupe amino substitué ou un atome d'halogène, et ces groupes ont chacun optionnellement un substituant,
R^{H1} n'est pas présent lorsque Z^{H1} est un atome d'azote, R^{H2} n'est pas présent lorsque Z^{H2} est un atome d'azote, R^{H3} n'est pas présent lorsque Z^{H3} est un atome d'azote, R^{H4} n'est pas présent lorsque Z^{H4} est un atome d'azote, R^{H5} n'est pas présent lorsque Z^{H5} est un atome d'azote, R^{H6} n'est pas présent lorsque Z^{H6} est un atome d'azote, R^{H7} n'est pas présent lorsque Z^{H7} est un atome d'azote, R^{H8} n'est pas présent lorsque Z^{H8} est un atome d'azote, R^{H9} n'est pas présent lorsque Z^{H9} est un atome d'azote, R^{H10} n'est pas présent lorsque Z^{H10} est un atome d'azote, R^{H11} n'est pas présent lorsque Z^{H11} est un atome d'azote, et R^{H12} n'est pas présent lorsque Z^{H12} est un atome d'azote, et
R^{H1} et R^{H2}, R^{H3} et R^{H4}, R^{H5} et R^{H6}, R^{H6} et R^{H7}, R^{H7} et R^{H8}, R^{H9} et R^{H10}, R^{H10} et R^{H11} et R^{H11} et R^{H12} peuvent chacun être combinés ensemble pour former un anneau conjointement avec les atomes de carbone auxquels ils sont attachés.

7. Dispositif luminescent selon l'une quelconque des revendications 1 à 6, dans lequel le composé phosphorescent représenté par la formule (1) est un composé phosphorescent représenté par la formule (1-B) : 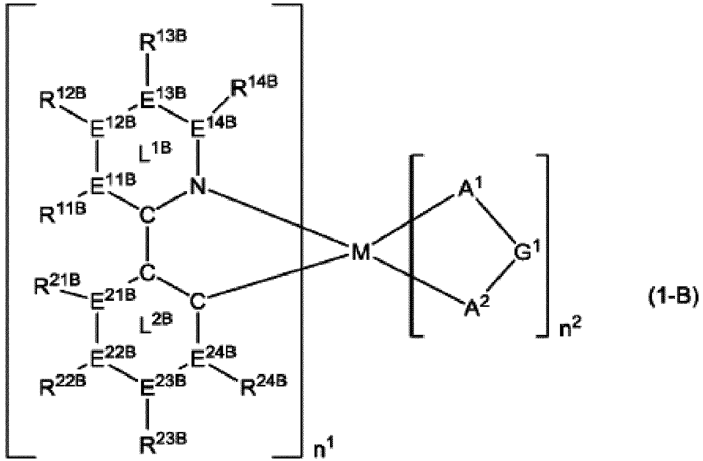 dans lequel
M, n¹, n² et A¹-G¹-A² représentent la même signification que celle définie ci-dessus,
E^{11B}, E^{12B}, E^{13B}, E^{14B}, E^{21B}, E^{22B}, E^{23B} et E^{24B} représentent chacun indépendamment un atome d'azote ou un atome de carbone, et, lorsqu'une pluralité de E^{11B}, de E^{12B}, de E^{13B}, de E^{14B}, de E^{21B}, de E^{22B}, de E^{23B} et de E^{24B} sont présents, ils peuvent être les mêmes ou différents dans chaque cas, R^{11B} n'est pas présent lorsque E^{11B} est un atome d'azote, R^{12B} n'est pas présent lorsque E^{12B} est un atome d'azote, R^{13B} n'est pas présent lorsque E^{13B}est un atome d'azote, R^{14B} n'est pas présent lorsque E^{14B} est un atome d'azote, R^{21B} n'est pas présent lorsque E^{21B} est un atome d'azote, R^{22B} n'est pas présent lorsque E^{22B} est un atome d'azote, R^{23B} n'est pas présent lorsque E^{23B} est un atome d'azote, et R^{24B} n'est pas présent lorsque E^{24B} est un atome d'azote,
R^{11B}, R^{12B}, R^{13B}, R^{14B}, R^{21B}, R^{22B}, R^{23B} et R^{24B} représentent chacun indépendamment un atome d'hydrogène, un groupe alkyle, un groupe cycloalkyle, un groupe alkoxy, un groupe cycloalkoxy, un groupe aryle, un groupe aryloxy, un groupe hétérocyclique monovalent, un groupe amino substitué ou un atome d'halogène, et ces groupes ont chacun optionnellement un substituant, et, lorsqu'une pluralité de R^{11B}, de R^{12B}, de R^{13B}, de R^{14B}, de R^{21B}, de R^{22B}, de R^{23B} et de R^{24B} sont présents, ils peuvent être les mêmes ou différents dans chaque cas, et R^{11B} et R^{12B}, R^{12B} et R^{13B}, R^{13B} et R^{14B}, R^{11B} et R^{21B}, R^{21B} et R^{22B}, R^{22B} et R^{23B}, et R^{23B} et R^{24B} peuvent chacun être combinés ensemble pour former un anneau conjointement avec les atomes auxquels ils sont attachés,
l'anneau L^{1B} représente un anneau pyridine ou un anneau pyrimidine constitué d'un atome d'azote, d'un atome de carbone, de E^{11B}, de E^{12B}, de E^{13B}et de E^{14B}, et
l'anneau L^{2B} représente un anneau benzène, un anneau pyridine ou un anneau pyrimidine constitué de deux atomes de carbone, de E^{21B}, de E^{22B}, de E^{23B} et de E^{24B}.

8. Dispositif luminescent selon la revendication 7, dans lequel le composé phosphorescent représenté par la formule (1-B) est un composé phosphorescent représenté par la formule (1-B1), un composé phosphorescent représenté par la formule (1-B2), un composé phosphorescent représenté par la formule (1-B3), un composé phosphorescent représenté par la formule (1-B4) ou un composé phosphorescent représenté par la formule (1-B5) : 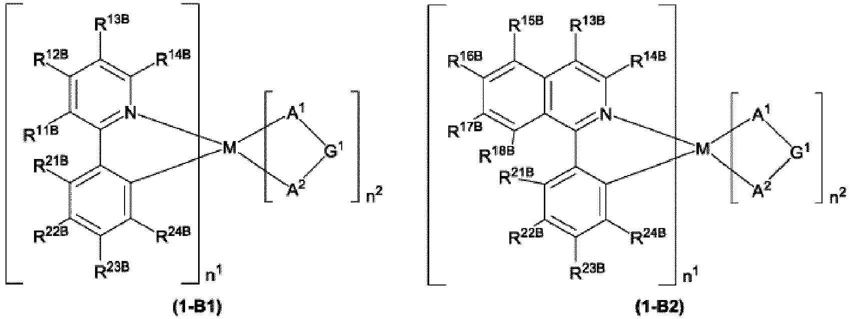 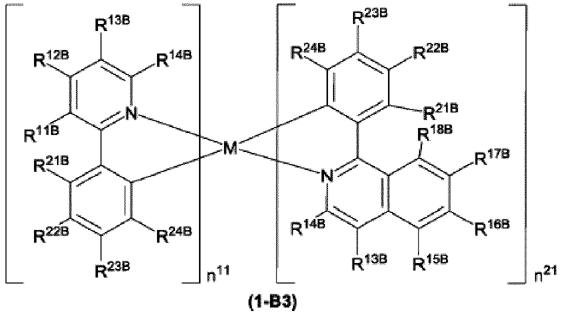 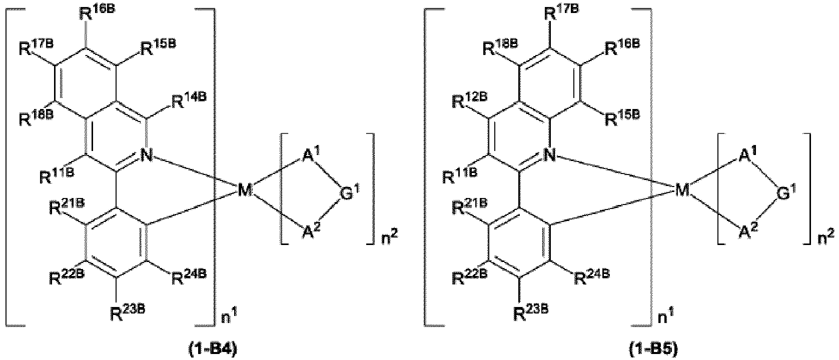 dans lequel
M, n¹, n², A¹-G¹-A², R^{11B}, R^{12B}, R^{13B}, R^{14B}, R^{21B}, R^{22B}, R^{23B} et R^{24B} représentent la même signification que celle définie ci-dessus,
n¹¹ et n¹² représentent chacun indépendamment un nombre entier relatif d' 1 ou plus, n¹¹+n¹² est 2 ou 3, n¹¹+n¹² est 3 lorsque M est un atome de ruthénium, un atome de rhodium ou un atome d'iridium, et n¹¹+n¹² est 2 lorsque M est un atome de palladium ou un atome de platine, et
R^{15B}, R^{16B}, R^{17B} et R^{18B} représentent chacun indépendamment un atome d'hydrogène, un groupe alkyle, un groupe cycloalkyle, un groupe alkoxy, un groupe cycloalkoxy, un groupe aryle, un groupe aryloxy, un groupe hétérocyclique monovalent, un groupe amino substitué ou un atome d'halogène, et ces groupes ont chacun optionnellement un substituant, et, lorsqu'une pluralité de R^{15B}, de R^{16B}, de R^{17B} et de R^{18B} sont présents, ils peuvent être les mêmes ou différents dans chaque cas, et R^{13B} et R^{15B}, R^{15B} et R^{16B}, R^{16B} et R^{17B}, R^{17B} et R^{18B}, et R^{18B} et R^{21B} peuvent chacun être combinés ensemble pour former un anneau conjointement avec les atomes auxquels ils sont attachés.

9. Dispositif luminescent selon l'une quelconque des revendications 1 à 6, dans lequel le composé phosphorescent représenté par la formule (1) est un composé phosphorescent représenté par la formule (1-A) : 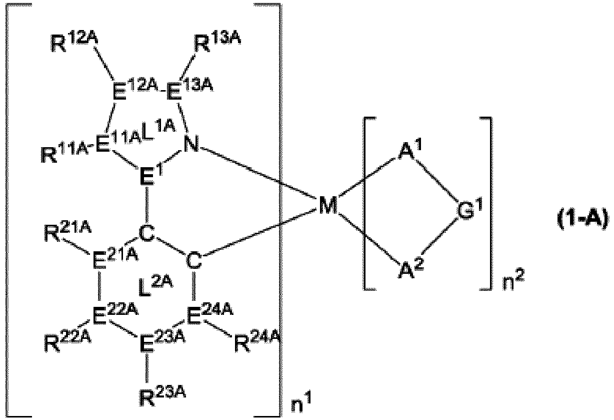 dans lequel
M, n¹, n², E¹ et A¹-G¹-A² représentent la même signification que celle définie ci-dessus,
E^{11A}, E^{12A}, E^{13A}, E^{21A}, E^{22A}, E^{23A} et E^{24A} représentent chacun indépendamment un atome d'azote ou un atome de carbone, et, lorsqu'une pluralité de E^{11A}, de E^{12A}, de E^{13A}, de E^{21A}, de E^{22A}, de E^{23A} et de E^{24A} sont présents, ils peuvent être les mêmes ou différents dans chaque cas, R^{11A} peut être soit présent soit non présent lorsque E^{11A} est un atome d'azote, R^{12A} peut être soit présent soit non présent lorsque E^{12A} est un atome d'azote, R^{13A} peut être soit présent soit non présent lorsque E^{13A} est un atome d'azote, R^{21A} n'est pas présent lorsque E^{21A} est un atome d'azote, R^{22A} n'est pas présent lorsque E^{22A} est un atome d'azote, R^{23A} n'est pas présent lorsque E^{23A} est un atome d'azote, et R^{24A} n'est pas présent lorsque E^{24A} est un atome d'azote,
R^{11A}, R^{12A}, R^{13A}, R^{21A}, R^{22A}, R^{23A} et R^{24A} représentent chacun indépendamment un atome d'hydrogène, un groupe alkyle, un groupe cycloalkyle, un groupe alkoxy, un groupe cycloalkoxy, un groupe aryle, un groupe aryloxy, un groupe hétérocyclique monovalent, un groupe amino substitué ou un atome d'halogène, et ces groupes ont chacun optionnellement un substituant, et, lorsqu'une pluralité de R^{11A}, de R^{12A}, de R^{13A}, de R^{21A}, de R^{22A}, de R^{23A} et de R^{24A} sont présents, ils peuvent être les mêmes ou différents dans chaque cas, et R^{11A} et R^{12A}, R^{12A} et R^{13A}, R^{11A} et R^{21A}, R^{21A} et R^{22A}, R^{22A} et R^{23A}, et R^{23A} et R^{24A} peuvent chacun être combinés ensemble pour former un anneau conjointement avec les atomes auxquels ils sont attachés,
l'anneau L^{1A} représente un anneau triazole ou un anneau diazole constitué d'un atome d'azote, de E¹, de E^{11A}, de E^{12A} et de E^{13A}, et
l'anneau L^{2A} représente un anneau benzène, un anneau pyridine ou un anneau pyrimidine constitué de deux atomes de carbone, de E^{21A}, de E^{22A}, de E^{23A} et de E^{24A}.

10. Dispositif luminescent selon l'une quelconque des revendications 1 à 9, dans lequel la première couche organique et la seconde couche organique sont adjacentes l'une à l'autre.

11. Dispositif luminescent selon l'une quelconque des revendications 1 à 10, dans lequel la seconde couche organique est une couche disposée entre l'anode et la première couche organique.

12. Dispositif luminescent selon l'une quelconque des revendications 1 à 6, dans lequel le substituant que l'anneau L¹ a optionnellement est un groupe aryle ou un groupe hétérocyclique monovalent, et ces groupes ont chacun optionnellement en outre un substituant, et le substituant que l'anneau L² a optionnellement est un groupe aryle ou un groupe hétérocyclique monovalent, et ces groupes ont chacun optionnellement en outre un substituant.

13. Dispositif luminescent selon la revendication 7 ou 8, dans lequel le R^{11B}, le R^{12B}, le R^{13B}, le R^{14B}, le R^{21B}, le R^{22B}, le R^{23B} et le R^{24B} représentent chacun indépendamment un atome d'hydrogène ou un groupe représenté par la formule (DA), un groupe représenté par la formule (D-B) ou un groupe représenté par la formule (D-C) 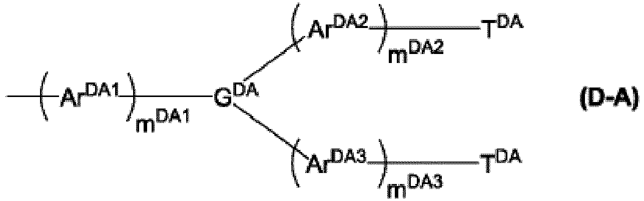 dans lequel
m^{DA1}, m^{DA2} et m^{DA3} représentent chacun indépendamment un nombre entier relatif de 0 ou plus,
G^{DA} représente un atome d'azote, un groupe hydrocarbure aromatique ou un groupe hétérocyclique, et ces groupes ont chacun optionnellement un substituant,
Ar^{DA1}, Ar^{DA2} et Ar^{DA3} représentent chacun indépendamment un groupe arylène ou un groupe hétérocyclique divalent, et ces groupes ont chacun optionnellement un substituant, et, lorsqu'une pluralité de Ar^{DA1}, de Ar^{DA2} et de Ar^{DA3} sont présents, ils peuvent être les mêmes ou différents dans chaque cas, et
T^{DA} représente un groupe aryle ou un groupe hétérocyclique monovalent, et ces groupes ont chacun optionnellement un substituant, et la pluralité de T^{DA} peuvent être les mêmes ou différents : 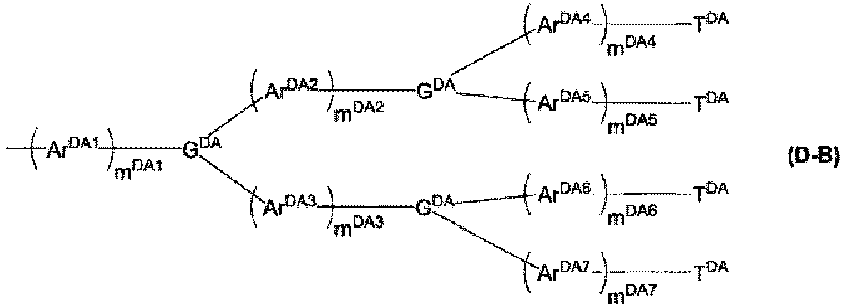
dans lequel
m^{DA1}, m^{DA2}, m^{DA3}, m^{DA4}, m^{DA5}, m^{DA6} et m^{DA7} représentent chacun indépendamment un nombre entier relatif de 0 ou plus,
G^{DA} représente un atome d'azote, un groupe hydrocarbure aromatique ou un groupe hétérocyclique, et ces groupes ont chacun optionnellement un substituant, et la pluralité de G^{DA} peuvent être les mêmes ou différents,
Ar^{DA1}, Ar^{DA2}, Ar^{DA3}, Ar^{DA4}, Ar^{DA5}, Ar^{DA6} et Ar^{DA7} représentent chacun indépendamment un groupe arylène ou un groupe hétérocyclique divalent, et ces groupes ont chacun optionnellement un substituant, et, lorsqu'une pluralité de Ar^{DA1}, de Ar^{DA2}, de Ar^{DA3}, de Ar^{DA4}, de Ar^{DA5}, de Ar^{DA6} et de Ar^{DA7} sont présents, ils peuvent être les mêmes ou différents dans chaque cas, et
T^{DA} représente un groupe aryle ou un groupe hétérocyclique monovalent, et ces groupes ont chacun optionnellement un substituant, et la pluralité de T^{DA} peuvent être les mêmes ou différents : 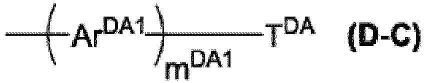
dans lequel
m^{DA1} représente un nombre entier relatif de 0 ou plus,
Ar^{DA1} représente un groupe arylène ou un groupe hétérocyclique divalent, et ces groupes ont chacun optionnellement un substituant, et, lorsqu'une pluralité de Ar^{DA1} sont présents, ils peuvent être les mêmes ou différents, et
T^{DA} représente un groupe aryle ou un groupe hétérocyclique monovalent, et ces groupes ont chacun optionnellement un substituant.

14. Dispositif luminescent selon l'une quelconque des revendications 2 à 13, dans lequel le groupe réticulable sélectionné parmi Groupe A de groupe réticulable est un groupe représenté par la formule (XL-17).
